# EUROPÄISCHE PATENTSCHRIFT

(11) **EP 1 572 735 B1**
(45) Veröffentlichungstag und Bekanntmachung des Hinweises auf die Patenterteilung: **16.12.2009**
(21) Anmeldenummer: 03799501.6
(22) Anmeldetag: 19.12.2003
(51) Int. Cl.: C07K 14/47, C12N 15/11, A61K 38/17, G01N 33/50, A01K 67/027

(54) **VERWENDUNG DES MULTIFUNKTIONELLEN TRANSKRIPTIONSFAKTORS YIN-YANG-1 UND VARIANTEN DAVON ZUR BEHANDLUNG VON TYP-1 DIABETES**
USE OF THE MULTIFUNCTIONAL TRANSCRIPTION FACTOR YIN-YANG-1 AND VARIANTS THEREOF FOR TREATING TYPE 1 DIABETES
UTILISATION DU FACTEUR DE TRANSCRIPTION MULTIFONCTIONNEL YIN-YANG-1 ET SES VARIANTS POUR TRAITER LE DIABETE DE TYPE 1

(30) Priorität: 20.12.2002 DE 10261650
(43) Veröffentlichungstag der Anmeldung: 14.09.2005
(73) Patentinhaber: Ernst-Moritz-Arndt-Universität Greifswald, 17487 Greifswald (DE)
(72) Erfinder: KLÖTING, Ingrid, 17495 Lühmannsdorf (DE); KLÖTING, Nora, 17495 Lühmannsdorf (DE)
(74) Vertreter: UEXKÜLL & STOLBERG
(86) Internationale Anmeldenummer: PCT/EP2003/014762
(87) Internationale Veröffentlichungsnummer: WO 2004/056857

(56) Entgegenhaltungen:
- WO-A-93/04076
- WO-A-98/33067
- KLÖTING I ET AL: "Genes of SHR rats protect spontaneously diabetic BB/OK rats from diabetes: lessons from congenic BB.SHR rat strains." BIOCHEMICAL AND BIOPHYSICAL RESEARCH COMMUNICATIONS. 4 MAY 2001, Bd. 283, Nr. 2, 4. Mai 2001 (2001-05-04), Seiten 399-405, XP002293254 ISSN: 0006-291X in der Anmeldung erwähnt

### SEQUENZPROTOKOLL

<110> Ernst-Moritz-Arndt-Universität Greifswald
<120> Verwendung des multifunktionellen Transkriptionsfaktors Yin-Yang-1 und Varianten davon zur Behandlung von Erkrankungen, insbesondere von Typ-1 Diabetes
<130> P 62096
<160> 8
<170> PatentIn version 3.1
<210> 1
   <211> 2256
   <212> DNA
   <213> Rattus norv.
<220>
   <221> CDS
   <222> (73)..(1125)
   <223> YY1 (BB/OK)
<220>
   <221> misc_feature
   <222> (1759)..(1917)
   <223> Zinkfinger
<220>
   <221> misc_feature
   <222> (955)..(1125)
   <223> Zinkfinger
<220>
   <221> Intron
   <222> (1126)..(1758)
   <223>
<220>
   <221> promoter
   <222> (1)..(72)
   <223>
<220>
   <221> CDS
   <222> (1759)..(1938)
   <223> YY1 (BB/OK)
<400> 1
   ccgcctcctc gcccgccctc ccgcagccca ggagccgagg ctgccgcggc cgtggcggcg      60
   gagccctcag cc atg gcc tcg ggc gac acc ctc tac att gcc acg gac ggc     111
                 Met Ala Ser Gly Asp Thr Leu Tyr Ile Ala Thr Asp Gly
                 1               5                   10
   tcg gag atg cca gcc gag atc gtg gaa ctg cat gag att gag gtg gag       159
   Ser Glu Met Pro Ala Glu Ile Val Glu Leu His Glu Ile Glu Val Glu
       15                  20                  25
   acc atc ccg gtg gag act atc gag acc acg gtg gtg ggc gag gag gag       207
   Thr Ile Pro Val Glu Thr Ile Glu Thr Thr Val Val Gly Glu Glu Glu
   30                  35                  40                  45
   gac gac gac gaa gac gac gag gat ggt ggc ggc gga gac cac ggt ggc       255
   Asp Asp Asp Glu Asp Asp Glu Asp Gly Gly Gly Gly Asp His Gly Gly
                   50                  55                  60
   ggg ggc ggc cac ggg cac gct ggc cac cac cat cac cac cac cac cac       303
   Gly Gly Gly His Gly His Ala Gly His His His His His His His His
               65                  70                  75
   cac cac ccg ccc atg atc gcg ctg cag ccg ctg gtc acc gac gac ccg       351
   His His Pro Pro Met Ile Ala Leu Gln Pro Leu Val Thr Asp Asp Pro
           80                  85                  90
   acc caa gtg cac cac cac caa gag gtg att ctg gtg cag acg cgc gag       399
   Thr Gln Val His His His Gln Glu Val Ile Leu Val Gln Thr Arg Glu
       95                  100                 105
   gag gta gtg ggt ggc gac gac tcg gac ggg ctg cgc gcc gag gac ggg       447
   Glu Val Val Gly Gly Asp Asp Ser Asp Gly Leu Arg Ala Glu Asp Gly
   110                 115                 120                 125
   ttc gag gac cag atc ctc att ccg gta ccc gcg ccg gcc ggc gga gac       495
   Phe Glu Asp Gln Ile Leu Ile Pro Val Pro Ala Pro Ala Gly Gly Asp
                   130                 135                 140
   gac gac tac atc gag cag acg ctg gtc acc gtg gcg gcg gcc ggc aag       543
   Asp Asp Tyr Ile Glu Gln Thr Leu Val Thr Val Ala Ala Ala Gly Lys
               145                 150                 155
   agc ggt ggc ggg tct tcg tcg ggc ggc ggc cgc gtt aag aag ggc ggc       591
   Ser Gly Gly Gly Ser Ser Ser Gly Gly Gly Arg Val Lys Lys Gly Gly
           160                 165                 170
   ggc aag aag agc ggc aag aag agt tac ctg ggc agc ggg gcc ggc gcg       639
   Gly Lys Lys Ser Gly Lys Lys Ser Tyr Leu Gly Ser Gly Ala Gly Ala
       175                 180                 185
   gcg ggc ggt ggc ggc gcc gac ccg ggt aat aag aag tgg gaa cag aag       687
   Ala Gly Gly Gly Gly Ala Asp Pro Gly Asn Lys Lys Trp Glu Gln Lys
   190                 195                 200                 205
   cag gtg cag atc aag acc ctg gag ggc gag ttc tcg gtc acc atg tgg       735
   Gln Val Gln Ile Lys Thr Leu Glu Gly Glu Phe Ser Val Thr Met Trp
                   210                 215                 220
   tct tca gat gaa aaa aaa gat att gac cat gaa aca gtg gtt gaa gag       783
   Ser Ser Asp Glu Lys Lys Asp Ile Asp His Glu Thr Val Val Glu Glu
               225                 230                 235
   cag atc att ggg gag aac tca cct cct gat tat tct gaa tat atg aca      831
   Gln Ile Ile Gly Glu Asn Ser Pro Pro Asp Tyr Ser Glu Tyr Met Thr
           240                 245                 250
   ggc aag aaa ctc cct cct gga ggg ata cct ggc att gac ctc tca gac      879
   Gly Lys Lys Leu Pro Pro Gly Gly Ile Pro Gly Ile Asp Leu Ser Asp
       255                 260                 265
   ccc aag caa ctg gca gaa ttt gcc aga atg aag cca aga aaa att aaa      927
   Pro Lys Gln Leu Ala Glu Phe Ala Arg Met Lys Pro Arg Lys Ile Lys
   270                 275                 280                 285
   gaa gat gat gct cca aga aca ata gct tgc cct cat aaa ggc tgc aca      975
   Glu Asp Asp Ala Pro Arg Thr Ile Ala Cys Pro His Lys Gly Cys Thr
                   290                 295                 300
   aag atg ttc agg gat aac tct gct atg aga aag cat ctg cac acc cac     1023
   Lys Met Phe Arg Asp Asn Ser Ala Met Arg Lys His Leu His Thr His
               305                 310                 315
   ggt ccc aga gtc cac gtc tgt gca gaa tgt ggc aaa gcg ttc gtt gag     1071
   Gly Pro Arg Val His Val Cys Ala Glu Cys Gly Lys Ala Phe Val Glu
           320                 325                 330
   agc tca aag cta aaa cga cac cag ctg gtt cat act gga gaa aag ccc     1119
   Ser Ser Lys Leu Lys Arg His Gln Leu Val His Thr Gly Glu Lys Pro
       335                 340                 345
   ttt cag gtagagccag ttcctgttcc ccaaactgca agctagggtg ctggtcaggg      1175
   Phe Gln
   350
   tggttgatat caagcactat ggggcaccgg ttggggtatt ttattcccat ccctcctgtc   1235
   tgcttgggtt cctggttact gctcgggact gcaggtgtta cagatggggg tggagggatt   1295
   atgcgaagca cccccacact aaatttctag caggtttaca aaaactcaac agttttgttt   1355
   tgtagtgagt agtgtgttga attactgata gagtgcttat aagtgctgtt ggctacagct   1415
   ccaggtgaca cttggtgctg cttatagaag actcgtgagt tgacagttgg catcactaaa   1475
   tatcttaatc atctgtagtc tacttcctag agtgtctctg aaaacactca agctgtaaat   1535
   ttgcactcag cacagccctt ctgtttctca agaactagcc atgggttgtt agtatcagag   1595
   atcccagtgt gtcagttcta aaataccctc agaagggttc cagacgagga aggaggcatg   1655
   ctcagcagaa tagtaggtgg tttccatcta agcagtgagc catcgatccc caggttctgg   1715
   tctcatttgc caagagggtt gatatctggt ttttccttga cag tgc aca ttc gaa     1770
                                                   Cys Thr Phe Glu
                                                               355
   ggc tgc ggg aag cgc ttt tca ctg gac ttc aat ttg cgc acg cat gtg     1818
   Gly Cys Gly Lys Arg Phe Ser Leu Asp Phe Asn Leu Arg Thr His Val
                   360                 365                 370
   cga atc cat acc gga gac agg ccc tat gtg tgc ccc ttc gac ggt tgt      1866
   Arg Ile His Thr Gly Asp Arg Pro Tyr Val Cys Pro Phe Asp Gly Cys
               375                 380                 385
   aat aag aag ttt gct cag tca act aac ctg aaa tct cac atc tta aca      1914
   Asn Lys Lys Phe Ala Gln Ser Thr Asn Leu Lys Ser His Ile Leu Thr
           390                 395                 400
   cac gct aaa gcc aaa aac aac cag tgaaaagaag agagaagacc ttctcgaccc     1968
   His Ala Lys Ala Lys Asn Asn Gln
       405                 410
   cgggaagcct cttcaggagt gtgattggga ataaatatgc ctctcctttg tatattattt    2028
   ctaggaagaa ttttaaaaat gaatcctaca cacttaaggg acatgttttg ataaagtagt    2088
   aaaaatttaa aaaaatactt taataagatg acattgctaa gatgctctat cttgctctgt    2148
   aatctcgttt caaaaacaag gtgtttttgt aaagtgtggc cccaacagga ggacaattca    2208
   tgaacttcgc atcaaaagac aattctttat acaacagtgc taaaaatg                 2256
<210> 2
   <211> 411
   <212> PRT
   <213> Rattus norv.
<220>
   <221> misc_feature
   <222> (1759)..(1917)
   <223> Zinkfinger
<220>
   <221> misc_feature
   <222> (955)..(1125)
   <223> Zinkfinger
<400> 2
   Met Ala Ser Gly Asp Thr Leu Tyr Ile Ala Thr Asp Gly Ser Glu Met
   1               5                   10                  15
   Pro Ala Glu Ile Val Glu Leu His Glu Ile Glu Val Glu Thr Ile Pro
               20                  25                  30
   Val Glu Thr Ile Glu Thr Thr Val Val Gly Glu Glu Glu Asp Asp Asp
           35                  40                  45
   Glu Asp Asp Glu Asp Gly Gly Gly Gly Asp His Gly Gly Gly Gly Gly
       50                  55                  60
   His Gly His Ala Gly His His His His His His His His His His Pro
   65                  70                  75                  80
   Pro Met Ile Ala Leu Gln Pro Leu Val Thr Asp Asp Pro Thr Gln Val
   85 90 95
   His His His Gln Glu Val Ile Leu Val Gln Thr Arg Glu Glu Val Val
   100 105 110
   Gly Gly Asp Asp Ser Asp Gly Leu Arg Ala Glu Asp Gly Phe Glu Asp
   115 120 125
   Gln Ile Leu Ile Pro Val Pro Ala Pro Ala Gly Gly Asp Asp Asp Tyr
   130 135 140
   Ile Glu Gln Thr Leu Val Thr Val Ala Ala Ala Gly Lys Ser Gly Gly
   145 150 155 160
   Gly Ser Ser Ser Gly Gly Gly Arg Val Lys Lys Gly Gly Gly Lys Lys
   165 170 175
   Ser Gly Lys Lys Ser Tyr Leu Gly Ser Gly Ala Gly Ala Ala Gly Gly
   180 185 190
   Gly Gly Ala Asp Pro Gly Asn Lys Lys Trp Glu Gln Lys Gln Val Gln
   195 200 205
   Ile Lys Thr Leu Glu Gly Glu Phe Ser Val Thr Met Trp Ser Ser Asp
   210 215 220
   Glu Lys Lys Asp Ile Asp His Glu Thr Val Val Glu Glu Gln Ile Ile
   225 230 235 240
   Gly Glu Asn Ser Pro Pro Asp Tyr Ser Glu Tyr Met Thr Gly Lys Lys
   245 250 255
   Leu Pro Pro Gly Gly Ile Pro Gly Ile Asp Leu Ser Asp Pro Lys Gln
   260 265 270
   Leu Ala Glu Phe Ala Arg Met Lys Pro Arg Lys Ile Lys Glu Asp Asp
   275 280 285
   Ala Pro Arg Thr Ile Ala Cys Pro His Lys Gly Cys Thr Lys Met Phe
   290 295 300
   Arg Asp Asn Ser Ala Met Arg Lys His Leu His Thr His Gly Pro Arg
   305 310 315 320
   Val His Val Cys Ala Glu Cys Gly Lys Ala Phe Val Glu Ser Ser Lys
   325 330 335
   Leu Lys Arg His Gln Leu Val His Thr Gly Glu Lys Pro Phe Gln Cys
   340 345 350
   Thr Phe Glu Gly Cys Gly Lys Arg Phe Ser Leu Asp Phe Asn Leu Arg
   355 360 365
   Thr His Val Arg Ile His Thr Gly Asp Arg Pro Tyr Val Cys Pro Phe
   370 375 380
   Asp Gly Cys Asn Lys Lys Phe Ala Gln Ser Thr Asn Leu Lys Ser His
   385 390 395 400
   Ile Leu Thr His Ala Lys Ala Lys Asn Asn Gln
   405 410
<210> 3
   <211> 2256
   <212> DNA
   <213> Rattus norv.
<220>
   <221> CDS
   <222> (73)..(1125)
   <223> YY1 (SHR)
<220>
   <221> misc_feature
   <222> (1759)..(1917)
   <223> Zinkfinger
<220>
   <221> misc_feature
   <222> (955)..(1125)
   <223> Zinkfinger
<220>
   <221> Intron
   <222> (1126)..(1758)
   <223>
<220>
   <221> promoter
   <222> (1)..(72)
   <223>
<220>
   <221> CDS
   <222> (1759)..(1938)
   <223> YY1 (SHR)
<400> 3
   ccgcctcctc gcccgccctc ccgcagccca ggagccgagg ctgccgcggc cgtggcggcg      60
   gagccctcag cc atg gcc tcg ggc gac acc ctc tac att gcc acg gac ggc     111
                 Met Ala Ser Gly Asp Thr Leu Tyr Ile Ala Thr Asp Gly
                 1               5                   10
   tcg gag atg cca gcc gag atc gtg gaa ctg cat gag att gag gtg gag       159
   Ser Glu Met Pro Ala Glu Ile Val Glu Leu His Glu Ile Glu Val Glu
       15                  20                  25
   acc atc ccg gtg gag act atc gag acc acg gtg gtg ggc gag gag gag       207
   Thr Ile Pro Val Glu Thr Ile Glu Thr Thr Val Val Gly Glu Glu Glu
   30                  35                  40                  45
   gac gac gac gaa gac gac gag gat ggt ggc ggc gga gac cac ggt ggc       255
   Asp Asp Asp Glu Asp Asp Glu Asp Gly Gly Gly Gly Asp His Gly Gly
                   50                  55                  60
   ggg ggc ggc cac ggg cac gct ggc cac cac cat cac cac cac cac cac       303
   Gly Gly Gly His Gly His Ala Gly His His His His His His His His
               65                  70                  75
   cac cac ccg ccc atg atc gcg ctg cag ccg ctg gtc acc gac gac ccg       351
   His His Pro Pro Met Ile Ala Leu Gln Pro Leu Val Thr Asp Asp Pro
           80                  85                  90
   acc caa gtg cac cac cac caa gag gtg att ctg gtg cag acg cgc gag       399
   Thr Gln Val His His His Gln Glu Val Ile Leu Val Gln Thr Arg Glu
       95                  100                 105
   gag gta gtg ggt ggc gac gac tcg gac ggg ctg cgc gcc gag gac ggg       447
   Glu Val Val Gly Gly Asp Asp Ser Asp Gly Leu Arg Ala Glu Asp Gly
   110                 115                 120                 125
   ttc gag gac cag atc ctc att ccg gta ccc gcg ccg gcc ggc gga gac       495
   Phe Glu Asp Gln Ile Leu Ile Pro Val Pro Ala Pro Ala Gly Gly Asp
                   130                 135                 140
   gac gac tac atc gag cag acg ctg gtc acc gtg gcg gcg gcc ggc aag       543
   Asp Asp Tyr Ile Glu Gln Thr Leu Val Thr Val Ala Ala Ala Gly Lys
               145                 150                 155
   agc ggt ggc ggg tct tcg tcg ggc ggc ggc cgc gtt aag aag ggc ggc       591
   Ser Gly Gly Gly Ser Ser Ser Gly Gly Gly Arg Val Lys Lys Gly Gly
           160                 165                 170
   ggc aag aag agt ggc aag aag agt tac ctg ggc agc ggg gcc ggc gcg       639
   Gly Lys Lys Ser Gly Lys Lys Ser Tyr Leu Gly Ser Gly Ala Gly Ala
       175                 180                 185
   gcg ggc ggt ggc ggc gcc gac ccg ggt aat aag aag tgg gaa cag aag       687
   Ala Gly Gly Gly Gly Ala Asp Pro Gly Asn Lys Lys Trp Glu Gln Lys
   190                 195                 200                 205
   cag gtg cag atc aag acc ctg gag ggc gag ttc tcg gtc acc atg tgg      735
   Gln Val Gln Ile Lys Thr Leu Glu Gly Glu Phe Ser Val Thr Met Trp
                   210                 215                 220
   tct tca gat gaa aaa aaa gat att gac cat gaa aca gtg gtt gaa gag      783
   Ser Ser Asp Glu Lys Lys Asp Ile Asp His Glu Thr Val Val Glu Glu
               225                 230                 235
   cag atc att ggg gag aac tca cct cct gat tat tct gaa tat atg aca      831
   Gln Ile Ile Gly Glu Asn Ser Pro Pro Asp Tyr Ser Glu Tyr Met Thr
           240                 245                 250
   ggc aag aaa ctc cct cct gga ggg ata cct ggc att gac ctc tca gac      879
   Gly Lys Lys Leu Pro Pro Gly Gly Ile Pro Gly Ile Asp Leu Ser Asp
       255                 260                 265
   ccc aag caa ctg gca gaa ttt gcc aga atg aag cca aga aaa att aaa      927
   Pro Lys Gln Leu Ala Glu Phe Ala Arg Met Lys Pro Arg Lys Ile Lys
   270                 275                 280                 285
   gaa gat gat gct cca aga aca ata gct tgc cct cat aaa ggc tgc aca      975
   Glu Asp Asp Ala Pro Arg Thr Ile Ala Cys Pro His Lys Gly Cys Thr
                   290                 295                 300
   aag agg ttc agg gat aac tct gct atg aaa aag cat ctg cac acc cac     1023
   Lys Arg Phe Arg Asp Asn Ser Ala Met Lys Lys His Leu His Thr His
               305                 310                 315
   ggt ccc aga gtc cac gtc tgt gca gaa tgt ggc aaa gcg ttc gtt gag     1071
   Gly Pro Arg Val His Val Cys Ala Glu Cys Gly Lys Ala Phe Val Glu
           320                 325                 330
   agc tca aag cta aaa cga cac cag ctg gtt cat act gga gaa aag ccc     1119
   Ser Ser Lys Leu Lys Arg His Gln Leu Val His Thr Gly Glu Lys Pro
       335                 340                 345
   ttt cag gtagagccag ttcctgttcc ccaaactgca agctagggtg ctggtcaggg      1175
   Phe Gln
   350
   tggttgatat caagcactat ggggcaccgg ttggggtatt ttattcccat ccctcctgtc   1235
   tgcttgggtt cctggttact gctcgggact gcaggtgtta cagatggggg tggagggatt   1295
   atgcgaagca cccccacact aaatttctag caggtttaca aaaactcaac agttttgttt   1355
   tgtagtgagt agtgtgttga attactgata gagtgcttat aagtgctgtt ggctacagct   1415
   ccaggtgaca cttggtgctg cttatagaag acacgtgagt tgacagttgg catcactaaa   1475
   tatcttaatc atctgtagtc tacttcctag agtgtctctg aaaacactca agctgtaaat   1535
   ttgcactcag cacagccctt ctgtttctca agaactagcc atgggttgtt agtatcagag   1595
   atcccagtgt gtcagttcta aaataccctc acaagggttc cagacgagga aggaggcctg   1655
   ctcagcagaa tagtaggtgg tttccatcta agcagtgagc catcgatccc caggttctgg   1715
   tctcatttgc caagagggtt gatatctggt tttccttgga cag tgc aca ttc gaa     1770
                                                   Cys Thr Phe Glu
                                                               355
   ggc tgc ggg aag cgc ttt tca ctg gac ttc aat ttg cgc acg cat gtg      1818
   Gly Cys Gly Lys Arg Phe Ser Leu Asp Phe Asn Leu Arg Thr His Val
                   360                 365                 370
   cga atc cat acc gga gac agg ccc tat gtg tgc ccc ttc gac ggt tgt      1866
   Arg Ile His Thr Gly Asp Arg Pro Tyr Val Cys Pro Phe Asp Gly Cys
               375                 380                 385
   aat aag aag ttt gct cag tca act aac ctg aaa tct cac atc tta aca      1914
   Asn Lys Lys Phe Ala Gln Ser Thr Asn Leu Lys Ser His Ile Leu Thr
           390                 395                 400
   cac gct aaa gcc aaa aac aac cag tgaaaagaag agagaagacc ttctcgaccc     1968
   His Ala Lys Ala Lys Asn Asn Gln
       405                 410
   cgggaagcct cttcaggagt gtgattggga ataaatatgc ctctcctttg tatattattt    2028
   ctaggaagaa ttttaaaaat gaatcctaca cacttaaggg acatgttttg ataaagtagt    2088
   aaaaatttaa aaaaatactt taataagatg acattgctaa gatgctctat cttgctctgt    2148
   aatctcgttt caaaaacaag gtgtttttgt aaagtgtggt cccaacagga ggacaattca    2208
   tgaacttcgc atcaaaagac aattctttat acaacagtgc taaaaatg                 2256
<210> 4
   <211> 411
   <212> PRT
   <213> Rattus norv.
<220>
   <221> misc_feature
   <222> (1759)..(1917)
   <223> Zinkfinger
<220>
   <221> misc_feature
   <222> (955)..(1125)
   <223> Zinkfinger
<400> 4
   Met Ala Ser Gly Asp Thr Leu Tyr Ile Ala Thr Asp Gly Ser Glu Met
   1 5 10 15
   Pro Ala Glu Ile Val Glu Leu His Glu Ile Glu Val Glu Thr Ile Pro
   20 25 30
   Val Glu Thr Ile Glu Thr Thr Val Val Gly Glu Glu Glu Asp Asp Asp
   35 40 45
   Glu Asp Asp Glu Asp Gly Gly Gly Gly Asp His Gly Gly Gly Gly Gly
   50 55 60
   His Gly His Ala Gly His His His His His His His His His His Pro
   65 70 75 80
   Pro Met Ile Ala Leu Gln Pro Leu Val Thr Asp Asp Pro Thr Gln Val
   85 90 95
   His His His Gln Glu Val Ile Leu Val Gln Thr Arg Glu Glu Val Val
   100 105 110
   Gly Gly Asp Asp Ser Asp Gly Leu Arg Ala Glu Asp Gly Phe Glu Asp
   115 120 125
   Gln Ile Leu Ile Pro Val Pro Ala Pro Ala Gly Gly Asp Asp Asp Tyr
   130 135 140
   Ile Glu Gln Thr Leu Val Thr Val Ala Ala Ala Gly Lys Ser Gly Gly
   145 150 155 160
   Gly Ser Ser Ser Gly Gly Gly Arg Val Lys Lys Gly Gly Gly Lys Lys
   165 170 175
   Ser Gly Lys Lys Ser Tyr Leu Gly Ser Gly Ala Gly Ala Ala Gly Gly
   180 185 190
   Gly Gly Ala Asp Pro Gly Asn Lys Lys Trp Glu Gln Lys Gln Val Gln
   195 200 205
   Ile Lys Thr Leu Glu Gly Glu Phe Ser Val Thr Met Trp Ser Ser Asp
   210 215 220
   Glu Lys Lys Asp Ile Asp His Glu Thr Val Val Glu Glu Gln Ile Ile
   225 230 235 240
   Gly Glu Asn Ser Pro Pro Asp Tyr Ser Glu Tyr Met Thr Gly Lys Lys
   245 250 255
   Leu Pro Pro Gly Gly Ile Pro Gly Ile Asp Leu Ser Asp Pro Lys Gln
   260 265 270
   Leu Ala Glu Phe Ala Arg Met Lys Pro Arg Lys Ile Lys Glu Asp Asp
   275 280 285
   Ala Pro Arg Thr Ile Ala Cys Pro His Lys Gly Cys Thr Lys Arg Phe
   290 295 300
   Arg Asp Asn Ser Ala Met Lys Lys His Leu His Thr His Gly Pro Arg
   305 310 315 320
   Val His Val Cys Ala Glu Cys Gly Lys Ala Phe Val Glu Ser Ser Lys
   325 330 335
   Leu Lys Arg His Gln Leu Val His Thr Gly Glu Lys Pro Phe Gln Cys
   340 345 350
   Thr Phe Glu Gly Cys Gly Lys Arg Phe Ser Leu Asp Phe Asn Leu Arg
   355 360 365
   Thr His Val Arg Ile His Thr Gly Asp Arg Pro Tyr Val Cys Pro Phe
   370 375 380
   Asp Gly Cys Asn Lys Lys Phe Ala Gln Ser Thr Asn Leu Lys Ser His
   385 390 395 400
   Ile Leu Thr His Ala Lys Ala Lys Asn Asn Gln
   405 410
<210> 5
   <211> 1600
   <212> DNA
   <213> Homo sapiens
<220>
   <221> CDS
   <222> (43)..(1284)
   <223> YY1 (Human)
<400> 5
   gaattcggca cgagggcggc cgtggcggcg gagccctcag cc atg gcc tcg ggc 54
   Met Ala Ser Gly
   1
   gac acc ctc tac atc gcc acg gac ggc tcg gag atg ccg gcc gag atc 102
   Asp Thr Leu Tyr Ile Ala Thr Asp Gly Ser Glu Met Pro Ala Glu Ile
   5 10 15 20
   gtg gag ctg cat gag atc gag gtg gag acc atc ccg gtg gag acc atc 150
   Val Glu Leu His Glu Ile Glu Val Glu Thr Ile Pro Val Glu Thr Ile
   25 30 35
   gag acc acg gtg gtg ggc gag gag gag gag gag gac gac gac gac gag 198
   Glu Thr Thr Val Val Gly Glu Glu Glu Glu Glu Asp Asp Asp Asp Glu
   40 45 50
   gac ggc ggc ggc ggc gac cac ggc ggc ggc ggg ggc ggc cac ggg cac      246
   Asp Gly Gly Gly Gly Asp His Gly Gly Gly Gly Gly Gly His Gly His
           55                  60                  65
   gcc ggc cac cac cat cac cac cac cac cac cac cac cac cac ccg ccc      294
   Ala Gly His His His His His His His His His His His His Pro Pro
       70                  75                  80
   atg atc gcg ctg gag ccg ctg gtg acg gac gac ccg acc caa gtg cac      342
   Met Ile Ala Leu Glu Pro Leu Val Thr Asp Asp Pro Thr Gln Val His
   85                  90                  95                  100
   cac ctc cag gag gtg atc ctg gtg cag acg cgc gag gag gtc gtc ggg      390
   His Leu Gln Glu Val Ile Leu Val Gln Thr Arg Glu Glu Val Val Gly
                   105                 110                 115
   ggg gac gac tcg gac ggg ctg cgc gcc gag gac ggc ttc gag gac gag      438
   Gly Asp Asp Ser Asp Gly Leu Arg Ala Glu Asp Gly Phe Glu Asp Glu
               120                 125                 130
   atc ctc atc ccg gtg ccc gcg ccg gcc ggc ggc gac gac gac tac ata      486
   Ile Leu Ile Pro Val Pro Ala Pro Ala Gly Gly Asp Asp Asp Tyr Ile
           135                 140                 145
   gag cag acg ctg gtc acc gtg gcg gcg gcc ggc aag agc ggc ggc ggg      534
   Glu Gln Thr Leu Val Thr Val Ala Ala Ala Gly Lys Ser Gly Gly Gly
       150                 155                 160
   gcc tcg tcg ggc ggc ggt cgc gtg aag aag ggc ggc ggc aag aag agc      582
   Ala Ser Ser Gly Gly Gly Arg Val Lys Lys Gly Gly Gly Lys Lys Ser
   165                 170                 175                 180
   ggc aag aag agt tac ctg ggc ggc ggg gcc ggc gcg gcg ggc ggc ggc      630
   Gly Lys Lys Ser Tyr Leu Gly Gly Gly Ala Gly Ala Ala Gly Gly Gly
                   185                 190                 195
   ggc gcc gac ccg ggg aat aag aag tgg gag cag aag cag gtg cag atc      678
   Gly Ala Asp Pro Gly Asn Lys Lys Trp Glu Gln Lys Gln Val Gln Ile
               200                 205                 210
   aag acc ctg gag ggc gag tcc tcg gtc acc atg tgg tcc tcg gat gaa      726
   Lys Thr Leu Glu Gly Glu Ser Ser Val Thr Met Trp Ser Ser Asp Glu
           215                 220                 225
   aaa aaa gat att gac cat gaa aca gtg gtt gaa gag cag atc att gga      774
   Lys Lys Asp Ile Asp His Glu Thr Val Val Glu Glu Gln Ile Ile Gly
       230                 235                 240
   gag aac tca cct cct gat tat tct gaa tat atg aca ggc aag aaa ctc      822
   Glu Asn Ser Pro Pro Asp Tyr Ser Glu Tyr Met Thr Gly Lys Lys Leu
   245                 250                 255                 260
   cct cct gga ggg ata cct ggc att gac ctc tca gac cct aag caa ctg      870
   Pro Pro Gly Gly Ile Pro Gly Ile Asp Leu Ser Asp Pro Lys Gln Leu
                   265                 270                 275
   gca gaa ttt gcc aga atg aag cca aga aaa att aaa gaa gat gat gct      918
   Ala Glu Phe Ala Arg Met Lys Pro Arg Lys Ile Lys Glu Asp Asp Ala
               280                 285                 290
   cca aga aca ata gct tgc cct cat aaa ggc tgc aca aag atg ttc agg      966
   Pro Arg Thr Ile Ala Cys Pro His Lys Gly Cys Thr Lys Met Phe Arg
           295                 300                 305
   gat aac tct gct atg aga aag cat ctg cac acc cac ggt ccc aga gtc     1014
   Asp Asn Ser Ala Met Arg Lys His Leu His Thr His Gly Pro Arg Val
       310                 315                 320
   cac gtc tgt gca gag tgt ggc aaa gcg ttc gtt gag agc tca aag cta     1062
   His Val Cys Ala Glu Cys Gly Lys Ala Phe Val Glu Ser Ser Lys Leu
   325                 330                 335                 340
   aaa cga cac cag ctg gtt cat act gga gaa aag ccc ttt cag tgc aca     1110
   Lys Arg His Gln Leu Val His Thr Gly Glu Lys Pro Phe Gln Cys Thr
                   345                 350                 355
   ttc gaa ggc tgc ggg aag cgc ttt tca ctg gac ttc aat ttg cgc aca     1158
   Phe Glu Gly Cys Gly Lys Arg Phe Ser Leu Asp Phe Asn Leu Arg Thr
               360                 365                 370
   cat gtg gga atc cat acc gga gac agg ccc tat gtg tgc ccc ttc gac     1206
   His Val Gly Ile His Thr Gly Asp Arg Pro Tyr Val Cys Pro Phe Asp
           375                 380                 385
   ggt tgt aat aag aag ttt gct cag tca act aac ctg aaa tct cac atc     1254
   Gly Cys Asn Lys Lys Phe Ala Gln Ser Thr Asn Leu Lys Ser His Ile
       390                 395                 400
   tta aca cac gct aaa gcc aaa aac aac cag tgaaaagaag agagaagacc       1304
   Leu Thr His Ala Lys Ala Lys Asn Asn Gln
   405                 410
   ttctcgaccc gggaagcctc ttcaggagtg agattgggaa taaatatgcc tctcctttgt   1364
   atattatttc taggaagaat tttaaaaatg aatcctacac acttaaggga catgttttga   1424
   taaagtagta aaaatttaaa aaatacttta ataagatgac attgctaaga tgctatatct   1484
   tgctctgtaa tctcgtttca aaaacaaggt gtttttgtaa agtgtggtcc caacaggagg   1544
   acaattcatg aacttcgcat caaaagacaa ttctttatac aacagtgcta aaaatg       1600
<210> 6
   <211> 414
   <212> PRT
   <213> Homo sapiens
<400> 6
   Met Ala Ser Gly Asp Thr Leu Tyr Ile Ala Thr Asp Gly Ser Glu Met
   1 5 10 15
   Pro Ala Glu Ile Val Glu Leu His Glu Ile Glu Val Glu Thr Ile Pro
   20 25 30
   Val Glu Thr Ile Glu Thr Thr Val Val Gly Glu Glu Glu Glu Glu Asp
   35 40 45
   Asp Asp Asp Glu Asp Gly Gly Gly Gly Asp His Gly Gly Gly Gly Gly
   50 55 60
   Gly His Gly His Ala Gly His His His His His His His His His His
   65 70 75 80
   His His Pro Pro Met Ile Ala Leu Glu Pro Leu Val Thr Asp Asp Pro
   85 90 95
   Thr Gln Val His His Leu Gln Glu Val Ile Leu Val Gln Thr Arg Glu
   100 105 110
   Glu Val Val Gly Gly Asp Asp Ser Asp Gly Leu Arg Ala Glu Asp Gly
   115 120 125
   Phe Glu Asp Glu Ile Leu Ile Pro Val Pro Ala Pro Ala Gly Gly Asp
   130 135 140
   Asp Asp Tyr Ile Glu Gln Thr Leu Val Thr Val Ala Ala Ala Gly Lys
   145 150 155 160
   Ser Gly Gly Gly Ala Ser Ser Gly Gly Gly Arg Val Lys Lys Gly Gly
   165 170 175
   Gly Lys Lys Ser Gly Lys Lys Ser Tyr Leu Gly Gly Gly Ala Gly Ala
   180 185 190
   Ala Gly Gly Gly Gly Ala Asp Pro Gly Asn Lys Lys Trp Glu Gln Lys
   195 200 205
   Gln Val Gln Ile Lys Thr Leu Glu Gly Glu Ser Ser Val Thr Met Trp
   210 215 220
   Ser Ser Asp Glu Lys Lys Asp Ile Asp His Glu Thr Val Val Glu Glu
   225 230 235 240
   Gln Ile Ile Gly Glu Asn Ser Pro Pro Asp Tyr Ser Glu Tyr Met Thr
   245 250 255
   Gly Lys Lys Leu Pro Pro Gly Gly Ile Pro Gly Ile Asp Leu Ser Asp
   260 265 270
   Pro Lys Gln Leu Ala Glu Phe Ala Arg Met Lys Pro Arg Lys Ile Lys
   275 280 285
   Glu Asp Asp Ala Pro Arg Thr Ile Ala Cys Pro His Lys Gly Cys Thr
       290                 295                 300
   Lys Met Phe Arg Asp Asn Ser Ala Met Arg Lys His Leu His Thr His
   305                 310                 315                 320
   Gly Pro Arg Val His Val Cys Ala Glu Cys Gly Lys Ala Phe Val Glu
                   325                 330                 335
   Ser Ser Lys Leu Lys Arg His Gln Leu Val His Thr Gly Glu Lys Pro
               340                 345                 350
   Phe Gln Cys Thr Phe Glu Gly Cys Gly Lys Arg Phe Ser Leu Asp Phe
           355                 360                 365
   Asn Leu Arg Thr His Val Gly Ile His Thr Gly Asp Arg Pro Tyr Val
       370                 375                 380
   Cys Pro Phe Asp Gly Cys Asn Lys Lys Phe Ala Gln Ser Thr Asn Leu
   385                 390                 395                 400
   Lys Ser His Ile Leu Thr His Ala Lys Ala Lys Asn Asn Gln
                   405                 410
<210> 7
   <211> 1080
   <212> DNA
   <213> Rattus norv.
<220>
   <221> CDS
   <222> (883)..(894)
   <223> Verkürzter Zinkfinger (BB.6S)
<220>
   <221> CDS
   <222> (898)..(1056)
   <223> Verkürzter Zinkfinger (BB.6S)
<400> 7
   atggcctcgg gcgacaccct ctacattgcc acggacggct cggagatgcc agccgagatc      60
   gtggaactgc atgagattga ggtggagacc atcccggtgg agactatcga gaccacggtg     120
   gtgggcgagg aggaggacga cgacgaagac gacgaggatg gtggcggcgg agaccacggt     180
   ggcgggggcg gccacgggca cgctggccac caccatcacc accaccacca ccaccacccg     240
   cccatgatcg cgctgcagcc gctggtcacc gacgacccga cccaagtgca ccaccaccaa    300
   gaggtgattc tggtgcagac gcgcgaggag gtagtgggtg gcgacgactc ggacgggctg    360
   cgcgccgagg acgggttcga ggaccagatc ctcattccgg tacccgcgcc ggccggcgga    420
   gacgacgact acatcgagca gacgctggtc accgtggcgg cggccggcaa gagcggtggc    480
   gggtcttcgt cgggcggcgg ccgcgttaag aagggcggcg gcaagaagag cggcaagaag    540
   agttacctgg gcagcggggc cggcgcggcg ggcggtggcg gcgccgaccc gggtaataag    600
   aagtgggaac agaagcaggt gcagatcaag accctggagg gcgagttctc ggtcaccatg    660
   tggtcttcag atgaaaaaaa agatattgac catgaaacag tggttgaaga gcagatcatt    720
   ggggagaact cacctcctga ttattctgaa tatatgacag gcaagaaact ccctcctgga    780
   gggatacctg gcattgacct ctcagacccc aagcaactgg cagaatttgc cagaatgaag    840
   ccaagaaaaa ttaaagaaga tgatgctcca agaacaatag ct tgc cct cat aaa       894
                                                 Cys Pro His Lys
                                                 1
   cag tgc aca ttc gaa ggc tgc ggg aag cgc ttt tca ctg gac ttc aat       942
       Cys Thr Phe Glu Gly Cys Gly Lys Arg Phe Ser Leu Asp Phe Asn
       5                   10                  15
   ttg cgc acg cat gtg cga atc cat acc gga gac agg ccc tat gtg tgc       990
   Leu Arg Thr His Val Arg Ile His Thr Gly Asp Arg Pro Tyr Val Cys
   20                  25                  30                  35
   ccc ttc gac ggt tgt aat aag aag ttt gct cag tca act aac ctg aaa      1038
   Pro Phe Asp Gly Cys Asn Lys Lys Phe Ala Gln Ser Thr Asn Leu Lys
                   40                  45                  50
   tct cac atc tta aca cac gctaaagcca aaaacaacca gtga                   1080
   Ser His Ile Leu Thr His
               55
<210> 8
   <211> 57
   <212> PRT
   <213> Rattus norv.
<400> 8
   Cys Pro His Lys Cys Thr Phe Glu Gly Cys Gly Lys Arg Phe Ser Leu
   1               5                   10                  15
   Asp Phe Asn Leu Arg Thr His Val Arg Ile His Thr Gly Asp Arg Pro
               20                  25                  30
   Tyr Val Cys Pro Phe Asp Gly Cys Asn Lys Lys Phe Ala Gln Ser Thr
           35                  40                  45
   Asn Leu Lys Ser His Ile Leu Thr His
       50                  55

## Beschreibung

Die vorliegende Erfindung betrifft die Beeinflussung der Aktivität und/oder der Expression des multifunktionellen Transkriptionsfaktors Yin-Yang-1 (YY1) und Varianten davon zur Behandlung verschiedenster Erkrankungen. Die Erfindung betrifft insbesondere eine mutierte Nukleinsäuresequenz, die für eine Variante des humanen YY1 kodiert und der erfindungsgemäß eine diabetesprotektive Wirkung zugeschrieben wird.

### Einführung

Der insulinabhängige Typ-1-Diabetes (IDDM- Insulin-Dependent Diabetes Mellitus) ist eine komplexe Erkrankung, bei der zellüläre und humorale Immunprozesse ablaufen, die eine Zerstörung der körpereigenen Insulin-produzierenden Beta-Zellen zur Folge haben. Man spricht daher beim Typ-1-Diabetes von einer Autoimmunerkrankung. Es besteht heute kein Zweifel mehr, daß der Typ-1-Diabetes genetisch determiniert ist. Gesichert ist, daß bestimmte Klasse-II-Gene des Haupthistokompatibilitätskomplexes (MHC), der beim Menschen als HLA (Human Leucocyte Antigens) bezeichnet wird, an der Entstehung des Typ-1-Diabetes beteiligt, aber allein nicht ausreichend sind. Diese diabetessuszeptiblen Klasse-II-Gene, die als IDDM1 bezeichnet werden, erklären nur etwa 40% des Risikos, an einem Typ-1-Diabetes zu erkranken. Daher wurde in den letzten Jahren versucht, chromosomale Regionen mit diabetogenen Nicht-MHC-Genen zu lokalisieren. Im Ergebnis dieser Studien wurden bis dato mehr als 17 Nicht-MHC-Gene (IDDM2, IDDM3, IDDM4 etc.), die auf verschiedenen Chromosomen kartieren, beschrieben. Mit Ausnahme des IDDM2 und IDDM18, bei denen das diabetogene Gen bekannt ist, sind bei den anderen IDDM's die entsprechenden Gene immer noch unbekannt (1,2).

Bei der Identifikation dieser Nicht-MHC-Gene sind Modelltiere, die ähnlich dem Menschen einen Typ-1-Diabetes entwickeln, sehr hilfreich. Als Modelltier für den Typ-1-Diabetes ist die spontan diabetische BB/O(ttawa)K(arlsburg)-Ratte ausgezeichnet geeignet, da dieses Modelltier sowohl klinisch, als auch ätiopathogenetisch ein hohes Maß an Analogien zum menschlichen Typ-1-Diabetes aufweist (3-7). Auch bei der BB/OK-Ratte sind die Klasse-II-Gene des MHC essentiell aber nicht ausreichend für die Diabetesentwicklung. Die BB/OK-Ratte ist homozygot für den MHC-Haplotyp RT1^{u}. Analog dem Menschen wird dieses diabetogene Gen als Iddm1 bezeichnet. Neben diesem Genkomplex ist bei der BB/OK-Ratte allerdings noch ein weiteres Gen, Iddm2, für die Diabetesentwicklung erforderlich. Es handelt sich dabei um ein Gen (1), daß eine Lymphopenie verursacht, rezessiv vererbt und als Iddm2 bezeichnet wird. Daß beide diabetogenen Gene, Iddm1 und Iddm2, zwar essentiell aber nicht ausreichend sind, wurde durch verschiedene Kreuzungsstudien unter Nutzung diabetischer BB/OK-Ratten und verschiedener diabetesresistenter Rattenstämme belegt (8-11). Gleichgültig, ob diabetesresistente LEW.1A, DA, SHR oder wilde Ratten (12) mit diabetischen BB/OK-Ratten gekreuzt wurden, entwickelte kein F1-Hybrid und nur etwa 50% der für beide diabetogenen Gene Iddm1 und Iddm2 bereits homozygoten ersten Rückkreuzungshybriden (R1) einen Typ-1-Diabetes. Der Prozentsatz von 50% an diabetischen Tieren bei den für Iddm1 und Iddm2 bereits homozygoten R1-Hybriden weist darauf hin, daß mindestens ein weiteres diabetogenes Gen, Iddm3, für die Entwicklung eines Diabetes bei BB/OK-Ratten notwendig ist. Die genomweite Suche nach diesem dritten Gen in zwei Kreuzungspopulationen, [(BB x DA)F1 x BB] und [(BB x SHR)F1 x BB], zeigte, daß dieses "dritte Gen" nicht nur ein Gen ist. Es wurden zwei diabetogene Gene, Iddm3 und Iddm4, auf Chromosom 18 bzw. 6 und ein Diabetes-protektives Gen, Iddm5r, auf Chromosom 1 kartiert (13,14). Während Iddm3 in beiden Kreuzungspopulationen nachgewiesen und damit bestätigt wurde (13), waren Iddm4 und Iddm5r nur bei [(BB x SHR)F1 x BB] R1-Hybriden nachweisbar (14).

Vergleicht man die homologen Bereiche zwischen Ratte und Mensch, zeigt sich, daß beim Menschen die homologen Regionen für Iddm3 auf 18q21-q23, Iddm4 auf 14q24-q32 und Iddm5r auf 11p15 kartieren. Es handelt sich dabei um Bereiche, bei denen auch beim Menschen die diabetogenen Gene IDDM6 auf 18q21-23 (Iddm3), IDDM11 auf 14q24-q32 (Iddm4) und IDDM2 auf 11p15 (Iddm5r) kartiert wurden (15-17).

Aufgabe der vorliegenden Erfindung ist es daher zunächst, die diabetogenen Gene zu identifizieren und therapeutische Ansätze zur Verhinderung der Typ-1-Diabetes zu formulieren.

Im Rahmen der Erfindung wurde überraschenderweise ein Gen identifiziert, das im diabetesprotektiven chromosomalen Bereich liegt, dessen mutierte Varianten geeignet sind, im Rattenmodell Typ-1-Diabetes zu verhindern. Infolge dieser Erkenntnis steht damit erstmals ein wichtiger therapeutischer Ansatz für Diagnose und Präventivtherapie von Typ-1-Diabetes zur Verfügung. Darüber hinaus ergeben sich durch weitere, erfindungsgemäß gewonnene Erkenntnisse vielfältige zusätzliche Anwendungsmöglichkeiten, die nachfolgend ebenfalls beschrieben werden.

### Vorarbeiten

Um zu prüfen, welche Bedeutung die kartierten Iddm's der BB/OK-Ratte tatsächlich bei den Entstehung eines Typ-1-Diabetes haben, wurden verschiedene kongene BB/OK-Rattenlinien etabliert.

Neben den bereits beschriebenen kongenen BB.SHR-Linien (BB.1K, BB.Sa, BB.LL, BB.Bp2, BB.Xs) wurden zwei weitere BB.SHR-Linien etabliert (18-24). Es wurde ein Bereich von Chromosom 6 (D6Rat184 - Iddm4 - D6Rat3, ca.15 cM) und einer von 18 (Olf - Iddm3 -D18Rat44, ca.24 cM) der BB/OK-Ratte durch den der SHR-Ratten ersetzt (24). Die phänotypische Charakterisierung dieser neu etablierten BB-Linien, kurz als BB.6S und BB.18S bezeichnet, zeigte, daß die Diabetesinzidenz in beiden Kongenen gesenkt werden konnte, besonders allerdings bei der kongenen BB.6S-Linie (24). Während ca. 86% aller BB/OK-Ratten einen Typ-1-Diabetes bis zur 32. Lebenswoche entwickeln, erkrankten im gleichen Zeitraum in der neu etablierten BB.6S-Linie nur 14% und das, obwohl die BB.6S-Tiere homozygot für Iddm1 und Iddm2 sind und im Restgenom der BB/OK-Ratte entsprechen, wie durch einen genomweite Analyse bestätigt wurde (24). D.h. **die Wirkung beider essentiellen diabetogenen Gene,** Iddm1 und Iddm2, wird durch ein oder mehr Gen/e auf dem transferierten Bereich der diabetes-resistenten SHR-Ratte **nahezu vollständig unterdrückt**. Ein Ergebnis, welches bis dato einmalig ist. Selbst bei der N(on)O(besen)D(iabetischen)-Maus, auch ein Modell für den Typ-1-Diabetes, wurden diabetogene Gene kartiert und analog kongene NOD-Linien etabliert, um den in vivo-Effekt dieser Gene zu prüfen. Eine drastische Senkung der Diabetesinzidenz wurde durch Austausch eines diabetogenen chromosomalen Bereiches wie bei BB.6S bis dato nicht beschrieben. Eine ähnlich deutliche Senkung der Diabetesinzidenz wie bei BB.6S wurde nur durch Austausch von 2 und mehr chromosomalen Regionen der NOD durch die diabetes-resistenten Stämme erreicht (25-28).

Unterschiede zwischen BB/OK- und BB.6S-Ratten wurden auch beim Manifestationsalter und bei den Lymphozytenphänotypen nachgewiesen. BB.6S-Ratten manifestieren signifikant später (137 ± 14 vs. 103 ± 30 Tage, p<0,001) und haben signifikant weniger aktivierte T-Lymphotzyten als BB/OK-Ratten (36,6 ± 6,9 vs. 65,6 ± 18,4 %, p<0,0001) (24). Darüber hinaus werden BB.6S-Ratten signifikant schwerer und haben signifikant höhere Serumcholesterolwerte als BB/OK-Ratten (24).

Da auch bei der SHR-Ratte immunologische Phänomene beschrieben wurden (29-31), wurde die drastische Senkung der Diabetesinzidenz von 86% bei BB/OK auf 14% bei BB.6S zunächst in der Weise interpretiert, daß in der transferierten Region ein SHR-Gen oder SHR-Gene lokalisiert ist/sind, dessen/deren Produkt/e die Autoaggression der diabetogenen Genprodukte der BB-Ratte weitgehend "neutralisieren" kann/können. Diese Annahme wurde durch die Etablierung einer weiteren kongenen BB/OK-Rattenline unterstrichen.

Neben diabetesresistenten SHR-Ratten wurden auch Wildfangratten zur Erzeugung von kongenen BB.K(arlsburg)W(ild)R(atte)-Ratten verwendet. Analog der BB.6S-Linie wurde der gleiche chromosomale Bereich der BB/OK von 15 cM durch den von Wildfangtieren ausgetauscht (BB.6W). Die Erkrankungshäufigkeit in dieser kongenen BB.6W-Linie ist mit der der BB/OK-Ratte vergleichbar (89% vs. 86%, 32). Daher wird erfindungsgemäß davon ausgegangen, daß die Diabetes-Protektion bei BB.6S das Ergebnis einer "neutralisierenden" Genwirkung der SHR-Ratte und nicht dem Ersatz von Iddm4 der BB/OK-Ratte anzulasten ist.

Die Identifikation der/des Gene/s war somit angezeigt, denn mit der Identifikation des/der Gens/Gene ist die Möglichkeit verbunden, eine genspezifische Behandlung und/oder Prävention des Typ-1-Diabetes bzw. von Autoimmunerkrankungen per se vornehmen zu können.

### Fortführende Arbeiten zur Identifikation des/der diabetesprotektiven Gens/Gene

Nach der Entschlüsselung des menschlichen Genoms (ca. 40.000 Gene) geht man heute davon aus, daß etwa 15-20 Gene pro cM und nicht wie bisher angenommen 50 Gene lokalisiert sind. Ausgehend von dieser Annahme sollten in dem transferierten chromosomalen Bereich der BB.6S-Ratte von ca. 15 cM etwa 225-300 Gene kartieren. Angesichts dieser Zahl ist die Identifikation von möglichen Kandidatengenen für eine Diabetesprotektion ein aussichtsloses Unterfangen, weshalb subkongene BB.6S-Ratten erzeugt wurden, um den chromosomalen Bereich mit der diabetesprotektiven Wirkung weiterhin einzugrenzen.

BB.6S-Ratten wurden mit diabetischen BB/OK-Ratten gekreuzt. Die resultierenden F1-Hybriden für den Bereich auf Chromosom 6 wurden untereinander gepaart (intercross) und genetisch analysiert, wobei das Markerspekrum erweitert wurde, um möglichst Rekombinationen in kleinen Bereiche erkennen zu können: D6Rat3-D6Rat1-D6wox13-D6Rat101-Ighe/Ckb-D6Mgh2-D6Rat94-D6Rat183-D6Rat10-D6Rat7-D6Rat75-D6Rat9-D6Rat6-D6Rat160-D6Mgh9-D6Rat184.

Die Zuordnung der Linien mit Diabetesprotektion erfolgte nach der Erkrankungshäufigkeit. Erkrankten bereits mehr als 50% der Nachkommen einer subkongenen Linie vor dem 100. Lebenstag, sprach dieser Phänotyp für den der BB/OK-Ratte und die Linie wurde eliminiert. Erkrankten weniger als 15% bis zum 100. Lebenstag, zeigte diese Linie den Phänotyp von BB.6S. Diese Linien wurden für die Weiterzucht zwecks Minimierung der Diabetes-protektiven Region eingesetzt. Durch die Etablierung verschiedener subkongener Linien (BB.6Sa,b,c..) mit dem Phänotyp der BB.6S-Ratte, wurde der in Frage kommende chromosomale Bereich auf < 2 cM ( 30-40 Gene) eingegrenzt.

Wie nachfolgend dargestellt, kartiert das diabetesprotektive Gen um den Locus D6Mgh2.

**Tabelle 1:**

| cM | | BB.6S | BB.6a | BB.6b | BB.6c | BB.6d | BB.6e | BB.6f |
|---|---|---|---|---|---|---|---|---|
| | D6Mgh4 | BB | BB | BB | BB | BB | BB | BB |
| 2.1 | D6Rat13 | BB | BB | BB | BB | BB | BB | BB |
| 4.4 | D6Wox5 | BB | BB | BB | BB | BB | BB | BB |
| 1.5 | D6Rat66 | BB | BB | BB | BB | BB | BB | BB |
| 2.0 | D6Rat184/D6M gh9 | SHR | BB | BB | BB | BB | BB | SHR |
| 0.8 | D6Rat160 | SHR | BB | BB | BB | BB | BB | SHR |
| 1.3 | D6Rat6 | SHR | BB | BB | BB | BB | BB | SHR |
| | D6Rat9 | SHR | BB | BB | BB | BB | BB | SHR |
| 0.4 | D6Rat75 | SHR | | | | | | |
| 0.4 | D6Rat7 | SHR | BB | BB | BB | BB | BB | SHR |
| 2.1 | D6Rat10 | SHR | BB | BB | BB | BB | BB | SHR |
| 1.6 | D6Rat183 | SHR | BB | SHR | BB | BB | BB | SHR |
| 0.4 | D6Rat94 | SHR | BB | SHR | BB | BB | BB | SHR |
| 1.9 | D6Mgh2 | SHR | SHR | SHR | BB | BB | BB | SHR |
| | Ighe | SHR | SHR | BB | SHR | SHR | SHR | SHR |
| 1.7 | Ckb/D6Rat101 | SHR | SHR | BB | SHR | SHR | BB | SHR |
| 1.0 | D6Rat1 | SHR | SHR | BB | BB | BB | SHR | BB |
| 1.9 | D6Rat3 | SHR | SHR | BB | BB | SHR | SHR | BB |
| | | | | | | | | |
| | Diabetesinzidenz | 15% | 10% | 22% | >50% | >50% | >50% | 14% |

Der Locus D6Mgh2 ist ein Mikrosatellit im Ef1-Gen. Ef1 ist ein Pseudogen und kartiert beim Menschen auf Chromosom 14q32 und bei der Maus auf Chromosom 12q14. Nach Angaben der Chromosomenkarte von Watanabe et al. (33) soll auf Chromosom 6q32 der Ratte auch das Gen Macs kartieren (http://ratmap.ims.u-tokyo.ac.jp). Dieses Gen ist aber beim Menschen auf Chromosom 6q21-6q22.2 und nicht auf 14q32 lokalisiert worden. Falls dies zutreffen würde, bedeutet es, daß 6q32 der Ratte sowohl homolog zum humanen Chromosom 14q32, als auch 6q21-6q22.2 sein kann. Zwecks Kandidatengensuche war es notwendig, abzuklären, ob Macs tatsächlich auf dem Rattenchromosom 6q32 kartiert. Durch einen Polymorphismus im Macs-Gen (zwischen 901 und 1321, KWR hat 1 Aminosäure mehr) zwischen BB/OK und Wildfangtieren (KWR) wurden die Sequenzen zwischen BB/OK, KWR und BB.KWR (Chr. 6; BB.6W) verglichen. Alle BB.6W-Tiere zeigten den Genotyp der BB/OK-Ratte. Dadurch konnte ausgeschlossen werden, daß Macs auf Chromosom 6q32 der Ratte lokalisiert ist und damit ein Kartierungsfehler vorliegt. Dadurch konnte die Kandidatengensuche durch Homologievergleiche auf 12q13 der Maus und 14q32 des Menschen beschränkt werden.

Da im diabetesprotektivem Bereich auch das Gen Aktl/Pkb kartiert, welches essentiell für die β-Zellfunktion ist (34, 35), wurde zunächst versucht, Aktl/Pkb relativ genau in der Region zu positionieren. Da BB/OK und SHR polymorph sind (Intron zwischen 1321 und 1561), konnte mit Hilfe der subkongenen BB.6S-Linien Aktl/PKB zwischen Ighe und Ckb, also außerhalb der diabetesprotektiven Region, kartiert werden. Damit schied dieses Gen als möglicher Kandidat aus.

Durch die parallel laufende in vivo-Charakterisierung der BB.6S-Ratten im Vergleich zum Parentalstamm BB/OK wurden weitere Erkenntnisse zur Funktion des möglichen Kandidatengens gewonnen:
1. Morphologische Untersuchungen des Pankreas von normoglykämischen BB/OK- und BB.6S-Ratten zeigten signifikante Unterschiede auf. Im Alter von 30 Tagen waren bei BB.6S signifikant mehr Langerhanssche Inseln mit Lymphozyten infiltriert (Insulitis) als bei BB/OK. Bei jeweils 12 untersuchten BB.6S bzw. BB/OK waren 51,2 ± 4,6% bzw. 7,5 ± 2,5 % (p<0,001) der Inseln infiltriert. Im Alter von 90 Tagen war der Prozentsatz an Inseln mit Insulitis zwischen beiden Stämmen vergleichbar und lag bei etwa 50% (37). Demnach tritt sowohl bei BB/OK als auch bei BB.6S eine Insulitis auf, die bei BB/OK bei etwa 86% der Tiere und bei BB.6S nur bei etwa 14% zur Zerstörung der Beta-Zellen und damit zum Typ-1-Diabetes führt, was für die Induktion einer Immuntoleranz bei BB.6S sprechen könnte.
   Signifikante Unterschiede fanden sich auch zwischen frischmanifestierten, diabetischen BB/OK und BB.6S im Insulingehalt und Prozentsatz Insulin-positiver β-Zellen. Im Vergleich zu diabetischen BB/OK war der Insulingehalt (0,15 ± 0,03 vs. 0,42 ± 0.13 pmol/mg, p<0,05) und der Prozentsatz Insulin-positiver β-Zellen (0,07 ± 0,02 vs. 0,19 ± 0,06%, p<0,05) signifikant niedriger als bei diabetischen BB.6S, was dafür sprechen könnte, daß die Zerstörung der Insulin-produzierenden β-Zellen nicht so aggressiv wie bei BB/OK-Ratten abläuft (37).
2. Untersuchungen zur Frakturheilung bei BB/OK- und diabetes-resistenten LEW.1A-Ratten (38) zeigten, daß der Knochen der BB/OK-Ratte deutlich brüchiger als der von LEW.1A-Ratten war, was auf eine mögliche Störung im Calcium-Stoffwechsel der BB/OK hinwies. Verschiedene Untersuchungen zum Calcium-Stoffwechsel ergaben allein signifikante Unterschiede im Serumcalcitoningehalt zwischen BB/OK und BB.6S-Ratten. Signifikant höhere Calcitonwerte wurden bei BB/OK als bei BB.6S nachgewiesen (2,4 ± 1,57 vs. 1,0 ± 0,65 pmol/ml; p=0,0002). Da Calcitonin nicht nur die Calcium-Freisetzung des Knochens hemmt, sondern ebenso über verschiedene Proteine und Rezeptoren die Insulinsekretion hemmen kann (39,40), wurde versucht, durch Modulation der Calcium-Zufuhr über die Nahrung von BB/OK und BB.6S einen weiteren Hinweis zum Zusammenspiel von Calcium (Ca) und Diabetesmanifestation zu erhalten.
   BB/OK und BB.6S-Ratten wurden mit Standarddiät (0,8% Ca), mit Ca-reicher (2%) und Ca-armer Diät (0,4%) ernährt und bis zur 30. Lebenswoche auf Diabetesentwicklung beobachtet. Während bei BB/OK-Ratten kein Einfluß der Diät auf die Erkrankungshäufigkeit nachweisbar war (Kontrolle=88%; Ca-arm=92%; Ca-reich=90%), erkrankten bei BB.6S-Ratten signifikant mehr Tiere an einem Diabetes, wenn sie mit Ca-reicher Diät ernährt wurden (Kontrolle=12%; Ca-arm=18%; Ca-reich= 45%, p=0.02). Darüber hinaus führte die Ca-reiche Ernährung zur Angleichung der Körpermasse und des Serumcholsterolgehaltes bei BB/OK und BB.6S. Signifikante Unterschiede zwischen beiden Linien traten bis zur 30. Lebenswoche nicht auf. Weitere in vivo-Manipulationen, wie Verabreichung von Ca-Kanal-Blockern oder verschiedenen Ca-Antagonisten, ergab keinerlei in vivo-Effekte.
3. In vitro Untersuchungen an isolierten Langerhansschen Inseln von BB/OK- und BB.6S- Ratten zeigten, daß durch Variation der Ca-Konzentration im Kulturmedium weder der Insulingehalt, noch die glukosestimulierte Insulinsekretion zwischen beiden Linien differierte.
   Signifikante Unterschiede fanden sich jedoch bei der Proteinexpression mittels Westernblottanalyse. Das Ca-bindende Protein, Calbindin-D28k, war in Inseln von BB.6S-Ratten bereits unter Basalbedingungen doppelt so hoch wie das von BB/OK-Ratten. Zunehmende Ca-Konzentration im Medium erhöhte deutlich die Proteinexpression von Calbindin-D28k bei BB.6S-Inseln während die Expression bei BB/OK-Ratten durch Ca fast unbeeinflußt blieb. Calbindin-D28k ist ein cytosolisches Cabindendes Protein, welches bevorzugt in der Niere, aber auch im Pankreas und Gehirn exprimiert wird und den apoptotischen (gengesteuerten) Zelltod verhindern kann (41,42). Funktional wird vermutet, daß Calbindin-D28k bei der Regulation der Ca-Reabsorption beteiligt ist.

Da das Kandidatengen
1. die zwei essentiellen diabetogenen Gene, Iddm1 und Iddm2, nahezu ausschalten kann,
2. bei der Immuntoleranz und beim apoptotischen Zelltod,
3. als auch bei der Regulation von Ca, Ca-bindenden Proteinen (Calbindin-D28k u.a.) und Hormonen (Calcitonin, u.a.),
4. bei der Entstehung einer Dyslipidämie involviert ist,
wurde der Schluß gezogen, daß das Gen ein **multifunktioneller Transkriptionsfaktor** ist.

### Das Kandidatengen

Nach Homologievergleichen und Genfunktionsprüfungen wurde der Transkriptionsfaktor Yin Yang-1 (YY1), der mit hoher Wahrscheinlichkeit in der diabetesprotektiven Region bei Mensch und Maus kartiert, im Rahmen der vorliegenden Erfindung als Kandidatengen favorisiert, denn er kann die Transkription einer großen Anzahl von zellulären und viralen Genen aktivieren, aber auch hemmen bzw. kann selbst Transkription initiieren und entspricht damit einem multifunktionellem Transkriptionsfaktor.

YY1 gehört zu der Klasse der regulatorischen Transkriptionsfaktoren (RTF), d.h. die Bindung erfolgt sequenzspezifisch an proximale und distale Regulationselemente der DNA. Darüber hinaus besitzt er die Fähigkeit, direkt oder indirekt einen Einfluß auf die Transkriptionmaschinerie zu nehmen. Durch seinen vielseitigen molekularen Strukturaufbau (Aktivierungsdomäne, Repressionsdomäne, Zinkfingerstrukturen) ist es ihm möglich, eine differentielle Genaktivität durchzuführen. Das heißt, er kann als zeitlicher, räumlicher, zellspezifischer, organspezifischer oder signalvermittelnder Parameter fungieren. Seine Funktionsmodule erfüllen die Aufgaben der spezifischen DNA-Erkennung, der Transkriptionsaktivierung, Transkriptionsrepression, aber auch die Interaktion mit anderen Proteinen.

### Genaufbau - Vergleich

Das Protein von Yin Yang ist gekennzeichnet durch die lokale Anhäufung von Aspraginsäure (D) und Glutaminsäure (E) zwischen den AS-bereichen 43 bis 53. Der Abschnitt ist homolog zw. Ratte und Mensch, jedoch unterscheidet sich die Anzahl und die Position von E und D voneinander. Die Ratte besitzt 5 x E und 6 x D, wobei der Mensch über 6 x E und 5 x D verfügt. Daran schließt sich ein Histidin-Cluster bei der Ratte von 54 bis 79 und beim Menschen von 54 bis 82 an. Es fehlen 3 Histidine bei der Ratte gegenüber der humanen Seqeuenz. Es folgt eine GA/GK reiche Domäne von 154 bis 198 beim Menschen. Bei der Ratte ist dieser Positionsbereich um 3 AS-Positionen in Richtung N- terminales Ende versetzt. (151-195). Die vier Zinkfinger der humanen Sequenz beginnen an Position 298 und enden an Position 407. In der Rattensequenz erstreckt sich dieser Bereich von 295 bis 404. Die gesamte AS-Länge beträgt beim Menschen 414 und bei der Ratte 411 AS (s. Sequenzprotokoll: SEQ ID NO:2 und 4; die Bezeichnung "SEQ ID NO:" entspricht hier und im folgenden der Sequenzkennzahl "<400>" nach WIPO Standard ST.25).

### Funktionelle Domänen

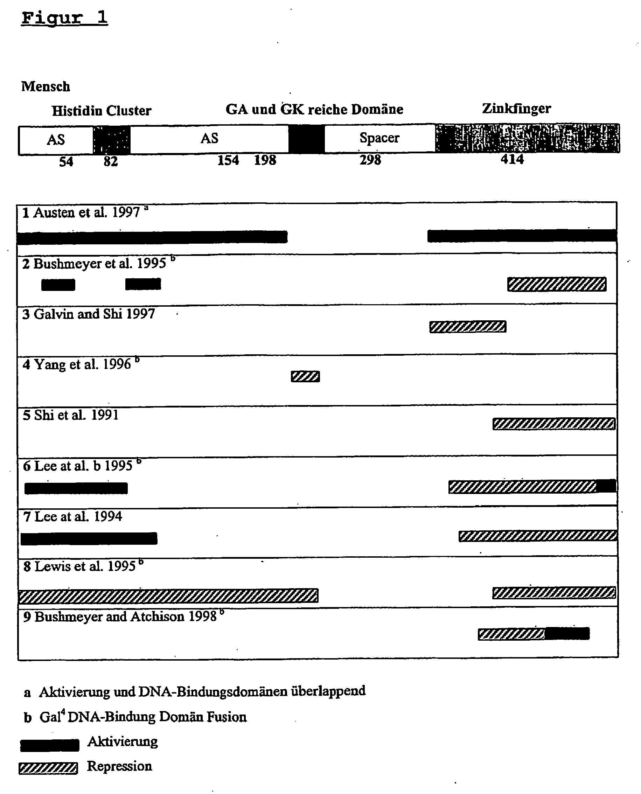

Viele Forschergruppen haben die Struktur und Funktion von YY1 mit Hilfe von Deletionsmutantan, sowie mit Reporterkonstrukten analysiert. Diese Ergebnisse sind in Fig. 1 zusammengefaßt.

Übereinstimmend wurden zwei Aktivierungsdomänen gefunden. Sie sind im Bereich des N-terminalen Endes und in der Region des Zinkfingers lokalisiert. Darüber hinaus konnten von zwei Gruppen weitere Domänen mit aktivierender Funktion in der Nähe des C-Terminus lokalisiert werden (397-414 und 370-397) (43,44). Man argumentierte die Maskierung der N-terminalen Aktivierungsdomäne durch die C-terminale Domäne, sowie daß bei Deletion der C-terminalen Domäne die N-terminale Domäne demaskiert wird, so daß YY1 als konstitutiver Aktivator agieren kann. Diese Ergebnisse führten zu dem Schluß, daß nur unter bestimmten Bedingungen YY1 zum Aktivator wird. Es zeigte sich, daß das E1A in der Lage war, Yin Yang in einen Aktivator umzuwandeln (43).

Repressionsdomänen konnten für den Zinkfingerbereich lokalisiert werden. Dabei werden dem Zinkfinger 1 und 2 die Repressionsaktivität zugesprochen, wobei Zinkfinger 2 und 3 für die DNA-Bindung zuständig sein sollen (45). Bei Betrachtung der Röntgen-Cokristallstruktur von Zinkfinger 2 und 3 stellte man im Gegensatz dazu fest, daß diese in der Lage waren, an den Basen, sowie an das Phosphatrückgrat der DNA anzugreifen (46). Für den Zinkfinger 1 konnten Kontaktstellen nur für das Phosphatrückgrat und für Zinkfinger 4 nur für einige wenige Basen gezeigt werden.

Weitere Repressionsdomänen zeigten sich überlappend mit der Aktivierungsdomäne an den Positionen von 1 bis 201, sowie von 170 bis 200 (47,48).

Die Interpretation dieser Ergebnisse von Thomas et al. (49) zeigten zusammenfassend, daß sich nur zwei Repressionsdomänen im YY1 Gen befinden, im Bereich von 170 bis 200 und in der Zinkfingerregion, die vielleicht zusammen interagieren können (vgl. Fig. 1).

### Protein/Protein Interaktionen

Yin Yang ist imstande, mit Proteinen, wie Transkriptionsfaktoren und Coaktivatoren/Corepressoren zu interagieren. Zwei Domänen, die interagieren sind die Bereiche um 150 bis 170 (zentrale Domäne), in denen die GA/GK reiche Domäne mit Teilstükken des Spacers, sowie die C-terminale Region und die Zinkfinger mit Teilen des Spacers lokalisiert sind.

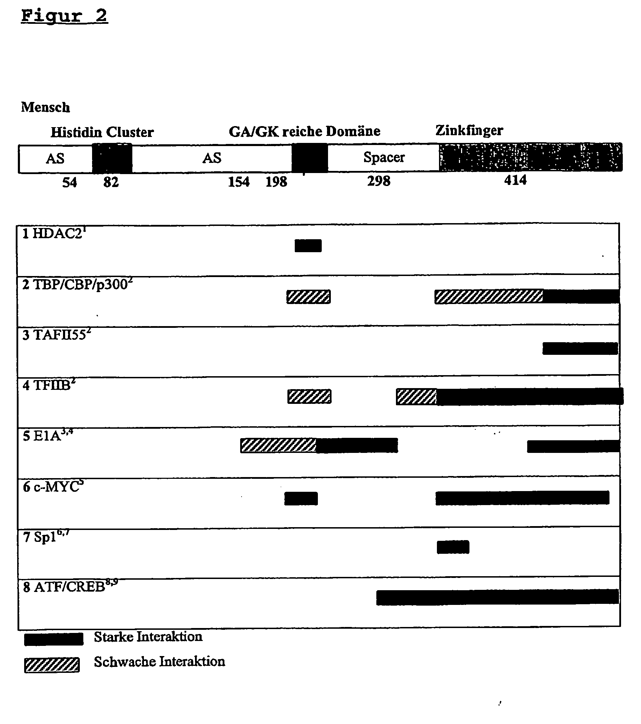

Einige Proteine, wie TBP, CBP/p300, TFIIB, E1A und c-MYC können an beide Regionen binden (43,45,47,50,51). Proteine, die nur mit einer dieser zwei Domänen interagieren können, sind: HDAC2 (zentrale Domäne), SP1 und ATF/CREB (C-terminale Domäne) (45,48,52-54) (vgl. Fig. 2).

Galvin and Shi demonstrierten (45), daß die Repression von CREB und SP1 durch YY1 aktivator-spezifisch ist. Die Autoren prostulierten, daß YY1 die Target (s) der Aktivatoren interferiert, anstelle von direkter Bindung zu diesen zwei Faktoren.

Nur ein Protein, E1A, wurde bisher gefunden, welches direkt mit der Aktivierungsdomäne von YY1 interagiert (43,47,55). Seit dieser Entdeckung wird diskutiert, ob YY1 seine Repressorfunktion in Richtung Aktivator auch verändern kann, möglicherweise durch Maskierung seiner Repressionsdomänen über Modifizierung, oder aber über Freilegung seiner Aktivierungsdomäne.

### Promotorbindung

Ausgezeichnet durch vier Cys2-His2-Zinkfinger ist Yin Yang in der Lage, entweder die Transkription zu aktivieren oder zu inhibieren, was jedoch von der Promotorsequenz der Gene und von der Konzentration des YY abhängig ist. Die Consensus-Sequenz (C/t/a)CATN(T/a)(T/g/c), die in vielen Promotoren von viralen und zellulären Genen zu finden ist, kann über den Zinkfinger gebunden werden (56).

### Repressionsmodelle

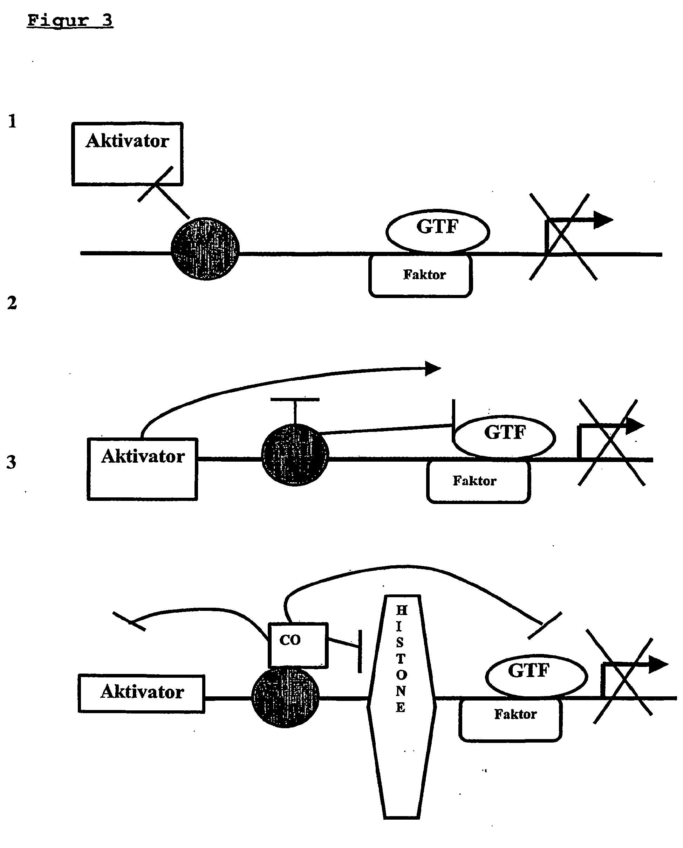

Neben der geregelten Aktivierung der Transkription stellt auch dessen Repression einen wichtigen Kontrollmechanismus der Genaktivität dar. Repression durch Umkehrung bzw. Aufhebung genaktivierender Prozesse kann durch YY1 bewirkt werden. Im YY1-Genabschnitt wurden mehrere Repressionsbereiche gefunden (siehe Fig. 3).

Drei Modelle der Repressoraktivität sind bis zum heutigen Stand bekannt. Im ersten Modell deplaziert YY1 den Aktivator.

Störende Eigenschaften weist er im zweiten Modell auf. Er behindert den Aktivator, sowie die generellen Transkriptionsfaktoren (GTF) in der Ausübung ihrer Funktion.

Eine indirekte Repression (Drittes Modell) führt YY1 über die Rekrutierung von Corepressoren aus. Er nimmt indirekten Einfluß auf die Chromatinstruktur (49). Von besonderem Interesse sind dabei die HDAC 1, 2 und 3, als Corepressoren. HDAC's stehen im Zusammenhang mit Auflockerung der Chromatinstruktur. Alle drei Proteine sind globale Regulatoren der RPD3, d.h. sie sind in der Lage, Histone zu deacetylieren (in vitro). Außerdem können die HDAC bei Dirigation zum Promotor direkt die Transkription blockieren (48, 57-60). Bei Überexpression von HDAC 2 konnte durch die Gruppe Yang et al. (48) gezeigt werden, daß die Repressionsfunktion von YY1 verstärkt wird. Durch die Entdeckung, daß HDAC 2 im Komplex mit HDAC 1 arbeitet, muß das gleiche auch für HDAC 1 gelten.

### Aktivierungsmodelle

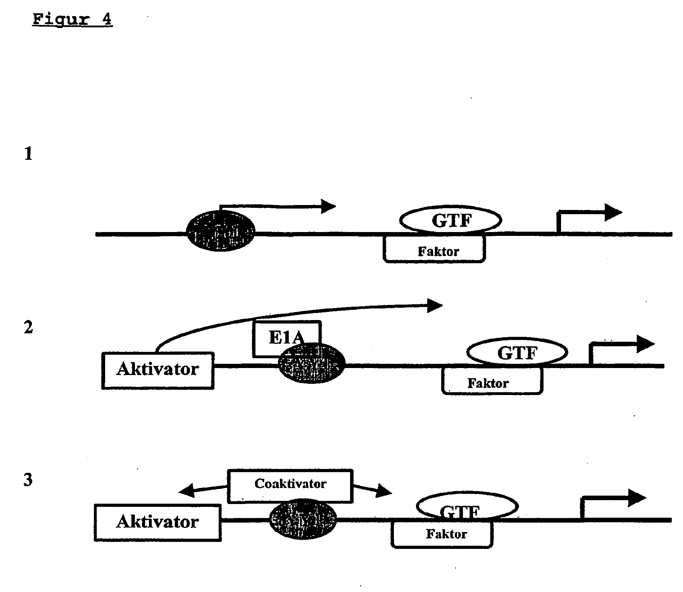

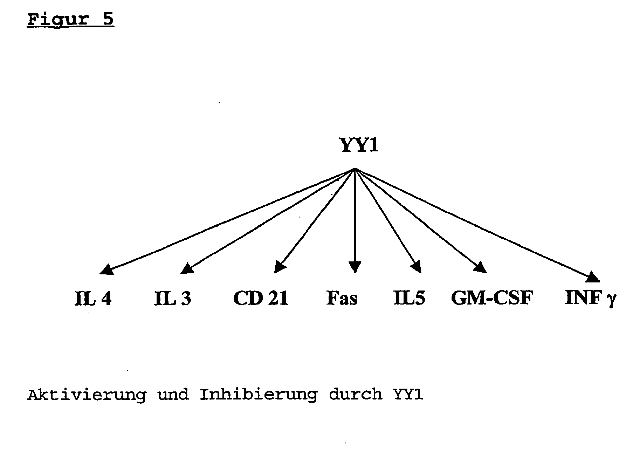

Drei Modelle sind bekannt, wie Yin Yang die Transkription aktiviert (siehe Fig. 4). Direkte Aktivierung konnte mit Hilfe des Adenovirus Protein E1A von der Gruppe Shi et al (61) gezeigt werden. Stimulation der Transkription wird erreicht durch die direkte Interaktion YY1 mit den GTF: TAFII55, TBP und TFIIB (62,63). Der zweite Mechanismus konnte am AAV P5 Initiator Element aufgeklärt werden (64). Darüber hinaus wird diskutiert, ob YY1 eine strukturelle Veränderung durchmacht, wenn er mit anderen Proteinen interagiert. Im dritten Modell rekrutiert Yin Yang Coaktivatoren, die mit anderen Transkriptionsfaktoren interagieren. Dieses Modell beinhaltet vielleicht auch die Modifikation des Chromatin durch p300. Durch die Auflockerung der Chromatinstruktur mittels p300 (HAT-Aktivität), wäre der Zugang zur DNA erleichtert und eine effiziente Bindung möglich (51,55,65).

### Regulation von YY1 durch Acetylierung und Deacetylierung:

Die Regulation des Transkriptionsfaktors erfolgt auf der posttranslationalen Ebene durch die Interaktion mit anderen Proteinen.

Die Transkriptionsaktivatoraktivität von YY1 ist direkt abhängig von der Assoziation zu den Coaktivatoren p300, CBP, sowie PCAF. Diese Coaktivatoren besitzen Histon-Acetyltransferase (HAT) und sind somit befähigt, Acetylgruppen zu transferieren. Im Gegensatz dazu ist die Repressionsaktivität von YY1 assoziiert mit der Histon-Deacetylase2 (HDAC2) im Bereich 170-200 der Repressionsdomäne.

Die selektive Assoziation von YY1 mit HAT oder HDAC entscheidet darüber, ob er als Aktivator oder Repressor fungiert.

### Acetylierung und Deacetylierung durch p300,PCAF und DHAC:

Yin Yang verfügt über 2 Acetylierungsdomänen. Die erste liegt im Bereich zwischen 170 bis 200, die Zweite überlappt den Zinkfingerbereich am C-terminalen Ende zwischen 261-414.

Die Acetylierungen im Bereich 170 bis 200 werden durch p300 und PCAF an den Lysin- Resten durchgeführt. Sechs Paare an Lysinen befinden sich in der ersten Domäne, wobei aber nur 3 verschiedene Lysine durch p300 und PCAF acetyliert werden. Um eine maximale Repressionsaktivität zu erhalten, muß YY1 an allen drei Stellen acetyliert werden. Würde nur ein Lysin durch ein Arginin ersetzt werden, wäre keine maximale Repressionsaktivität mehr gewährleistet, aufgrund der eintretenden Konformationsveränderung.

Ein weiterer, entscheidender Punkt der Acetylierung zwischen 170 bis 200 ist, daß sich die Bindungswahrscheinlichkeit von DHAC signifikant erhöht und somit in diesem Bereich auch eine Deacetylierung durch HDAC 1,2 erfolgen kann.

Erstaunlicherweise ist es HDAC auch möglich, in dem zweiten Acetylierungsbereich (261-333) zu binden. Im Gegensatz zur ersten Acetylierungsdomäne findet hier jedoch keine Deacetylierung statt.

Weiterhin konnte gezeigt werden, daß sich durch die Acetylierung von PCAF im überlappenden Bereich des Zinkfingers (261-333) die DNA-Bindungsaktivität von YY1 erniedrigt (66).

### Feinkartierung von YY1

Da die Sequenz der Ratte bis Dezember 2002 noch nicht in der Genbank vorhanden war, wurde erfindungsgemäß die Gensequenz der Maus benutzt, um Primer zu rekrutieren, denn die erste Aufgabe bestand in der Feinkartierung von YY1. Es mußte sichergestellt werden, daß das Gen tatsächlich im diabetesprotektiven Bereich liegt.

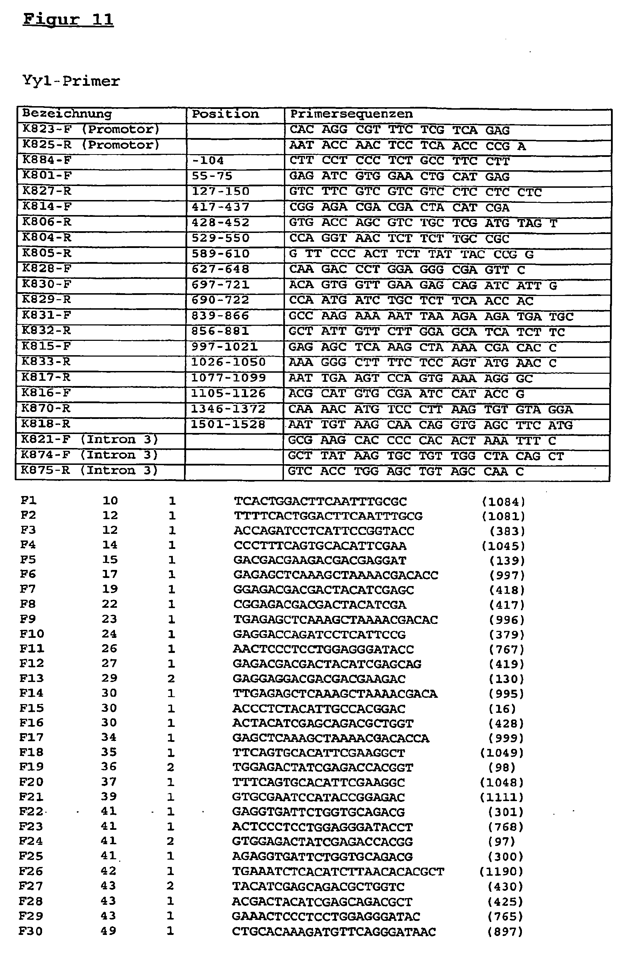

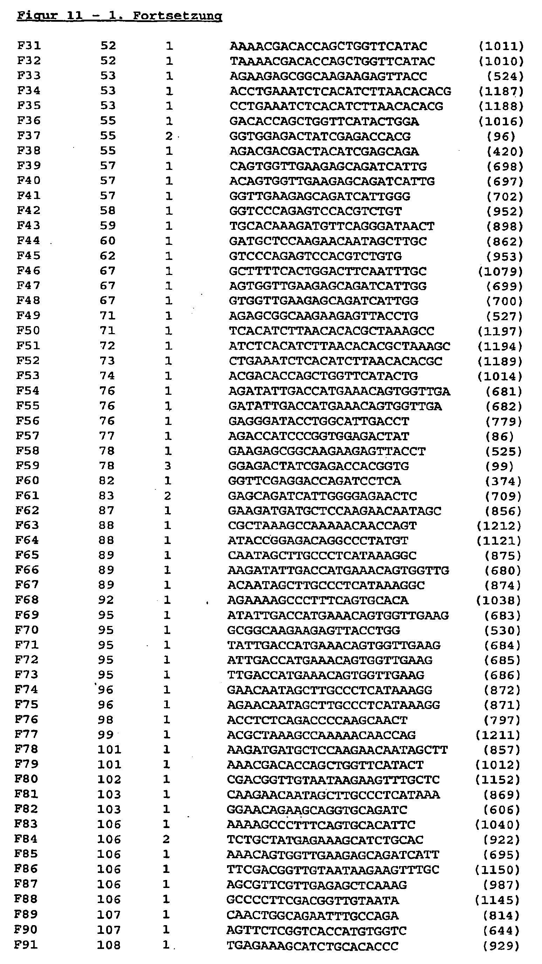

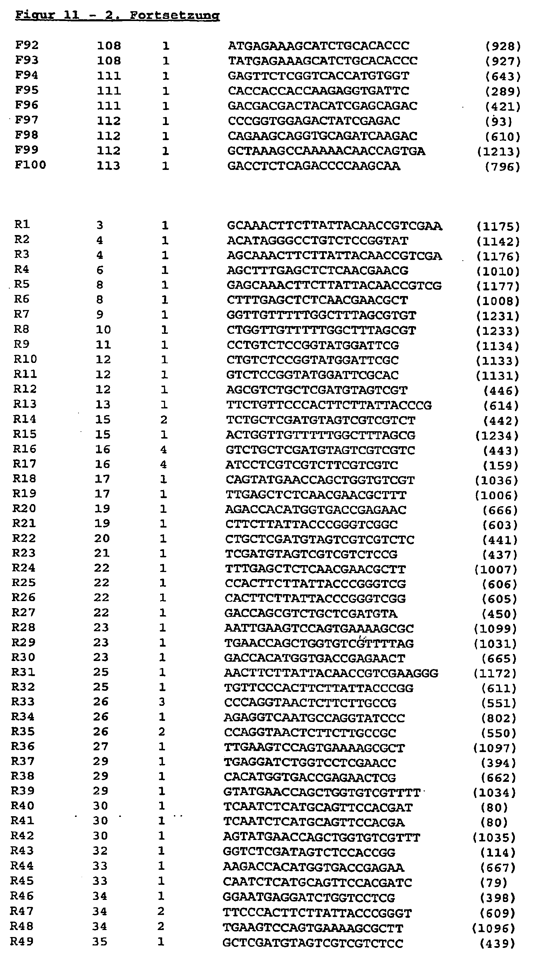

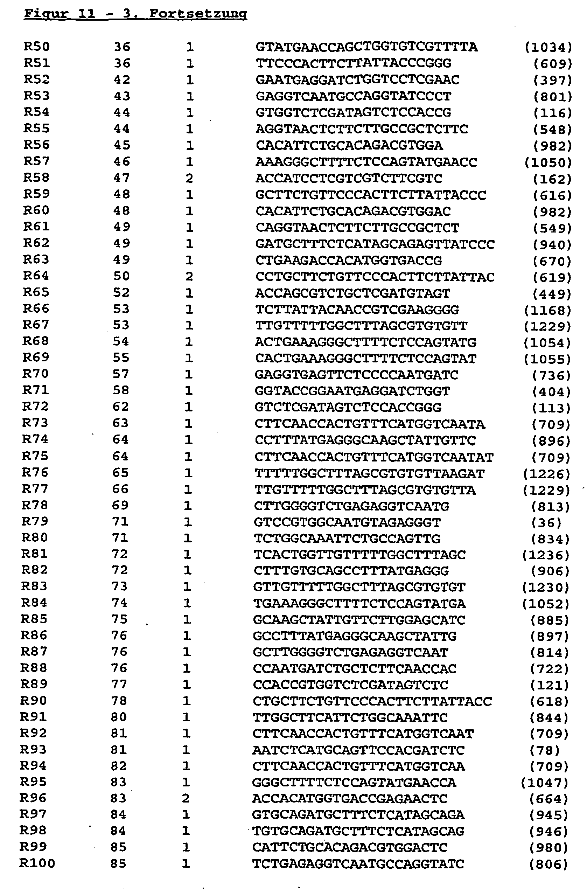

Da die Chance, im Intron einen Polymorphismus zu finden, recht groß ist, wurden Primer zur Amplifikation der Introns verwendet (vgl. Fig. 11; Intron 1: K828-F/K829-R; Intron 2: K830-F/K832-R; Intron 3: K831-F/K833-R; Intron 4: K815-F/K817-R; K815-F/K875; K821-F/K817-R; K821-F/K870-R; K874-F/K870-R). Es wurde nur ein sequenzierbares Genprodukt mit genomischer DNA vom Intron 4 erhalten. Das Intron hat 633 Basenpaare (bp) und unterscheidet sich zwischen BB- und SHR-Ratten an den Positionen 323 (t-a), 502 (g-c) und 528 (a-c). Zur genauen Positionierung von YY1 auf Chromosom 6q32 wurde dieser Polymorphismus unter Verwendung der subkongenen BB.6S-Linien benutzt. YY1 wurde zwischen D6Mgh2 und Ighe kartiert und befindet sich somit im diabetesprotektiven Bereich.

Um zu prüfen, ob der Polymorphismus im Intron 4 auch bei anderen Stämmen vorkommt oder weitere Unterschiede auftreten können, wurde das Intron 4 von folgenden Ratten sequenziert und verglichen: 2 Wildfangtiere (Wildtyp, M+W), jeweils 1 Tier von diabetes-resistenten, ingezüchteten DA/K, BN/Mol, LEW/K und WOKW-Ratten.

WOKW-Ratten entwickeln keine Hyperglykämie (=Diabetes), aber ein komplettes metabolisches Syndrom (Fettsucht, Hyperinsulinämie, Dyslipidämie, gestörte Glukosetoleranz, Hypertonie) und stammen wie BB/OK-Ratten aus der gleichen Wistarratten-Auszuchtpopulation der BioBreeding Laboratories, Ottawa, Canada (67,68).

Im Ergebnis zeigte sich, daß die Intronsequenz zwischen BB/OK und Wildfangtieren, sowie zwischen SHR und DA, BN, LEW.1W, als auch WOKW identisch ist.

### Sequenzierung von YY1

Sowohl genomische als auch cDNA wurde mittels PCR unter Verwendung verschiedener Primer so amplifiziert, daß überlappende Genprodukte zur Sequenzierung zur Verfügung standen (vgl. Fig. 11; Promotor: K823-F/K825-R*; Promotor und Exon 1: K884-F/K806-R; Exon 1: K801-F/K804-R; K814-F/K832-R*; Exon 2 und 3: K828-F/K833-R; K831-F/K817-R; Exon 4: K815-F/K870-R; K815-F/K818-R; K816-F/K819-R; K834-F/K836-R). Die PCR und Sequenzierung wurden wie im Material & Methoden-Teil (M&M) beschrieben durchgeführt.

Das YY1-Gen der Ratte umfaßt 1236 Basenpaare (bp) und besteht aus 411 Aminosäuren (AS), was von Nishiyama et al. 2003 bestätigt wurde (69). Sequenzunterschiede zwischen BB/OK und SHR bzw. BB.6S wurden an Position 603 (c-t), 980 (t-g) und 1004 (g-a) nachgewiesen (vgl. SEQ ID NO:1 und 3). Dieser Basenaustausch führt zur Änderung der Aminosäuren an Position 303 und 311 im Zinkfingerbereich (vgl. SEQ ID NO:2 und 4). Bei BB/OK-Ratten kodieren die ÄS Methionin (Nukleotide 907-910 ab Startcodon) und Arginin (Nukleotide 931-933 ab Startcodon) und bei SHR-Ratten die AS Arginin und Lysin.

Der Promotorbereich wurde nur teilweise vom Transkriptionsstart (-72) sequenziert, wobei noch ein Bereich von 470 bp mit der GC-Box vorliegt. Unterschiede zwischen BB/OK und SHR waren nicht nachweisbar.

Nach Sequenzvergleichen mit der Maus sollte der Promotorbereich ca. 891 bp vom Transkriptionsstart entfernt sein (Acc: L13969, 1-464, 86% Homologie). Beim Vergleich mit der Humansequenz (Acc: AF047455 Promotor in dieser Sequenz nur -42bp) waren Übereinstimmungen zwischen -636 bis -585 (216-269; 47/54; 87% Übereinstimmung) und -464 bis -392 (392-464; 64/73; 87%) Basen vom Transkriptionsstart zu finden.

### Sequenzvergleich der Nukleinsäure und des Proteins zwischen Ratte und Mensch

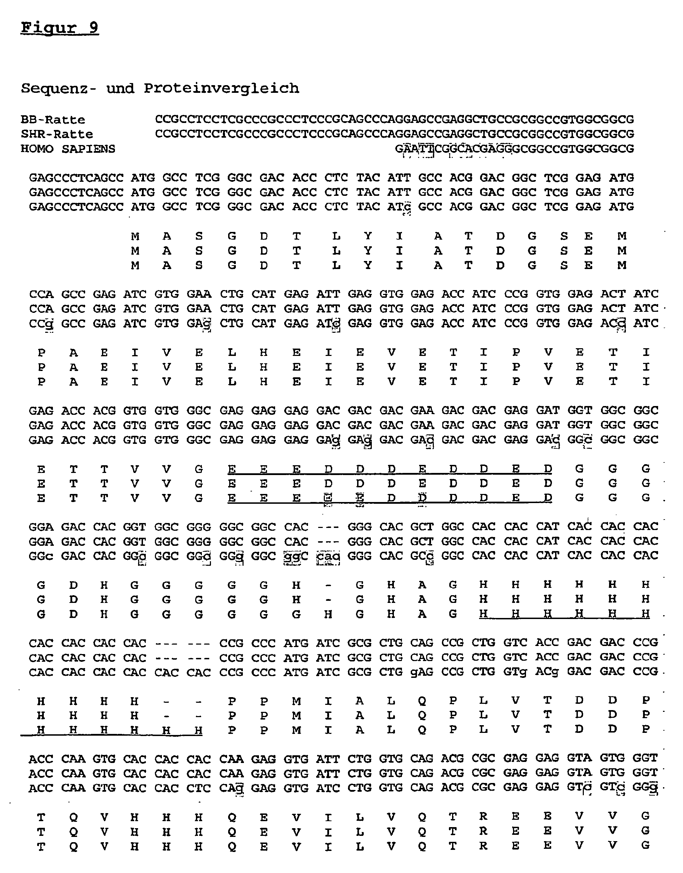

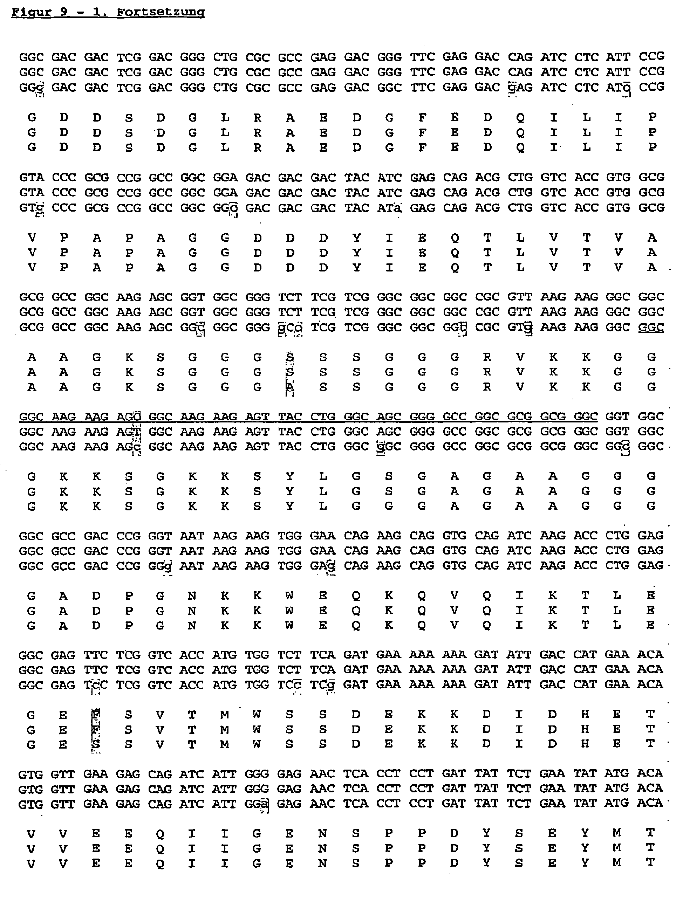

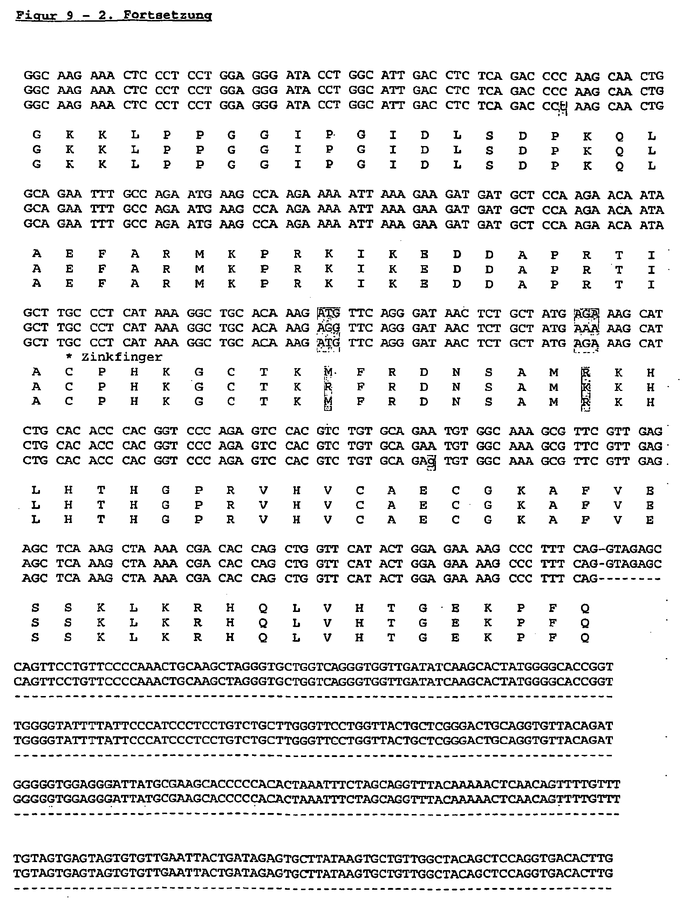

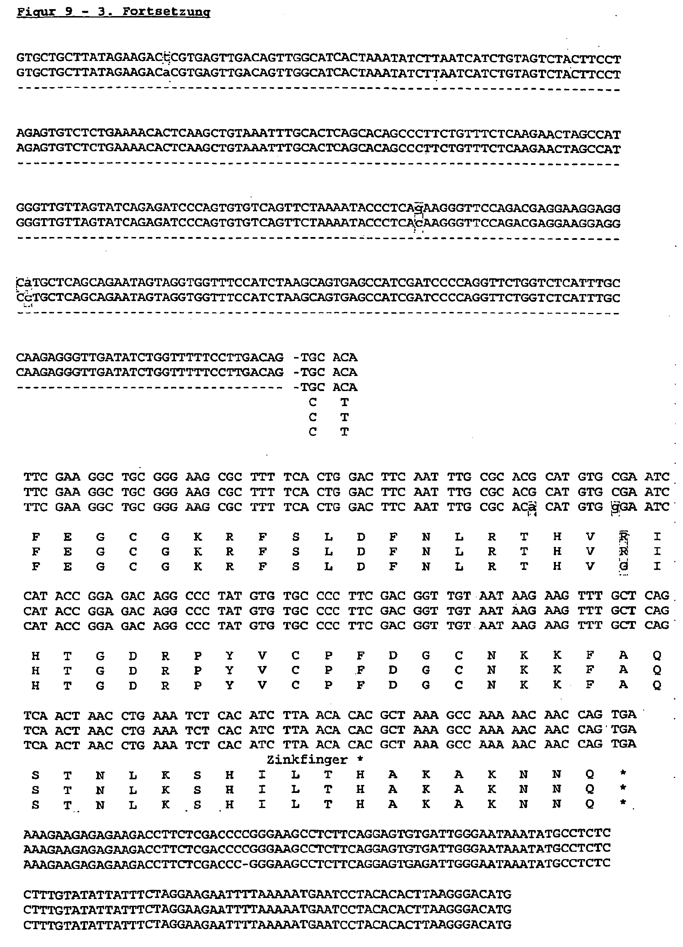

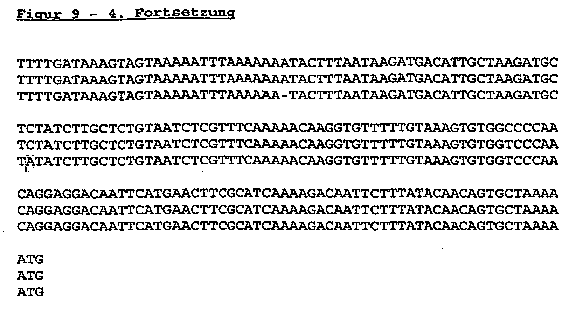

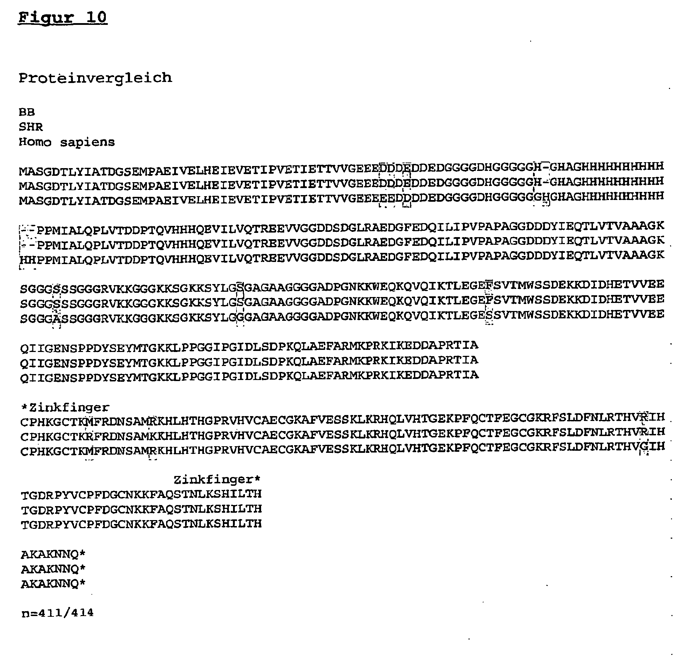

Der Sequenzvergleich zwischen BB/OK-, SHR-Ratte und Mensch, wie in Fig. 9 und 10 zusammengestellt, zeigten eine bp-Übereinstimmung von 95,6% bzw. 95,3% und die AS von 96,9 bzw. 96,4%. Berücksichtigt man die Tatsache, daß der Ratte 3 AS fehlen (3 Histidine, 1 x zwischen Aktivierungs- und erster Represssionsdomäne, Position 66 beim Menschen und 2 in der ersten Repressionsdomäne, statt 12 Histidinen hat die Ratte nur 10), erhöht sich die Übereinstimmung im codierenden Bereich zwischen Ratte und Mensch bei den bp auf 97,0 (1206/1239) bzw. 96,8% (1200/1239) und bei den AS auf 97,6% (401/411) bzw. 97,1% (399/411). Im Zinkfinger, der 330 bp und 110 AS umfaßt, stimmen bei der BB/OK-Ratte 99% der bp (327/330) und AS (109/110) und bei SHR-Ratten 98% der bp (325/330) und 97,3% (107/110) der AS überein.

### Genexpressionsstudien

Um zu prüfen, inwieweit die Sequenzunterschiede eine Auswirkung auf die Genexpression von YY1 haben, wurden Genexpressionsstudien durchgeführt.

Es wurde mRNA aus isolierten Langerhannschen Inseln, Pankreas, Leber, Gehirn, Thymus und Milz von 8 Tage alten Ratten der Inzuchtstämme BB/OK, SHR, BB.6S und LEW ("unbelastete" Kontrolle) gewonnen, in ssDNA umgeschrieben und für die Genexpressionsstudien eingesetzt (vgl. Material&Methoden-Teil).

Es wurde wiederholt gezeigt, daß der YY1-Zinkfingerbereich unter Verwendung der Primer K831-F/K818-R und K831-F/K870-R in isolierten Langerhansschen Inseln der BB/OK-Ratte bzw. LEW-Ratte stark (YY1-Zinkfinger/GPDH-Intensität = 0.19 ± 0.04) und signifikant erniedrigt in den von SHR- und BB.6S-Ratten exprimiert ist (YY1-Zinkfinger/GPDH-Intensität = 0.03 ± 0.02; p<0.001). Im Pankreas zeigte sich ein ähnlicher Expressionsunterschied, jedoch etwas abgeschwächt. Die Untersuchung der Expression unter Verwendung der Primer K831-F und K870-R zeigte, daß im Pankreas, der Leber und im Gehirn bei BB.6S-Ratten eine zweite, um ca. 150 bp kürzere Bande auftrat, die weder bei BB/OK noch bei SHR und LEW zu beobachten war. Interessant war auch die Tatsache, daß **Geschlechtsunterschiede in der Genexpression** auftraten. Die Expression bei männlichen Tieren war stets stärker als bei Weibchen. In allen anderen untersuchten Organen wird YY1 exprimiert. Augenfällige Unterschiede zwischen den Stämmen, außer zwischen männlichen und weiblichen Tieren, wurden nicht beobachtet.

### Sequenzierung der zweiten Bande bei BB.6S

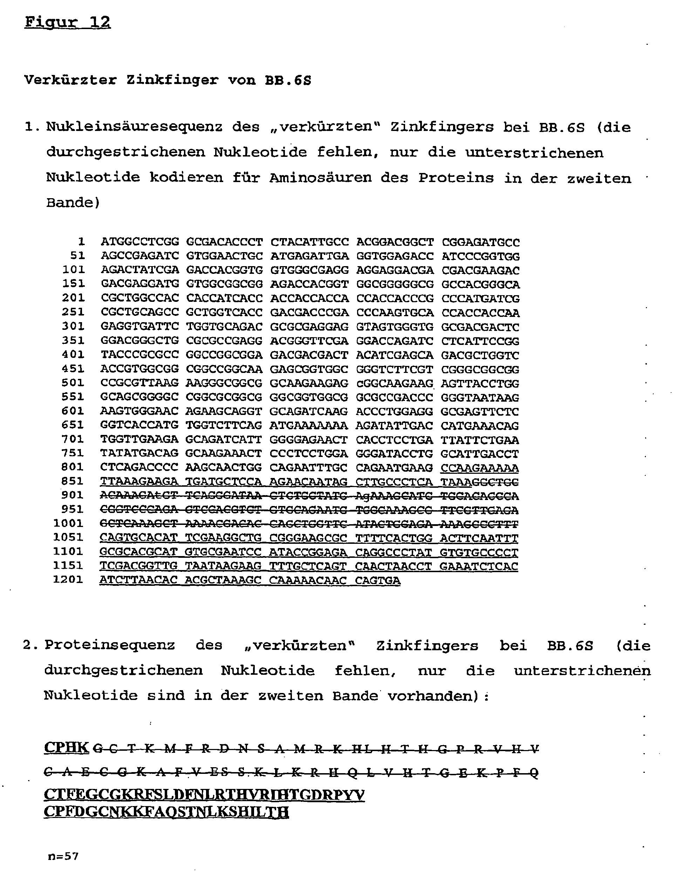

Da eine zweite Bande nur bei BB.6S-Ratten im Pankreas, in der Leber und im Gehirn auftrat, wurde diese zweite Bande eluiert, amplifiziert und sequenziert (vgl. M&M). Im Ergebnis zeigte sich, daß diese Bande eine verkürzte Zinkfingersequenz ist (vgl. Fig. 12 und SEQ ID NO:7). Danach fehlt ein Teil vom Zinkfinger 1, und Zinkfinger 2 fehlt vollständig (967 bis 1125; vgl. SEQ ID NO:8).

### Schlußfolgerungen

Da gezeigt werden konnte, daß
1. YY1 als multifunktioneller Transkriptionsfaktor im diabetesprotektivem Bereich kartiert,
2. Sequenzunterschiede mit Änderung der AS zwischen BB/OK und SHR im Zinkfingerbereich (AS an Position 303 - Methionin/Aginin und 311 Arginin/Lysin sind different) und im Intron 4, das im Zinkfinger liegt, nachweisbar sind,
3. bei BB/OK-Ratten YY1 in isolierten Langerhansschen Inseln und Pankreas deutlich und bei SHR- und BB.6S-Ratten kaum, in den anderen untersuchten Organen jedoch vergleichbar, aber mit 2 Banden bei BB.6S nach Amplifizierung des Zinkfingerbereiches exprimiert ist,
wurde der Schluß gezogen, daß YY1 bei BB.6S in Langerhansschen Inseln unterexprimiert ist und damit diabetesprotektiv wirkt, wobei die zweite Bande im Pankreas und anderen Geweben ebenfalls für die Diabetesprotektion von Bedeutung ist, zumal dem Zinkfinger 2 eine besondere Rolle bei der Transkription zugesprochen wird. Eine Beobachtung, die durch die AS-Änderung im Zinkfingerbereich und/oder im Intron (unterschiedliches Spleißverhalten bei BB/OK und BB.6S) verursacht ist.

Da die Änderung im Zinkfingerbereich eine diabetesprotektive Wirkung hat, sind mehrere Gene in ihrer Regulation/Aktivität beeinträchtigt/verändert. Für eine Beteiligung des Introns an der Diabetesprotektion spricht die Tatsache, daß die Sequenzunterschiede zwischen BB/OK und SHR nachweisbar waren, nicht aber zwischen BB/OK und Wildfangtieren. Die Sequenz zwischen BB/OK und Wildfangtieren war gleich. Dies wiederum erklärt, warum BB.6S-, nicht aber BB.6W-Ratten vor der Entwicklung eines Diabetes geschützt sind.

Infolge der erfindungsgemäß gewonnenen Erkenntnisse eröffnet die Modulation des multifunktionellen Transkriptionsfaktors YY1 auf Expressions- und Regulationsebene zum einen erstmals einen Weg, um Typ-1-Diabetes zu verhindern, zum anderen lassen sich durch diese Modulation auch Autoimmunerkrankungen als solche verhindern. Ferner lassen sich auch weitere Erkrankungen wie Krebs, AIDS, Fettstoffwechselstörungen und Hypertonie positiv beeinflussen.

Gegenstand der vorliegenden Erfindung ist daher ein Protein, das die in SEQ ID NO:4 dargestellte Aminosäuresequenz aufweist. Ferner eingeschlossen sind Homologe des Proteins, die Arginin und an Position 311 Lysin aufweisen, wobei das Protein eine diabetesprotektive Wirkung aufweist. Der Homologiegrad beträgt mindestens 95%, vorzugsweise mindestens 97%, und besonders bevorzugt mindestens 99%.

Die Erfindung betrifft auch Peptide, die Fragmente eines vorgenannten Proteins sind und eine Aminosäuresequenz aufweisen, die den die Aminosäurepositionen 303 und 311 in SEQ ID NO:4 (bzw. 306 und 314 in SEQ ID NO:6) umfassenden Sequenzbereich enthält. Die Länge der erfindungsgemäßen Peptide beträgt beispielsweise 53 bis 315 Aminosäuren, vorzugsweise z.B. 315, 117 oder 53 Aminosäuren, wobei die Peptide vorzugsweise die Sequenzbereiche von Position 1 bis 315, von 295 bis 411 bzw. von 299 bis 351 umfassen.

Erfindungsgemäß sind ferner folgende Fragmente wichtig: Aminosäuren 165-214; 255-333; 255-411. Die Numerierung bezieht sich auf die Numerierung gemäß SEQ ID NO:4.

Die Erfindung betrifft ferner eine Nukleinsäure, die für ein vorgenanntes Protein oder Peptid kodiert. Die für das Protein mit der in SEQ ID NO:4 codierende Nukleinsäure weist vorzugsweise die in SEQ ID NO:3 dargestellte Nukleinsäuresequenz auf. Eine für die vorgenannten Homologen kodierende Nukleinsäure weist eine Nukleinsäuresequenz auf, die den die Nukleinsäurepositionen 979-981 und 1003-1005 in SEQ ID NO:3 umfassenden Sequenzbereich enthält. Die Codons an den genannten Positionen können selbstverständlich infolge der Degeneration des genetischen Codes variieren, solange sie für die Aminosäuren Arginin bzw. Lysin kodieren.

Erfindungsgemäß ist ferner die Intronsequenz wichtig: 1126-1758. (Die Lage der Exons in SEQ ID NO:3 ist: Exon 1:73 bis 729; Exon 2: 730 bis 825; Exon 3: 826 bis 978; Exon 4: 979 bis 1125; Exon 5: 1759 bis 1938; siehe Sequenprotokoll.) Die Numerierung bezieht sich auf die Numerierung gemäß SEQ ID NO:3.

### Diabetes Mellitus Typ 1 und Autoimmunerkrankungen an sich

Wie bereits erwähnt, resultiert Typ-1-Diabetes aus der selektiven Zerstörung der Insulin-produzierenden β-Zellen im Pankreas. Der Untergang der β-Zellen wird durch autoreaktive T-Zellen, die gegen β-Zell-spezifische Antigene (Autoantigene) gerichtet sind, gesteuert . Mit der Diabetesmanifestation verbunden ist eine Insulitis (69). Dieser Prozess ist gekennzeichnet durch die lymphozytäre Infiltration der β -Zelle. Da eine Insulitis nicht immer die Antwort auf eine β-Zelldestruktion sein muß, unterscheidet man zwischen einer benignen und destruktiven Insulitis (70). Dieser Prozeß korreliert mit der Cytokinproduktion, wie für Modelltiere (BB-Ratte) und für den Menschen gezeigt werden konnte (71-78). Dabei spielen die Cytokine IFNγ und IFNα während der destruktiver Insulitis eine Hauptrolle. Für die benigne Insulitis werden IL10 und TGFβ als Hauptfaktoren angesehen. Darüber hinaus konnte in nicht-diabetischen Mäusen Insulitis durch TNFα, TNFβ, sowie IL6 induziert werden, was jedoch nicht zu einer β-Zellzerstörung führte. Bei transgener Expression von IFNα, IFNγ und IL2 konnte gezeigt werden, daß nicht-diabetische Mäuse eine Insulitis und einen autoimmunen Typ-1-Diabetes entwickeln. Demnach sind IFNγ, IFNα, IL2 und IL10 bei der Entstehung, TNFα, IL4, IL6, sowie TGFβ bei der Verhinderung des Typ-1-Diabetes involviert (71-81).

Da alle BB.6S-Ratten eine Insulitis entwickeln, Diabetes aber nur bei etwa 15% der Tiere auftritt (82), muß YY1 auch beim Insulitisprozeß eine Rolle spielen. Entweder schaltet YY1 die protektiven Cytokine an, unterdrückt die pathogenen Cytokine oder er balanciert beide Cytokingruppen so, daß zum einen die β-zeildestruktive Insulitis auftritt oder aber die benigne Insulitis, die nicht zum autoimmunen Diabetes führt, zum Tragen kommt. Da er auch in der Lage ist, IL4 zu aktivieren (83) und IFNγ zu inhibieren (84), wird erfindungsgemäß davon ausgegangen, daß er auch die Relation der T-Helferzellpopulationen, TH1 und TH2, zugunsten von TH2 verschiebt.

Ist durch eine Mutation die AS-Sequenz von YY1 bei Typ-1-Diabetikern verändert, kann er nicht mehr an bestimmte Sequenzen in der DNA binden, oder aber seine Affinität zur Bindungsstelle ist reduziert. D.h., er kann nicht mehr an den IFNγ-Promotor binden, oder mit zu niedriger Affinität, und die IFNγ-Synthese auch nicht mehr hemmen. Eine Überproduktion von IFNγ wäre die Folge. Diese Überproduktion könnte dann zu einer Insulitis führen. Da IFNγ die TH1 Antwort aktiviert, indem er die MHC-Klasse-1-Proteine auf der Oberfläche von β-Zellen hochreguliert (85,86) und sogleich die T-Zelldifferenzierung in Richtung TH1 beeinflußt und dadurch die TH2-Antwort reduziert, wäre eine Verschiebung zwischen TH1 und TH2 gegeben, wie es beim autoimmunen Diabetes postuliert wird. Darüber hinaus ist bekannt, daß Makrophagen zuerst in der β-Zelle nachweisbar sind. Da IFNγ ein Stimulator für Makrophagen ist, kann die frühe Einwanderung in die β-Zelle dadurch erklärt werden, daß IFNγ nicht mehr durch YY1 gehemmt wird. Zusätzlich ist YY1 auch prädestiniert, die IL4 Synthese einzuleiten (83). Ist dieser Fakt durch Sequenzveränderung oder Unterproduktion von YY1 auch nicht gegeben, kann die TH1-Antwort nicht mehr durch IL4 als protektives Cytokin unterdrückt werden. Aufgrund einer erhöhten Aktivität von TH1 können cytotoxische T-Zellen (CTL oder CD8⁺) und NK-Zellen ihre Funktion in der β-Zelle als "Killer Zellen" wahrnehmen. Der apoptotische β-Zelltod, induziert durch FAS-Rezeptoren unter Interaktion mit dem FAS-Liganden, kann durch YY1 auch zusätzlich noch induziert werden (87). Ist der Transkriptionsfaktor bei Typ-1-Diabetikern mutiert, kann er nicht mehr in vollem Maße die FAS-Expression hemmen. Somit erscheint FAS auf der Oberfläche von β-Zellen, und die apoptotische Signalkaskade ist aktiviert. Das Resultat ist der β-Zelltod und damit Typ-1-Diabetes.

Da neonatale Thymektomie bei Modelltieren (BB-Ratte und NOD-Maus) die Entstehung eines Typ-1-Diabetes verhindert (88), wird u.a. auch vermutet, daß beim Typ-1-Diabetes autoreaktive T-Zellen schon während der T-Zelldifferenzierung entstehen. D.h., YY1 müßte schon in einem frühen Stadium der T-Zellentwicklung eingreifen, was man sich wie folgt vorstellen könnte:

Um ein funktionales Lymphozytenrepertoire zu entwickeln, müssen die Vorläuferzellen, sogenannte Präthymozyten, verschiedenste Entwicklungsstadien im Thymus durchlaufen. Da nur etwa 2% reifer T-Zellen den Thymus verlassen können, liegt eine strenge Selektion während der T-Zellreifung vor, welche über Tod oder Überleben entscheidet. Ein Regulationspunkt ist im frühen Thymozytenstadium, dem "Doppelt Negativen" (DN), verankert. Hier entscheidet der auf der Oberfläche befindliche prä-T-Zell-Rezeptor (preTCR), bestehend aus CD44 (β-Kette) und der invarianten Kette pTα, ob ein Eintreten in den Zellzyklus möglich ist. Dieser Kontrollpunkt wird auch als "Beta-Checkpoint" bezeichnet. Die unreifen T-Zellen verweilen solange in diesem Stadium, bis das richtige α-Ketten-Gen umgeordnet ist und der T-Zell-Rezeptor (TCR), bestehend aus α- und β-kette, auf der Oberfläche exprimiert werden kann. Erscheinen beide T-Zell-Rezeptoren CD4 plus CD8 zusammen mit CD3 auf der Oberfläche, werden diese T-Zellen nun als "Doppelt Positiv" (DP) bezeichnet. Etwa 95% erreichen dieses Stadium nicht. Dies unterstreicht die Bedeutung des Kontrollpunktes beim Übergang vom DN- zum DP-Stadium (89).

Als kritischer Gesichtspunkt wird dabei die Regulation der Expression der pTα-Kette betrachtet. Die pTα-Gentranskription scheint bisher vorallem durch den upstream Bereich, dem Enhancer, entscheidend reguliert zu werden. Mehrere Proteine, unter ihnen befindet sich auch YY1, die in der Lage sind, an diesen Bereich zu binden, wurden identifiziert (90). Als weitere Bindungsproteine des Enhancer sind zu nennen: SP1/3, ZBP-89 und c-MYC. Betrachtet man diese Proteine im Zusammenhang mit YY1, kann man schlußfolgern, daß alle, ausgenommen ZBP-89, mit YY1 interagieren, so daß als weiterer Regulationspunkt von YY1 auch die T-Zellreifung angesehen wird. Unterstützt wird diese Annahme durch Befunde von Iritani et. al. (91). Sie konnten zeigen, daß c-MYC allein nicht in der Lage ist, die pTα-Expression zu regulieren. Nur verschiedenste c-MYC Konzentrationen schienen zum Teil einen Einfluß auf den Zellwachstumsarrest im späten DP-Stadium zu haben. Ist YY1 im Thymus überexprimiert, schaltet er die Transkription pTα an. In umgekehrter Weise, bei Unterexpression, verhindert er die pTα-Transkription. Bei Überexpression oder Unterexpression, sowie Sequenzveränderungen von YY1 können T-Zellen während ihrer Entwicklung in den Arrest geschickt oder aber in ein weiteres T-Zellstadium überführt werden.

Da die T-Zellreifung und deren Selektion bei allen Autoimmunerkrankungen, wie Rheumatoide Arthritis, Multiple Sklerose, Autoimmune Encephalomyelitis, Zöliakie u.a., entscheidend ist, können neben dem Typ-1-Diabetes auch Autoimmunerkrankungen per se durch YY1 beeinflußt werden, was durch Auf- oder Abregulation von YY1 erreicht wird.

Es wird davon ausgegangen, daß sich die YY1-Sequenz zwischen Typ-1-Diabetiker und gesunden Probanden unterscheidet. Mehrere Sequenzvarianten werden für Typ-1-Diabetiker erwartet, da es neben dem klassischen Typ-1-Diabetiker auch noch weitere Formen für Typ-1-Diabetes existieren, wie zum Beispiel der LADA (Latent Autoimmune Diabetes in Adults). Diese Sequenzvarianten sollen als genetischer Marker für die Prädiabetiker genutzt werden. Der Nachweis der Änderung basiert auf der DNA-Sequenzierung, wobei Vollblut gewonnen, DNA bzw. RNA, die umgeschrieben wird in ssDNA, isoliert und dann sequenziert wird.

Parallel dazu wird das Expressionsprofil für Typ-1-Diabetiker erstellt. In Anbetracht der Tatsache, daß die Krankheit eines jeden Typ-1-Diabetikers individuell verläuft, wird erwartet, daß das Expressionsprofil von Proband zu Proband unterschiedlich ist. In Abhängigkeit vom Alter des Probanden, vom Geschlecht, vom Autoantikörperstatus [GAD (Glutamatdecarboxylase), IA-2 (Proteintyrosinphosphatase), ICA (zytoplasmatische Inselzellautoäntikörper)] vom BMI, sowie vom HLA-Genotyp (HLA-DQB1) werden unterschiedliche Expressionsprofile erwartet. Demnach soll eine entsprechende Prophylaxe/Therapie auch individuell zugeschnitten werden. Durch die genetische Heterogenität interagiert YY1 bei jedem Individuum mit einem differenten genetischen Hintergrund, so daß die YY1-Expression zwischen den Individuen großen Schwankungen unterliegen wird.

Eine Abregulation kann über Applikation von Antisense und durch Herstellung spezifischer Antikörper für den jeweiligen Probanden erreicht werden. Die Struktur der Antisense und der Antikörper ergibt sich aus der Sequenzfolge des Probanden. Die zu applizierende Menge der Antisense sowie Antikörper ergibt sich aus dem Expressionsprofil des Probanden (Auf- und Abregulation vgl. M&M).

Daß YY1 selbst als Autoantigen fungiert, liegt nahe, weil bis dato das auslösende Autoantigen für die Zerstörung der Insulin-produzierenden Beta-Zelle immer noch nicht bekannt ist. Liegt eine Bestätigung vor, daß YY1 als Autoantigen fungiert, soll über orale Verabreichung von YY1 Toleranz induziert werden, wie es bei Allergikern durch Hypersensibilisierung bereits erfolgt. Dabei soll für den jeweiligen Probanden oder Probandengruppen die Sequenzvarianten erfaßt und anschließend die mutierte Form synthetisch hergestellt werden, um es oral verabreichen zu können. Damit soll eine Verschiebung des TH1/TH2 Profils erreicht werden, um der autoimmunen Zerstörung entgegenzuwirken.

Daß durch Applikation von nackter cDNA oder DNA die Entstehung eines Typ-1-Diabetes bei BB/OK-Ratten verringert werden kann, zeigten jüngste Ergebnisse einer Pilotstudie. 174 Nachkommen nicht-diabetischer Muttertiere wurde mit 2, 8, 12, 16, 20, 25 und 30 Tagen nackte DNA der Primer K815 und K817, K831 und K818 (Figur 11) sowie gereinigte PCR-Produkte unter Verwendung der Primer K815 und K817 (Amplifikation genomischer BB/OK-DNA, Intron von YY1, Sequenz 1069 bis 1804) sowie K831 und K870 (Amplifikation von BB/OK-cDNA, Exon 3, 4 und 5, Zinkfinger, Sequenz 911 bis 1125 und 1758 bis 2078) dosisabhängig (100, 200 und 400 ng in 50 µl) einmal an den o.g. Tagen s.c. appliziert. Darüber hinaus wurden nicht-diabetische BB/OK-Weibchen gezielt angepaart, die bei Spermanachweis and dann am 4., 8., 12., 16. und 18 Schwangerschaftstag mit 100 oder 400 ng nackter DNA (Primer K815/K817) behandelt wurden. Die 24 Nachkommen dieser Mütter und die 174 post-partuum behandelten Nachkommen wurden bis zur 30. Lebenswoche auf Diabetesvorkommen beobachtet. Als Kontrolle dienten unbehandelte sowie behandelte BB/OK-Ratten, denen 50 µl Wasser s.c. im gleichen Alter appliziert wurde. Nach 30 Wochen waren 93% der behandelten (13/14) und 86% der unbehandelten Kontrolltiere (19/22) erkrankt. Eine recht deutliche Senkung der Diabeteshäufigkeit wurde in 2 Versuchsgruppen beobachtet. Die Häufigkeit sank auf 62% (8/13) bei Applikation des Zinkfinger-PCR-Produktes (400 ng) und auf 50% (6/12) bei den Nachkommen, die von dem mit 400ng behandelten Muttertier geboren wurden: Alle übrigen Versuchsgruppen erkrankten mit den der Kontrollgruppen vergleichbaren Häufigkeit (74 bis 100%), wobei eine Dosisabhängigkeit zu beobachten war. Eine weitere Studie zeigte, dass die Applikation von 400ng/50µl PCR-Produkt, amplifiziert mit den Primern K831/K870 die Diabetesinzidenz weiter senken konnte. Im Vergleich zur "Wasserkontrolle" (16/19=84%) erkrankten signifikant weniger BB/OK-Ratten (16/19 vs. 13/27; p=0.013)der behandelten Gruppe. Angesichts dieser ersten Ergebnisse wird davon ausgegangen, daß durch eine häufigere und/oder längere Applikation nackter DNA und/oder PCR-Produkten eine Senkung der Diabeteshäufigkeit bei BB/OK-Ratten gegen 0% erreicht werden kann.

### Protoonkogene und Krebs

Das c-MYC Gen wurde ursprünglich als zelluläres Homolog des retroviralen v-myc Onkogens in den 70er Jahren entdeckt. Embryonale Lethalität ist das Ergebnis einer Deletion dieses Gens. Das Produkt eines c-MYC Protoonkogens ist das c-MYC Protein, welches - ebenso wie YY1- einen multifunktioneller Transkriptionsfaktor darstellt. Eine entscheidende Rolle spielt c-MYC in der Differenzierung, dem Zellwachstum, Proliferation, Transformation und Apoptosis von Zellen. Die Dysregulationen der c-MYC Expression stehen im Zusammenhang mit abnormen malignen Zellwachstum und somit der Tumorentstehung von Lungenkrebs, Brustkrebs und Darmkrebs. Dies zeigt, daß c-MYC als starker Regulator des Zellwachstums, der Zelldifferenzierung und Zellproliferation ebenso stark kontrolliert wie balanciert werden muß, um die Carzinomentstehung zu verhindern (102-104).

Da YY1 als vielseitiger Regulator in Abwesenheit von dem essentiellen Partnerprotein (MAX) direkt an den C-terminalen Teil des basischen Helix-Loop-Helix (bHLH) Zinkfingers von c-MYC binden kann, spielt YY1 auch bei der Krebsentstehung eine bedeutende Rolle. Hierbei ist YY1 in der Lage, jeden seiner vier Zinkfinger dazu zu benutzen, um an das c-MYC Protein zu binden. Nicht möglich ist es YY1 jedoch, die Verbindung zwischen dem Komplex von c-MYC/MAX zu unterbrechen. Da aber trotz Komplexbildung zwischen YY1 und c-MYC die sequenzspezifische DNA-Bindung von YY1 nicht blockiert wird, ist die Regulation auf DNA-Ebene durch YY1 immer noch möglich.

Das humane c-MYC Gen kodiert zwei Polypeptide (439 und 453 Aminosäuren) mit einem Molekulargewicht von 64 und 67 kDa und ist im Zellkern lokalisiert. Der Translationsstart des größeren Proteins liegt am Ende des ersten Exons, während die kleinere und meist vorherrschende Form des Proteins durch Translation im zweiten Exon initiiert wird.

C-MYC ist ein Transkriptionsfaktor, der basische Helix-Loop-Helix/Leuzin-Zipper Domäne (bHLH-LZ) aufweist, wobei diese konservierten Domänen Bestandteil vieler Transkriptionsfaktoren sind und Protein/Protein, sowie Protein/DNA-Interaktionen vermitteln.

C-MYC ist Teil einer Genfamilie, die noch weitere Mitglieder wie N-, L-, S- und B-myc beinhaltet (105-108).

Die Bindung von c-MYC an die DNA erfolgt über die sogenannte E-Box, eine spezifische regulatorische Sequenz der Basen 5'-CACGTG-3'. Zur Aktivierung der Transkription muß das c-MYC Protein mit dem Partnerprotein MAX heterodimere Komplexe ausbilden (109). MAX gehört ebenfalls zu den bHLH-LZ Proteinen, besitzt aber keine transaktivierende Domäne wie c-MYC (110). Die Heterodimerisierung mit MAX ist essentiell für die Funktionen von c-MYC, wie Regulation des Zellzyklus, Einleiten von Apoptose oder Transformation von Zellen. MAX bildet weiterhin mit den bHLH-LZ Proteinen der MAD-Familie Komplexe. Diese Heterodimere binden ebenfalls spezifisch an E-Boxen und wirken transkriptionsreprimierend über Sin3-vermittelte Rekrutierung von Histondeacetylasen (111).

Demnach sollte durch Ab- oder auch Aufregulation von YY1, bedingt durch seine vielfältigen Interaktionen mit anderen Genen, auch über c-MYC, eine Beeinflussung der Krebsentstehung möglich sein, was durch folgende Beobachtungen unterstrichen wird.

BB/OK-Ratten entwickeln nicht nur einen Typ-1-Diabetes sonder auch Tumore wie eine Langzeitstudie zeigte. 202 BB/OK-Ratten wurden bis zu ihrem natürlichen Tod beobachtet.87 von den 202 wurden diabetisch. Die verbleibende 115 Tiere überlebten im Mittel 576 ± 79 Tage. Nur 37% (42/115)wurden älter als 400 Tage. Die Mehrzahl der Tiere (73/115) starb zwischen dem 200. und 400. Lebenstag vornhemlich an Tumoren (50/115). Betroffen waren Leber, Lyphknoten, Lunge, Darm, Pankreas und Milz (112). Analog wurde eine Studie mit BB.6S-Ratten durchgeführt. 47 Tiere wurden bis zum 600. Lebenstag beobachtet. 6 von 47 Ratten (12,8%) wurden bis zur 30. Lebenswoche diabetisch, 2 weitere Tiere manifestieren im Alter von 437 und 560 Tag. 62% der Tiere (24 von 39 nichtdiabetischen Tieren) überlebten den Beobachtungszeitraum von 600 Tagen. 6 Tiere starben, ohne makroskopisch eine Todesursache feststellen zu können. 9 Tiere mußten auf Grund ihres schlechten Allgemeinzustandes getötet werden, wobei als Todesursache in 7 Fällen Darmverschluß und bei 2 Tieren Tumore in der Leber diagnostiziert wurden. Demnach überleben die kongenen BB.6S-Ratten signifikant länger als BB/OK-Ratten und die Tumorrate ist signifikant niedriger bei BB.6S als bei BB/OK (50/115 vs. 2/39, p<0.0001). Angesichts dieser Befunde, scheint die ausgetauschte Region und damit YY1 bei BB,6S-Ratten die Tumorentstehung deutlich zu senken und die Lebenserwartung zu erhöhen. Demnach sollte durch Abregulation von YY1 die Tumorentstehung verhindert und die Lebenserwartung gesteigert werden können.

### Lipidstoffwechsel

### - Sterbidhormonsynthese

Steroide werden in spezialisierten Zellen der Nebennieren, Eizellen, Hoden, Placenta und im Gehirn synthetisiert. Sie sind essentiell für die Aufrechterhaltung der normalen Körperhomöostase. Die Synthese aller Steroidhormone beginnt mit der Umwandlung von Cholesterol in Pregnenolon. Dies ist der erste enzymatische Schritt, der an der Matrix der innermitochondrialen Membran erfolgt.

Das Steroidogenic Acute Regulatory Protein (StAR) spielt eine Schlüsselrolle im Transport von Cholesterol von der äußeren zur innermitochondrialen Membran. Dieser Transport ist der ratenlimitierende Schritt in der Steroidogenese. Mutationen im StAR-Gen verursachen die potentiell lethalen Bedingungen, die als congenital lipoid adrenale Hyperplasie bekannt sind. Gleiche Beobachtungen konnten an StAR-Knock-out Mäusen gemacht werden (131).

StAR-Expression kann durch Agenzien positiv und negativ reguliert werden, die vorwiegend am Promotor wirken. Die hormonstimulierte Steroidsynthese ist begleitet von einem schnellen Anstieg der StAR-mRNA-Spiegel. cAMP hat einen positiven und schnellen Effekt auf den Anstieg der StAR-mRNA, scheint aber nicht direkt an der Promotorsequenz zu wirken.

Der erste Transkriptionsfaktor als potentieller Regulator des StAR-Gens war der Steroidogenic factor 1 (SF 1), auch Orphan Nuclear Rezeptor Transkriptionsfaktor genannt.

Der StAR-Promotor besitzt verschiedene Consensus-Bindungssequenzen für SF 1. Zwei von diesen an Position -97 und -42 sind hoch konserviert. Eine weitere an Position -132 wurde nur in Mäusen und Ratten gefunden.

Ein weiterer Kandidat ist das CCAAT/enhancer Bindungsproteine (C/EBPs), das zur Familie der bRegion/leucine zipper Transkriptionsfaktoren gehört. Zwei Mitglieder dieser Familie werden in steroidogenen Zellen exprimiert (C/EMPα und C/EMPβ). Der StAR-Promotor hat zwei mögliche Bindungssstellen für C/EMP. SF 1 und C/EMP bilden einen Komplex am StAR-Promotor.

Sowohl der humane als auch der Ratten-StAR-Promotor besitzt weiterhin Bindungsstellen für SREBP-1a (Sterol regulatory element binding protein-1a). SREBP-1a ist ein bedeutender Aktivator für den StAR-Promotor.

Weitere Transkriptionsfaktoren wie SF 1, NF-Y, YY1, und SP1 sind in die Wirkung von SREBP am StAR-Promotor involviert. SREBP-1a reguliert koordinierend zusammen mit den anderen Faktoren den StAR-Promotor.

CREB kann ebenfalls binden und auf schnelle Weise die Transkription aktivieren (cAMP Bindung).

DAX-1 bindet direkt an der Hairpin-Struktur des StAR-Promotors sowie direkt an SF-1 und inhibiert in beiden Fällen deren Expression (131).

Wenn SREBP-1a eine koordinierende Rolle in der Bereitstellung von Cholesterol in der Steroidhormonsynthese übernimmt, kommt ihm eine essentielle Bedeutung in der Cholesterolhomöostase zu. Der StAR-Promotor der Ratte besitzt 5 sogenannte SRE-Bindungsstellen (Sterol Regulatory Element), an die die aktivierte Form von SREBP-1a binden und die Transkription aktivieren kann (132). Eine weitere SRE-Bindungsstelle wurde im humanen StAR-Promotor gefunden, an die sowohl SREBP-1a als auch YY1 binden kann und bedingungsabhängig bei hohen Konzentrationen von SREBP-1a aktiv ist (133). SREBPs gehören zur Familie der basic helix-loop-helix leucine zipper (bHLH-Zip) Transkriptionsfaktoren, die als 125 kDa membrangebundene Precursor-Proteine im endoplasmatischen Retikulum synthetisiert werden und bei Sterolmangel in der Zelle durch enzymatische Abspaltung der 68 kDa N-terminalen, die bHLH-Zip Domäne enthaltenden Region des Proteins aktiviert werden und in den Nucleus einwandern. Dort binden sie spezifisch an die DNA-Sequenz von SRE (sterol regulatory element) und aktivieren die Transkription ihrer Zielgene (134, 135).

Es wurden bisher drei SREBPs beschrieben, die SREBP-1a, SREBP-1c und SREBP-2 genannt werden. SREBP-1a und -1c werden vom gleichen Gen unter Nutzung alternativer Promotoren exprimiert. SREBP-2 wird von einem separaten Gen exprimiert (135, 136).

Cholestolgene, die klassische SRE-Bindungsstellen in ihren Promotoren besitzen, werden stark und effektiv von SREBP-1a und SREBP-2 aktiviert. SREBP-1c ist inaktiv für diese Bindungsstellen.

SREBP-1c, auch ADD 1 genannt, weist eine Besonderheit auf. Es bindet an E-Boxen (universelle cis-Elemente für bHLH-Proteine). Damit weist es eine duale Bindungsspezifität zu beiden, den klassischen palindromen E-Boxen und den nonpalindromen SREs, auf. Diese einmalige Bindungsspezifität ist auf den Thyrosinrest in der basischen Region zurückzuführen, der einmalig für die SREBP-Familie ist, denn alle bekannten bHLH-Proteine besitzen an dieser Position Arginin (136). Dieser Austausch zerstört die Transaktivität aller SREBPs für SRE-Bindungsstellen, erhöht aber markant die Aktivität von SREBP-1 für Bindung an E-Boxen (siehe SREBP-1c) aber auch von SREBP-2. Dieses ist jedoch inaktiv und aktiviert das Zielgen nicht (136).

Die SREBPs sind unterschiedlich starke Transkriptionsfaktoren und benötigen meist Cofaktoren. Solche sind NF-Y, SP1, CBP (CREB-Bindungsprotein).

SREBP-Zielgene werden in 2 Gruppen eingeteilt(136):
- Cholesterol-Biosynthese-Gene
   • HMG-CoA-synthase und -reduktase
   • Farnesyl-diphosphatsynthase
   • Squalensynthase
   • SREBP-2
   • LDL-Rezeptor
   • HDL-Rezeptor (137)
      Alle enthalten die klassische SRE-Sequenz (ATCACCCCAC) oder das SRE 3 Motif (CTCACACGAG) und angrenzende Cofaktor-Bindungsstellen für NF-Y oder SP1 in ihren Promotoren.
- Lipogene Enzym-Gene
   Sind nahrungsreguliert auf der Transkriptionsebene (z.B.Glukose, Insulin)
   • Acetyl-CoA-Carboxylase
   • Fettsäuresynthase (FAS, erhöht TG)
   • Steryl- CoAdesaturase 1 und 2
   • Glycerol-3-phosphat-Acyltransferase
   • Diacepam-binding Inhibitor (Acyl-CoA-Bindungsprotein)
   • Spot 14 (Leberenzym S 14)
      Die SREBP-Bindungs- und -Aktivierungsstellen in den Promotoren dieser Gene scheinen sich von den klassischen SRE-Cohsehsus-Sequenzen zu unterscheiden und werden als SRE-like-Sequenzen bezeichnet.

Einige weitere Enzyme wie Liver-Type-Pyruvatkinase (PK) und Glukokinase (GK) enthalten E-Box oder E-Box-like Sequenzen im Promotor, was bedeuten kann, daß sie Kohlenhydrat, Glukose- und Insulin-sensitiv sind und potentiell SREBP-Targets sein können.

Alle aktiv wachsenden Zellkulturen produzierten prädominant SREBP-1a und SREBP-2, wogegen die meisten Organe einschließlich Leber von adulten Tieren SREBP-1c und SREBP-2 produzieren. Alle SREBPs sind in der Lage, jedes der bekannten Zielgene zu aktivieren, jedoch mit unterschiedlicher Effizienz. SREBP-1c ist schwächer (kürzere Transaktivierungsdomäne) als SREBP-1a und SREBP-2.

Die spezifische Rolle der SREBP-Isoformen in vivo wurde in transgenen Mäusen untersucht Die differente Überexpression ergab, daß die SREBP-1-Isoformen selektiv die Fettsäurebiosynthesegene aktivieren und SERBP-2 spezifisch die Cholesterol-biosynthese kontrolliert.

SREBP-1a und -1c spielen eine große Rolle in der nahrungsabhängigen Induktion hepatisch lipogener Enzyme und der Cholesterogenese. SREBP-2 dagegen bestimmt die Sterolregulation durch Abbau des membrangebundenen Precursor-Proteins, um die aktive Form für die Einwanderung in den Nukleus bereitzustellen. SREBP-1 kontrolliert lipogene Enzyme durch Selbstregulation seines eigenen Transkriptionslevels.

Der Fakt, daß die SREBPs relativ schwache Transkriptionsfaktoren sind und Cofaktoren benötigen, wurde bereits erwähnt. Bennett et.al. (138) zeigten beispielsweise, daß die Aktivierung der SREBPs durch Sterolmangel in vivo in einer erhöhten Bindung von SP1 an einer, an die SREBP-Bindungsstelle angrenzenden Sequenz im Promotor für das LDL-Rezeptorgen bindet. Ähnlich werden die zwei coregulierenden Faktoren NF-Y und CREB (cAMP Responsive Element Binding Protein) verstärkt an den Promotor von HMG CoA-Reduktase gebunden. SREBP-Aktivierung erhöht die Histonacetylierung von H 3, nicht aber von H 4 im Chromatin beider Promotoren (HMG-CoA-Synthase und -Reduktase). Die Resultate zeigen, daß feine Differenzen im Muster der Kern-Histon-Acetylierung eine Rolle in der selektiven Genaktivierung spielen. Das zeigt, daß die Bindung der Transkriptionsfaktoren an die DNA eine notwendige, aber nicht ausreichende Bedingung für die Transaktivierung ist (138).

SREBP-1 kann einige andere E-Box oder E-Box-like-Sequenzen im FAS-Promotor und im S14-Promotor aktivieren, ist aber völlig inaktiv für degenerierte E-Box-Sequenzen z.B. im PK Promotor. Promotoren mit SRE-Bindungsstellen benötigen unbedingt NF-Y oder SP1 als Cofaktoren für die SREBP-Aktivierung. SREBP-1 hat eine wesentlich höhere Affinität und ist effizienter in der Aktivierung von SRE enthaltenden Promotoren als von E-Box enthaltenden Promotoren.

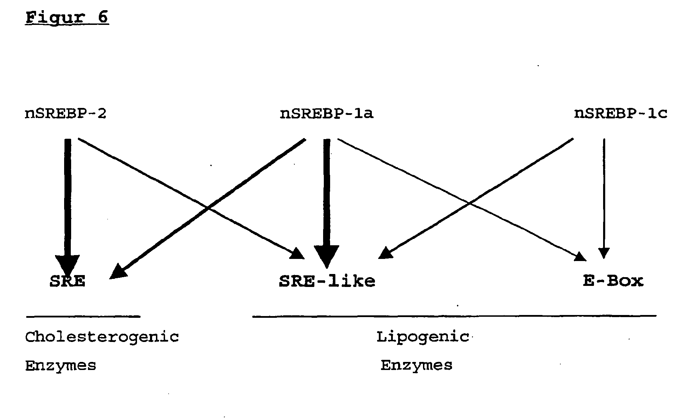

In lipogenen Enzymen, die Promotoren mit SRE-like-Sequenzen enthalten, aktivieren alle Isoformen von SREBP die Transkription (SREBP-1a stärker als SREBP-1c, SREBP-1a bevorzugt gegenüber SREBP-2). Die Bindung der SREBP-Isomeren an die verschiedenen Bindungsdomänen der Genpromotoren (136) ist in Fig. 6 dargestellt.

### - Einfluß von YY1 auf die Regulation der lipogenen Gene

YY1 ist bekannt als Transkriptionsmodulator, der sowohl als Enhancer und Repressor aber auch als Initiator-Bindungsprotein wirken kann, was von der Herkunft der Zelle und den Bindungsstellen des Promotors abhängig ist. YY1 ist auch in der Lage, den gleichen Promotor zu aktivieren und zu hemmen, wenn das intrazelluläre Milieu verändert ist, das Bindungselement im Promotor mutiert (verändert) ist, oder die Bindungsstelle umgebende DNA-Sequenz alteriert ist.

In Hinblick auf die betrachteten Gene lassen sich folgende Einflüsse von YY1 folgern.

YY1 besitzt eine hohe Affinität zu SREBP-1a. Im StAR-Promotor befindet sich eine YY1-Bindungsstelle, die mit der proximalen Bindungsstelle für SREBP-1a überlappt. Die gleichzeitige Bindung von SREBP-1a und YY1 am StAR-Promotor reprimiert dessen Transkription. Dieser Fakt beeinflußt wiederum den Cholesteroltransport und somit die Steroidhormonsynthese.

YY1 wirkt als Aktivator spezifischer Repressor, konkurriert mit den Bindungsstellen der SREBPs und deren Cofaktoren bzw. behindert sterisch die Bindung der SREBPs z.B. an SREs der lipogenen Gene. Er wirkt sowohl als Typ-I-Repressor (Bindung an Cofaktoren, Komplexbildung) als auch als Typ-II-Repressor (direkte Bindung an die DNA, sterische Blockierung von Bindungsstellen). Durch Mutation der Bindungsstelle in YY1 konnte nachgewiesen werden, daß die SREBP-1a Aktivierung des StAR-Promotors um ein Vielfaches anstieg. Untersuchungen an transfizierten HepG2 Zellen ergaben, daß YY1 Mutanten (YY Δ 296-331, YY Δ 399-414, YY Δ 334-414 oder YY Δ 154-199) z.B. die sterolaktivierte Expression des LDL-Rezeptor und FPPS-Rezeptors nicht reprimierte. Die Mutationen YY Δ 334-414 oder YY Δ 154-199 verhindern die nukleare Lokalisation, die DNA-Bindung und die Interaktion mit CBP. YY Δ 296-331, YY Δ 399-414 waren inaktiv bei der Repression der SREBP-1a abhängigen Induktion der Expression des HMG-CoA Synthase-Promotors (134).

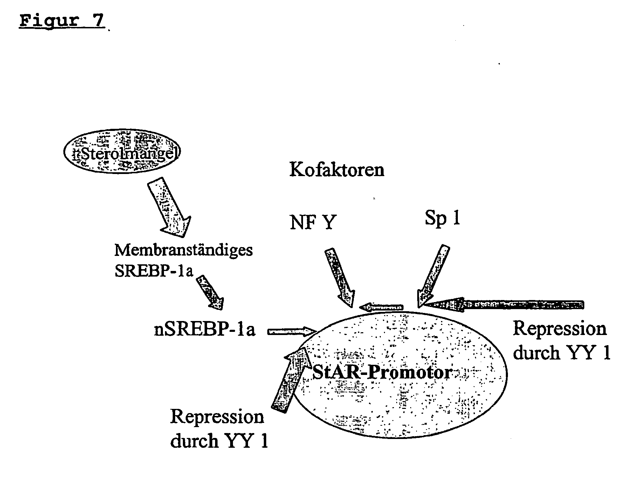

Zahlreiche weitere Promotoren der sterolregulierten Gene enthalten potentielle Bindungsstellen für YY1 (CCAT oder ACAT) überlappend oder angrenzend an Bindungsstellen der Coaktivatoren bzw. Transkriptionsaktivatoren. Diese können durch YY1 verdrängt oder blockiert und damit die Genexpression reprimiert werden (vgl. Fig. 7).

Nach dem gleichen Prinzip werden auch die anderen Promotoren durch YY1 reprimiert.

HDL-Rezeptor → positiv reguliert durch SREBP-1a plus SP1 ↔YY1 (Repr.)

LDL-Rezeptor → positiv reguliert durch SREBP-1a plus SP1 ↔YY1 (Repr.)

Fettsäuresynthase

Farnesyl Synthase → positiv reguliert durch SREBP-1a plus NF-Y ↔ YY1(Repr.)

HGM-Co A Synthase

HGM-CoA-Reduktasegenexpression wird durch YY1 nicht reprimiert.

Der Synergismus der Bindung von SREBP und NF-Y bzw. SP1 an Promotoren der SREBP-responsiven Gene, einschließlich SREBP-2, führt zur Aktivierung der Transkription, die die Cholesterolhomöostase (LDL-Rezeptor, HDL-Rezeptor, HMG-CoA-Synthase und - Reduktase, FPP(Farnesylphosphat)-Synthase, Squalensynthase) die Fettsäuresynthese (Fettsäuresynthase und AcetylCoA-Acetylase), den Fettsäureabbau (Stearyl-CoA-Desaturase) und die Triglyceridsynthese (Glycerol-3-phosphat-Acetyltransferase) kontrolliert (134).

YY1 kann über die o.g. Mechanismen in diese Prozesse eingreifen. YY1 kann auch durch Interaktion mit SREBP-1a die Erkennung der Domäne auf SREBP-1a für die RNA-Polymerase-II behindern und damit die Transkription reprimieren, was einen weiteren regulatorischen Mechanismus der Transkriptionsregulation darstellt (134).

### Beeinflussung der SREBP Aktivierung bei verschiedenen Erkrankungen

In hepatischen Zellen wird vorwiegend SREBP-1c exprimiert, das in diesen Zellen auch durch Glukose und Insulin sowohl auf mRNA als auch auf Proteinebene stimuliert wird (139).

Bei experimentellem Streptozotozin-Diabetes von Ratten wurde eine massive Erhöhung der Expression von SREBP-1a und Fettsäuresynthase gefunden. Daraus resultierte eine starke Triglyceridakkumulation (TG). Behandlung mit Insulin konnte beides senken. In Nierenzellen war bei hoher Glukose die Expression von SREBP-1a und -1c mRNA und Proteinsynthese erhöht ebenfalls die Fettsäuresynthese und damit die TG-Akkumulation. SREBP-1-Expression ist bei Diabetes mellitus erhöht. SREBP-1 spielt eine bedeutende Rolle in der Erhöhung der Lipidsynthese, TG-Akkumukation, mesangialen Expansion, Glomerulosklerose und Proteinurie, indem es die Expression von TNFβ und des vaskulären Endothelwachstumsfaktors erhöht (140).

Bei Typ-2-Diabetikern wurde eine signifikante Verminderung der SREBP-1-Expression im subkutanen Fettgewebe und im Skelettmuskel gefunden. Ex vivo konnte dieser Effekt durch TNFα behoben werden (141).

Daher kann durch Auf- bzw. Abregulation von YY1 der Lipidstoffwechsel entsprechend beeinflußt werden wie nachfolgende Ergebnisse bestätigen.

Wie eingangs bereits erwähnt, unterscheiden sich BB/OK- und BB.6S-Ratten nicht nur signifikant in der Diabeteshäufigkeit und Manifestationsalter. Im Alter von 12 Wochen sind BB.6S-Ratten im Vergleich zu BB/OK auch schwerer und das Serumgesamtcholesterol ist bei männlichen und weiblichen Tieren und die Serumtriglyzeride sind bei Weibchen signifikant erhöht (24). Nach der 24. Lebenswoche sind auch die Serumtriglyzeride bei den männlichen BB.6S-Ratten signifikant gegenüber BB/OK erhöht. Auf Grund dieser Befunde lag der Schluß nahe, daß YY1 auch im Fettstoffwechsel involviert ist. Daher wurde in einer Pilotstudie geprüft, inwieweit die Applikation von YY1-Antisense der 4 Zinkfinger die Lipide beeinflussen kann. 10-12 männlichen Ratten der Stämme BB/OK, LEW.1A, LEW.1W, WOKW und SHR wurden 600ng/100µl Antisense für 2 Wochen, beginnend in der 9. Lebenswoche, appliziert. Die Serumtriglyzeride (TG), - gesamtcholesterol (Chol) und - HDL-Choelesterol (HDL) wurden vor (8. Woche) und nach Applikation (10. Woche) bestimmt. Die genetisch und phänotypisch differenten Stämme reagierten erwartungsgemäß unterschiedlich wie nachfolgend zusammengestellt ist.

| Stamm | TG | Chol | HDL | HDL/Chol-Ratio# |
|---|---|---|---|---|
| BB/OK | ↑* | | | ↑** |
| BB.LL | ↑* | | | |
| LEW.1A | | ↓** | ↓* | ↑* |
| LEW.1W | | ↓** | ↓** | |
| SHR | ↓* | ↓** | ↓** | ↑** |
| WOKW | | ↓** | ↓** | |

| | | | | |
|---|---|---|---|---|
| ↑ ↓ signifikant erhöht bzw. erniedrigt. * p<0.05 ** p<0.01 # HDL/Cholesterol-Ratio | | | | |

Interessanterweise erkrankten von den 12 BB/OK-Ratten bis zur 30. Lebenswoche nur 7 Tiere (58%), was den diabetesprotektiven Effekt von Yy1-PCR-Produkten erneut unterstreicht (s.S.32).

Wie für den Typ 1 Diabetes beschrieben sollte es in Abhängigkeit vom Expressionsprofil der Probanden möglich sein, auch die Blutfette zu beeinflussen (vgl. Material & Methoden-Teil).

### Vitamin D, Calciumstoffwechsel und Entwicklungsprozesse

Vitamin D gehört neben dem Parathormon (PTH), Calcitonin (CT) und dem PTH related Peptide (PTHrP) zu den Hauptregulatoren des Calciumstoffwechsels. Seit 1966 ist bekannt, daß Vitamin-D in einer aktiven Form vorliegen muß, um seine funktionelle Aufgaben wahrnehmen zu können (143). Die aktive Form entsteht durch die Hydroxylierung am C25 sowie am C1 Atom. Bezeichnet wird diese Form als 1,25-Dihydroxycholecaliferol oder als Vitämin-D₃. Eine Aufgabe des Calciferols besteht darin, der Absenkung des Plasmacalciumspiegels entgegen zu wirken. Erreicht wird dies über eine vermehrte intestinale Calciumresorption, durch gesteigerte renale Calciumresorption und durch gesteigerte Calciummobilistion aus dem Skelettsystems (144). Die intestinale Resorption benötigt ein aktives Transportsystem (Calcium bindende Proteine, Calbindin), das nur in Anwesenheit von Vitamin-D₃ zu finden ist. Darüber hinaus ist Vitamin-D₃ auch für die Erhaltung der Funktionsfähigkeit der intestinalen Mucosazellen verantwortlich. Calbindine, Osteocalcin, Matrix-Gla-Protein, Osteopontin, Kollagen Typ I etc. werden durch Calciferol induziert. Einfluß auf die Proliferation, Differenzierung, Hormonsekretion und Apoptose werden auch durch Calciferol ausgeübt. Die Bedeutung des Vitamin-D₃ wird an Mangelerscheinungen, sogenannten Hypovitaminosen (Rachitis, Osteomalazie) deutlich (144).

Mit der Entdeckung des Vitamin-D-Rezeptors (VDR; 145, 146) konnte der Wirkmechanismus untersucht werden. Mit Hilfe des VDR konnte gezeigt werden, daß Vitamin-D im Kern von Keratinozyten der Haut, Langerhansschen Inseln des Pankreas, Lymphozyten, Promyelozyten etc. lokalisiert ist. Die aktive Form des Vitamin-D ist lipophil, so daß es über Diffusion durch die Zellmembran in die Zelle und weiter in den Zellkern gelangt und dort mit hoher Affinität an seinen Rezeptor bindet. Der VDR gehört zu der Familie der ligandengesteuerten Transkriptionsfaktoren. Nach Aktivierung durch seinen Liganden, moduliert VDR die Transkription solcher Genabschnitte, die ein dem Rezeptor zugeordnetes DNA-Element tragen, die sogenannten Hormone-Response-Elemente (HRE). Für den VDR werden diese Genabschnitte Vitamin-D-Response Elemente (VDREs) genannt. Gene, die VDRE enthalten, sind u.a. Osteocalcin (OC), Calbindin D9k (CALB3) und D28k (CALB1), Vitamin-D 24-Hydroxylase (CYP24), Osteopontin (OPN), atriale natriuretische Peptid (ANP), Parathormon (PTH), Carbohanhydrase II (CA-II), Integrin, beta-3 (ITGB3), Fibronektin (FN1), c-fos, Parathormon-related Peptid (PTHrP), "slow myosin heavy chain 3" (slowMyHC3), gewebespezifischer Plasminogenaktivator (t-PA), Wachstumsfaktor 1 (PIT1; POU1F) und Involucrin (IVL).

Die Studie von DeLuca et al. (147) zeigte, daß sich die aktive Form des Vitamins auch in Lymphozyten (CD4 und CD8), insbesondere in aktivierten T-Lypmphozyten befindet. Immunsupression von Autoimmunkrankheiten konnte auf die aktive Form von Vitamin-D₃ und Ca²⁺ zurückgeführt werden. Da im Rahmen der vorliegenden Erfindung gezeigt werden konnte, daß bei Ca²⁺-reicher Diät die Diabetesinsidenz von BB.6S-Ratten von 15 auf 45% erhöht werden kann, muß hier ein weiterer Regelmechanismus von YY1 existieren.

Guo et al. (148) konnte erstmals die Rolle von YY1 im Calciumstoffwechsel für das Osteocalcingen zeigen. YY1 reprimiert die 1,25-Dihydroxcholecalciferol vermittelte Transaktivierung des knochenspezifischen Osteocalcingens. Aufgrund der Interaktion von YY1 mit beiden Komponenten, VDR und Transkritionsfaktor IIB (TFIIB), ist er in der Lage, die von Vitamin-D abhängigen Transkriptionen zu regulieren. Darüber hinaus konkurriert das VDR/RXR (Retinoid X Receptor) -Heterodimer mit YY1 um die Bindungsstellen der VDR-Elemente im Osteocalcingen. Da VDREs in vielen Genen vorhanden sind, übernimmt YY1 dort weitere regulatorische Rollen.

Zusätzlich ist es YY1 möglich, bereits in die Synthese von Calciferol einzugreifen. Durch die Repression der Transkription des Enzyms, 25-Hydroxyvitamin-D3-24-hydroxylase [24(OH)ase; CYP24], ist der Katabolismus gestört. Eine erhöhte Repression konnte in Anwesenheit von TFIIB oder CBP (CREB-Binding-Protein) festgestellt werden (149). Darüber hinaus konnte bei transgenen Ratten, bei denen CYP24 überexprimiert wurde, gezeigt werden, daß diese Tiere eine Albuminurie und Hyperlipidämie entwickelten. Eine Beobachtung, die in diesem Zusammenhang an die erhöhten. Blutfette der BB.6S-Ratten im Vergleich zum Parentalstamm, der BB/OK-Ratte, erinnert. Auch entwickelten diese transgenen Ratten atherosklerotische Läsionen an der Aorta (150).

Daß der VDR im Zusammenhang mit dem Typ1-Diabetes steht, konnte von der Gruppe Chang et al. (151) gezeigt werden. Als direkter negativer Faktor fungiert die aktive Form von Vitamin-D auch im Renin-Angiotensin-System. Dieses System spielt eine wichtige Rolle in der Regulation des Flüssigkeits- und Elektrolythaushalts und somit auch in der Blutdruckkontrolle. Bei Abweichung des Vitamin-D-Levels kommt es zur direkten Inhibierung der Renin-Gen-Expression. Somit spielt Vitamin-D nicht nur als Regulator bei der Aufrechterhaltung der Calcium-Hömostase eine Rolle sondern auch im Hömostase-Prozess der Elektrolyte, dem Blutvolumen und Blutdruck (152). Unterstützend dazu kann die Studie von Bhalla et al. (153) herangezogen werden. Man konnte zeigen, daß YY1 synergistisch mit GATA-4 als transkriptioneller Komplex über CBP/p300 die "brain natriuretic" Peptid (BNP)-Gen-Transkription aktiviert. BNP gehört zur Familie der natriuretischen Peptide, die aus 3 Peptiden (atriale natriuretische Peptid, ANP; BNP; natriuretische Peptid Typ C, CNP) besteht. Ihre Hauptwirkung liegt in einer Zunahme der Natriumausscheidung, in einer Gefäßerweiterung (Vasodilatation) und in einer Abnahme der Aldosteronsekretion (wichtigstes Mineralocorticoid, beeinflußt den Natrium-, Kalium- und Wasserhaushalt in sämtlichen Geweben), womit sie den eigentlichen Gegenspieler des Renin-Angiotensin-Aldosteron-Systemes darstellens GATA-4 gehört zu der Familie der GATA-Zink-Finger-Transkriptionsfaktoren und ist im adulten Gewebe des Herzens, im Darmepithel und Gonaden exprimiert. Während der Fetalentwicklung ist GATA-4 bei der Herzformation beteiligt. Daher fungieren GATA-4 als auch YY1 gleichermaßen als Schlüsselelement bei der myocardialen Differenzierung und Funktion. Beide beeinflussen zahlreiche Herz-Gene (154-158). Unter Anderem konnte gezeigt werden, daß YY1 in Verbindung mit der Hypertrophy in Herzmyocyten (über IL1β vermittelt) steht (159).

In Anbetracht der bisher vorliegenden Arbeiten, sowie eigener Ergebnisse (s.o.), kann der Schluß gezogen werden, daß YY1 in den Calciumstoffwechsel selbst eingreift oder aber durch Calcium (und deren Komponenten) beeinflußt wird. Außerdem kann davon ausgegangen werden, daß YY1 eine wesentliche Rolle bei der Blutdruckregulation und Muskelkontraktion zukommt. Daher soll je nach betroffenem Gewebe und in Abhängigkeit vom Geschlecht YY1 ab- oder aufreguliert werden.

Mit Hilfe von Maus-Embryonen konnte dargestellt werden, daß YY1 in dem Entwicklungsprozess von Knochen eine bedeutende Rolle hat. Er ist in der Lage das MSX2 Gen zu aktivieren (160). MSX2 gehört zu der Klasse der Homöobox-Genen und wird in zahlreichen Geweben von Embryonen exprimiert. Als Schlüsselmediator ist MSX2 während verschiedenster Entwicklungsprozesse, wie zum Beispiel bei der Formation von Schädel, Zähnen, Augen, sowie in der cranio-facialen Morphogenese beteiligt. Er ist involviert in der epithelialen-mesenchymalen Interaktion und Apoptose (161-165). Bei Dysregulation des Expressionslevel von Mxs2 konnte abnormes Wachstum festgestellt werden. Zwei Faktoren, BMP4 (Bone Morphogenetic Protein Typ 4) und YY1 regulieren die Expression von MSX2 in embryonalen Geweben (166). Unabhängig von BNP4 ist YY1 im Stande an drei Stellen des Promotors von MSX2 zu binden und somit zu aktivieren (160). Die Involvation von YY1 in die Prozesse der Embryogenese, Neuronal-, Skelettmuskel- und Knochenentwicklung läßt den Schluß zu, daß YY1 mit Sicherheit bei Mutation oder Fehlregulation an Erkrankungen der Muskeln, Knochen und dem Gehirn entscheidend beteiligt sein wird (167). Daher kann auch hierbei je nach betroffenem Gewebe und in Abhängigkeit vom Geschlecht, YY1 ab- oder aufreguliert werden.

### Nutzung der Erkenntnisse

### 1. Diagnostik

### DNA-Sequenz und Genexpression

Ausgehend von der vorliegenden YY1 Sequenz werden unter Verwendung der in Fig. 11 aufgeführten Primer Sequenzänderungen in YY1 zur Diagnose von möglichen Fehlleistungen genutzt. Dazu wird genomische DNA und ssDNA aus mononukleären Blutzellen mit den Primern amplifiziert und sequenziert bzw. Punktmutationsanalysen (SNPs) durchgeführt (vgl. Kwok P.Y. SNP genotyping with fluorescence polarisation detection. Hum. Mutat. 19, 2002, 315-323).

Darüber hinaus wird RNA aus mononukleären Blutzellen und Gewebe durch Biopsie (Muskel, Fettgewebe, Haut) bzw. durch Punktion von Niere und Leber gewonnen, umgeschrieben in ssDNA und sequenziert (Umschreibung in ssDNA) sowie zur Genexpressionsanalyse eingesetzt, wie oben beschrieben (vgl. S.22/23, "Genexpressionsstudien"). Es wird davon ausgegangen, daß in den Geweben unterschiedliche Expressionsmuster und auch Spleißvarianten (s. verkürzter Zinkfinger bei BB.6S in Leber, Pankreas und Hirn) auftreten können und wichtige Hinweise zur Regulation von YY1 in den Geweben und zur Erkrankung geben.

Diese Gewebe kommen in Betracht, da beim Typ-1-Diabetes diabetische Folgeerkrankungen auftreten können, die sich in verschiedenen Geweben manifestieren (diabetische Nephropathie, Retinopathie, Neuropathie etc.). Folgeerkrankungen sind u.a. Fettstoffwechselstörungen (Fettgewebe, Leber), Bluthochdruck (Niere), Herz-Kreislauferkrankungen (Muskelgewebe) aber auch dermatologische Erkrankungen (Haut). Da nicht alle Diabetiker gleichermaßen an allen Folgeerkrankungen leiden, wird erwartet, daß das Expressionsprofil von Proband zu Proband unterschiedlich ist.

### ELISA (Enzyme-linked immunosorbent assay)

Um serolögische Tests zur Diagnostik durchführen zu können, kann die Sequenz genutzt werden, um verschiedene ELISA-Verfahren zu etablieren. Dafür werden monoklonale und polyklonale Antikörper erzeugt.

Herstellung von polyklonalen und monoklonalen Antikörpern (s. Ref. 89)

Es werden erfindungsgemäß polyklonale Antikörper gegen YY1 hergestellt. Zur Erzeugung können Kaninchen genutzt werden.

Das Protein YY1, synthetisch hergestellt und individuell für den Probanden, wird mit einem sogenannten vollständigen Freundschen Adjuvans versetzt. Das Adjuvans schützt das Protein YY1 vor der Degradierung. Das Freundsche Adjuvans besteht aus einer Mischung von Paraffinöl und Manidmonooleat, der inaktivierte und getrocknete Tuberkulosebakterien (Mycobacterium tuberculosis) zugesetzt wurden. Dies bewirkt im Tier eine allgemeine Immunreaktivitat, die primäre Immunantwort. Das Gemisch wird intradermal, subkutan oder intramuskulär ins Kaninchen injiziert. Nach ungefähr vier bis sechs Wochen wird das gleiche Protein mit inkompletten Adjuvans (Bakterien fehlen) erneut injiziert, um eine sekundäre Immunantwort auszulösen. In Abhängigkeit vom Antikörpertiter wird das Tier entblutet und das Antiserum gewonnen.

Für die Herstellung monoklonaler Antikörper (mAK) wird die von Köhler und Milstein entwickelte Hybridoma-Technik angewandt.

Mäuse werden mit dem YY1-Protein, daß in Abhängigkeit von der DNA-Sequenz des Probanden synthetisch hergestellt wird, immunisiert. Die antikörperproduzierenden Milz-Lymphozyten werden anschließend in Kultur mit Myelomzellen (Krebszellen) fusioniert in Anwesenheit von Polyethylenglykol. Nach Fusionierung werden die sogenannten Hybridome in Mikrotitertestplatten (0,2 ml Volumen) verteilt und mit dem HAT (Hypoxanthin-Aminopterin-Thymidin) -Selektionsmedilim kultiviert. Das Medium gewährleistet, daß nur Hybridome wachsen. Nach 10 Tagen Kultur werden die Hybridome vereinzelt und in Kultur weitervermehrt. Die mAK werden aus den Zellkulturuberstanden isoliert.

### Reinigung der Antikörper (Affinitätschromatographie) (s. Ref. 89)

Da Antiseren neben den spezifischen Immunglobulinen, die gegen YY1 gerichtet sind, noch weitere Antikörper enthalten, soll mittels einer Affinitätschromatographie die Aufreinigung erfolgen. YY1 wird dabei an eine feste Matrix gebunden. Nur Antikörper, die spezifisch gegen YY1 gerichtet sind, binden. Alle anderen Antikörper passieren die Säule. Die spezifischen Antikörper werden durch pH-Wert Veränderung (auf 2,5 oder über 11) von YY1 eluiert.

Diese spezifisch aufgereinigten Antikörper gegen YY1 können dann in weiteren Schritten im ELISA-Verfahren oder Western blot genutzt werden.

In diesem Zusammenhang dienen auch Sandwich-ELISA zum Antigennachweis, und geeignet ist auch der Nachweis spezifischer Antikörper gegen ein Antigen mittels enzymmarkierten Sekundärantikörpern.

### Etablierung der ELISA-Verfahren (s. Ref. 89)

Nachweis spezifischer Antikörper gegen YY1 mittels enzymmarkierten Sekundärantikörpern

Synthetisch hergestellte YY1-Proteine werden an einen festen Träger (Kunststoffoberflächen, Mikrotiterplatte) gekoppelt. Anschließend wird das zu untersuchende Probandenmaterial (Serum) überschichtet. Spezifisch gegen YY1 gerichtete Antikörper im Serum befindlich, binden. Ungebundene Komponenten werden heruntergewaschen. Mittels eines Sekundärantikörpers, chemisch mit einem Enzym markiert, wird in der Farbreaktion das Substrat umgewandelt. Anhand eines mitgeführten Standars kann dann, nach Erstellung der Eichkurve, direkt mit der Antikörperkonzentration korreliert werden.

Sandwich-ELISA (Antigennachweis) (s. Ref. 89)

Die Antikörper, spezifisch gegen YY1 gerichtet, werden an einen festen Träger (Kunststoffoberflächen, Mikrotiterplatte) gebunden. Im Anschluß daran wird das Probandenmaterial überschichtet. Die YY1-Proteine binden spezifisch an den Antikörper. Ungebundene Komponente werden heruntergewaschen. Mittels monoklonaler Antikörper und eines Anti-Antikörpers (Enzym-markiert) wird die Menge an YY1-Proteinen ermittelt.

### Westernblot (s. Ref. 144)

Es wird davon ausgegangen, daß zwischen den Probanden, bedingt durch alternatives Spleißen oder bereits genetisch bedingt, sich die YY1-Proteine in ihrer Größe (Molekulargewicht) unterscheiden.

Daher kann mit Hilfe einer SDS-Polyacrylamid-Gelelekrophorese (SDS-Page) YY1-Proteine nach ihrer Größe aufgetrennt werden. Durch das SDS erhalten die Proteine negative Ladungen (SDS-Bindung). Nach dem gelelektrophoretischen Lauf wird das Trenngel auf eine immobilisierte Membran (Nitrozellulose) transferiert. Mittels spezifischer Antikörper gegen YY1 gerichtet und Alkalischer Phoshatase werden die Proteine angefärbt.

Gegenstand der Erfindung ist somit ein Verfahren zur Bestimmung der Neigung, an Typ-1-Diabetes, zu erkranken, bei dem man genomische DNA aus isolierten mononukleären Blutzellen amplifiziert, sequenziert und man Änderungen oder Abweichungen der Nukleinsäuresequenz, die für ein Protein mit der in SEQ ID NO:6 dargestellten Aminosäuresequenz kodiert, bestimmt, wobei Abweichungen von Codons (nicht-stille Mutationen) eine erhöhte Neigung anzeigen, an einer der vorgenannten Krankheiten zu erkranken.

Weitere Mutationen, die auf höhere Diabetesneigung hindeuten können, liegen in folgenden Sequenzbereichen und lassen sich unter Verwendung folgender Primer nachweisen: Intron 4: Primer: K815-F/K817-R; K815-F/K875; K821-F/K817-R; K821-F/K870-R; K874-F/K870-R. Zur Amplifikation verwendet man vorzugsweise die Primer K823-F/K825-R; K884-F/K806-R; K801-F/K804-R; K814-F/KK832-R; K828-F/K833-R; K831-F/K817-R; K815-F/K870-R; K815-F/K818-R; K816-F/K819-R; K834-F/K836-R, F15/R12; F15/R14; F15/R13; F57/R16; F57/R20; F57/R21; F59/RR25; F59/RR30; F59/R33; F95/R34; F96/R39; F95/R48; F95/R50; F60/R7; F60/R8; F60/R66; F60/R67; F96/R76; F96/R77; F96/R81 F96/R83; F33/R1; F33/R4; F33/R15; F39/R28; F40/R3; F41/R5; oder andere Kombinationen.

Ferner eingeschlossen ist ein Verfahren zur Bestimmung der Neigung, an Typ-1-Diabetes zu erkranken, bei dem man RNA Langerhansschen Inseln oder Pankreas isoliert, amplifiziert und quantifiziert, wobei eine erhöhte/verminderte Expression eine erhöhte/verminderte Neigung anzeigt, an Typ-1-Diabetes zu erkranken. (vgl. oben zu 'Genexpression').

### 2.Behandlung

### 2.1 Nutzung von DNA oder Antisense

Zur Abregulation von YY1 soll DNA oder Antisense von der vorliegenden Sequenz mittels PCR erzeugt und appliziert werden. Sowohl DNA als auch Antisense-Oligonukleotide werden modifiziert, um ihre Stabilität gegenüber den meisten Exo- und Endonukleasen zu erhöhen (168). Die Applikation der stabilisierten DNA oder Antisense erfolgt subkutan (s.c.) oder intramuskulär (i.m.). Die Dosis richtet sich nach der individuellen Expression von YY1, die in bestimmten Abständen geprüft werden soll, um eine ausgewogene Balance von YY1 im Hinblick auf andere Gene zu erreichen.

### Antisense Oligonukleotide

Da die Antisense Oligonukleotide eine hohe Spezifität besitzen, können sie spezifisch binden, die RNA blockieren und somit die Expression verhindern. Die Antisense Oligonukleotide sollen im Abstand von 2 bis 20 Basen über die Sequenz verteilt werden. Dabei wird vorzugsweise bei Position 73 begonnen.

Wichtige Fragmente, bei denen mehrere Antisense-Oligonukleotide hergestellt wurden (immer um eine Position verschoben), sind: 73 bis 564, 565 bis 717, 718 bis 834, 835 bis 1071; 955 bis 999, 955 bis 1041, 1000 bis 1041, 1042-1125, 1759 bis 1848, 1849 bis 1938, 967 bis 1125, 1071 bis 1938 sowie die Intronsequenz 1126-1758. Die PCR-Amplifikation erfolgt wie beschrieben (12).

### Oligonukleotid-Modifikationen

Zur Verbesserung der Effizienz/Stabilität mit Hilfe von:
1.) Methylphosphonaten
2.) Phosphorothioaten
   - Nuclease-resistent, wasserlöslich, starke Hybrydisierung mit m-RNA
   - steigert RNase H Aktivität, die verantwortlich ist für den Abbau von m-RNA im Doppelstrang
3.) Phosphodithionate
4.) Polyamid-Rückgrat (PNA-DNA-Chimäre) statt Pentose-Phosphat (Uhlmann E., Peyman A., Breipohl G., Angew. Chem. 1998, 110, 2954-2983.)
   - Chimäre besser für die Zellaufnahme

Verbesserung der Transporteigenschaften mit Hilfe von:
1.) Alkylierung (Hydrophobie erhöht)
2.) Koppelung an Zucker

### Ribozyme

Ribozyme sind katalytische RNA ,mit enzymatischen Eigenschaften (Phosphatester-Hydrolyse; Nobelpreis Chech/Altmann). Ribozyme erkennen bestimmte Basensequenzen in der mRNA und zerschneiden das Molekül ah diesen Stellen. Sie haben demnach eine Doppelfunktion, das Erkennen einer bestimmten Struktur, dann hydrolytische Spaltung einer Phosphordiesterbindung an einer bestimmten Stelle. Zwei Klassen von Ribozymen haben besondere Bedeutung. Solche mit einer hammerförmigen ("hammerhead")-Struktur und andere, die eine Haarnadel ("hairpin")-Struktur aufweisen. Die von den Ribozymen entwickelte Endonukleaseaktivität kann praktisch gegen alle RNA-Strukturen wirksam werden.

### Triple-Helix-Bindung

Darüber hinaus soll ein dritter Nukleotidstrang an eine DNA-Doppelhelix über zusätzliche (Hoogsteen-) Basenpaarung gekoppelt werden. Damit entsteht ein Stück eines Dreifachstranges, das nicht mehr transkribiert werden kann. Die Positionen in der DNA sind abhängig von der Sequenzstruktur des Probanden. Nachteil: Erkennnung nur in Purin-reichen Regionen

Gegenstand der Erfindung ist daher auch die Verwendung einer vorgenannten Nukleinsäure oder eines Antisense-Oligonukleotids zur Herstellung einer pharmazeutischen Zusammensetzung. Die pharmazeutische Zusammensetzung dient insbesondere zur Behandlung von Typ-1-Diabetes.

Ferner betrifft die Erfindung eine pharmazeutische Zusammensetzung, die eine vorgenannte Nukleinsäure oder ein Antisense-Oligonukleotid sowie gegebenenfalls zusätzliche, pharmazeutisch verträgliche Hilfs- und/oder Trägerstoffe enthält. Die Zusammensetzungen sind vorzugsweise in einer zur intravenösen (i.v.), subcutanen (s.c.) oder intramuskulären (i.m.) Applikation geeigneten Form formuliert.

### 2.2 Nutzung des Proteins

Das Protein YY1 oder Peptide desselben werden synthetisiert, um appliziert werden zu können. Bevorzugte AS-Fragmente sind: 1 bis 165, 166 bis 215, 216 bis 254, 255 bis 323, 255 bis 302, 255 bis 309 (mit und ohne Mutation), 310 bis 323 (mit und ohne Mutation), 324 bis 351, 352 bis 381, 382 bis 411. Darüber hinaus wird der verkürzte BB.6S- Bereich von 299 bis 351 bevorzugt. In diesem Zusammenhang kann die für ein Protein mit der in SEQ ID NO:6 kodierende Nukleinsäuresequenz, insbesondere die Sequenz gemäß SEQ ID NO:5, oder Fragmente derselben in einen Expressionsvektor eingebracht und unter Bedingungen exprimiert werden, die für das gewählte Vektorsystem geeignet oder vorteilhaft sind. Entsprechende Verfahren sind dem Fachmann wohlbekannt.

Dabei stehen Modifikationen des Proteines oder der Peptide durch:
a) Acetylierungen
b) Deacetylierungen
c) Methylierungen
d) Phosphorylierungen
e) O- und N- Glykosylierungen
im Vordergrund, um Stabilität und Aktivität zu erhöhen bzw. zu verändern.

Die Erfindung betrifft somit auch die Verwendung eines oder mehrerer der oben genannten Proteine und/oder Peptide zur Herstellung einer pharmazeutischen Zusammensetzung. Die pharmazeutische Zusammensetzung dient insbesondere zur Behandlung von Typ-1-Diabetes.

Eingeschlossen sind ferner pharmazeutische Zusammensetzungen, die ein oder mehrere der oben genannten Proteine und/oder Peptide enthalten, wobei die Proteine und Peptide gegebenenfalls wie oben beschrieben modifiziert sind. Die Zusammensetzungen enthalten gegebenenfalls zusätzliche, pharmazeutisch verträgliche Hilfs- und/oder Trägerstoffe und sind vorzugsweise in einer zur intravenösen (i.v.), subcutanen (s.c.) oder intramuskulären (i.m.) Applikation geeigneten Form formuliert.

### Spleißvarianten

Darüber hinaus können alle möglichen Spleißvarianten (s. unten) mit Hilfe der im Rahmen der Erfindung untersuchten Sequenz erfaßt werden, um verschiedenste Proteine erzeugen zu können. Die Spleißvarianten unterschiedlicher Größe sind nachfolgend aufgezeigt. Diese Proteine werden vorzugsweise wiederum modifiziert (s. oben), um einen spezifischen Einsatz zu ermöglichen. Sie können entweder einzeln auch in Kombination eingesetzt werden.
Potentielle Spleißvarianten (http://www.itba.mi.cnr.it)

### DONOR SITES:

| POSITION | EXON | INTRON | SCORE |
|---|---|---|---|
| 157 | GAG | GTGGAG | 78. |
| 379 | GAG | GTGATT | 85. |
| 406 | GAG | GTAGTG | 81. |
| 409 | GTA | GTGGGT | 81. |
| 475 | CCG | GTACCC | 75. |
| 668 | CGG | GTAATA | 80. |
| 694 | CAG | GTGCAG | 78. |
| 1129 | CAG | GTAGAG | 79. |
| 1172 | CTG | GTCAGG | 83. |
| 1214 | GGG | GTATTT | 73. |
| 1273 | CAG | GTGTTA | 77. |
| 1363 | GTA | GTGAGT | 80. |
| 1370 | GTA | GTGTGT | 72. |
| 1423 | CAG | GTGACA | 84. |
| 1453 | CTC | GTGAGT | 79. |
| 1605 | CCA | GTGTGT | 78. |
| 1671 | ATA | GTAGGT | 80. |
| 1675 | TAG | GTGGTT | 77. |
| 1693 | GCA | GTGAGC | 79. |
| 2172 | AAG | GTGTTT | 78. |

### ACCEPTOR SITES:

| POSITION | INTRON | EXON | SCORE |
|---|---|---|---|
| 31 | CTCCCGCAG | CCCA | 87. |
| 36 | GCAGCCCAG | GAGC | 85. |
| 335 | GCGCTGCAG | CCGC | 78. |
| 747 | GGTCTTCAG | ATGA | 80. |
| 882 | ACCTCTCAG | ACCC | 80. |
| 1036 | CGGTCCCAG | AGTC | 78. |
| 1053 | TCTGTGCAG | AATG | 77. |
| 1274 | GGACTGCAG | GTGT | 80. |
| 1333 | TTCTAGCAG | GTTT | 79. |
| 1365 | GTTTTGTAG | TGAG | 80. |
| 1418 | TGGCTACAG | CTCC | 77. |
| 1424 | CAGCTCCAG | GTGA | 81. |
| 1447 | TGCTTATAG | AAGA | 80. |
| 1510 | ACTTCCTAG | AGTG | 81. |
| 1572 | TTTCTCAAG | AACT | 84. |
| 1713 | GATCCCCAG | GTTC | 80. |
| 1734 | TTTGCCAAG | AGGG | 78. |
| 1763 | CCTTGACAG | TGCA | 85. |
| 1989 | CCTCTTCAG | GAGT | 79. |
| 2037 | TATTTCTAG | GAAG | 83. |

Die Spleißvarianten werden durch entsprechende Restriktionsenzyme erhalten. Die Verwendung der Spleißvarianten hängt von der individuellen Situation der Probanden ab.

### Antikörper

Darüber hinaus werden monoklonale Antikörper für Bereiche des YY1-Proteins erzeugt und zur Abregulation verwendet. Auch hierbei sind die Antikörper der individuelle Situation anzupassen.

### 2.3 Erhöhung der YY1-Expression

Die gesamte DNA-Sequenz oder Teilsequenzen von YY1 (Positionen 73 bis 564, 565 bis 717, 718 bis 834, 835 bis 1071; 955 bis 999, 955 bis 1041, 1000 bis 1041, 1042-1125, 1759 bis 1848, 1849 bis 1938, 967 bis 1125, 1071 bis 1938 sowie die Intronsequenz 1126-1758), die sich nach der individuellen Situation der Probanden richtet, werden in Plasmiden unter Kontrolle eukaryotischer, gewebsspezifischer Promotoren kloniert und zur Langzeitexpression von YY1 an sich oder seiner Teilsequenzen appliziert, um eine gewebsspezifische Erhöhung der Expression von YY1 oder seiner Teilsequenzen zu erreichen. Die Vektorsysteme werden entsprechend dem Stand der Technik eingesetzt (169).

### 2.4. Regulation anderer Gene durch YY1-Antisense

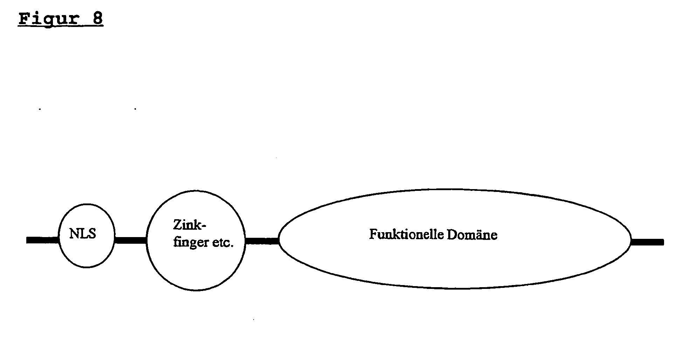

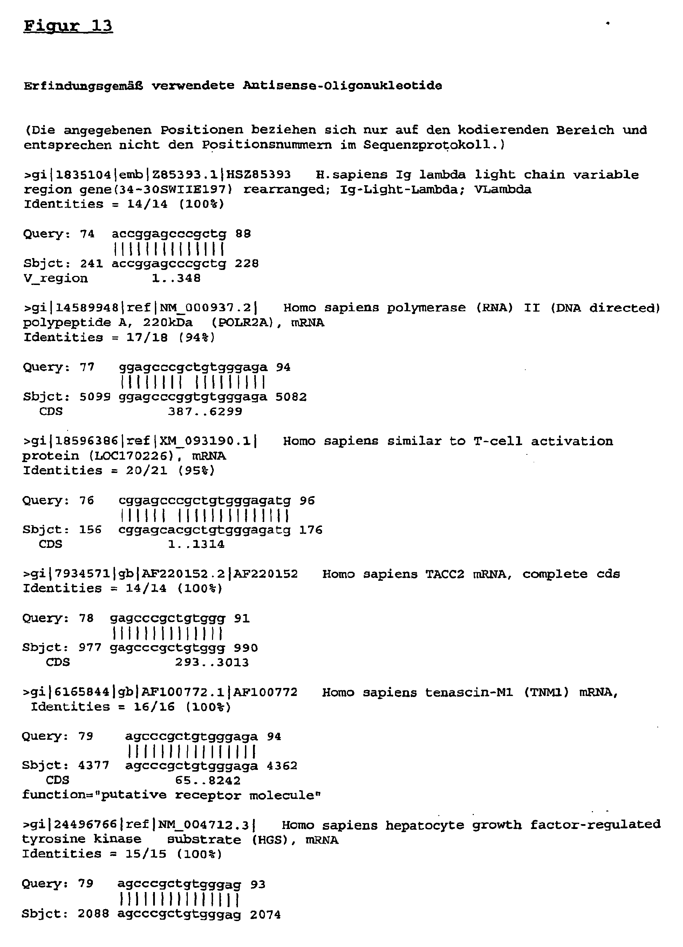

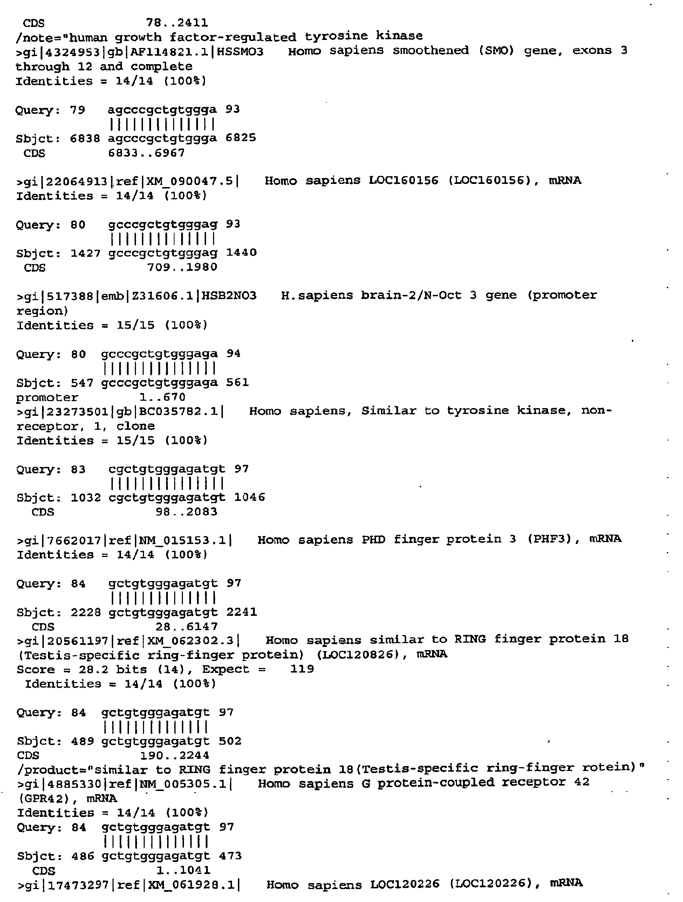

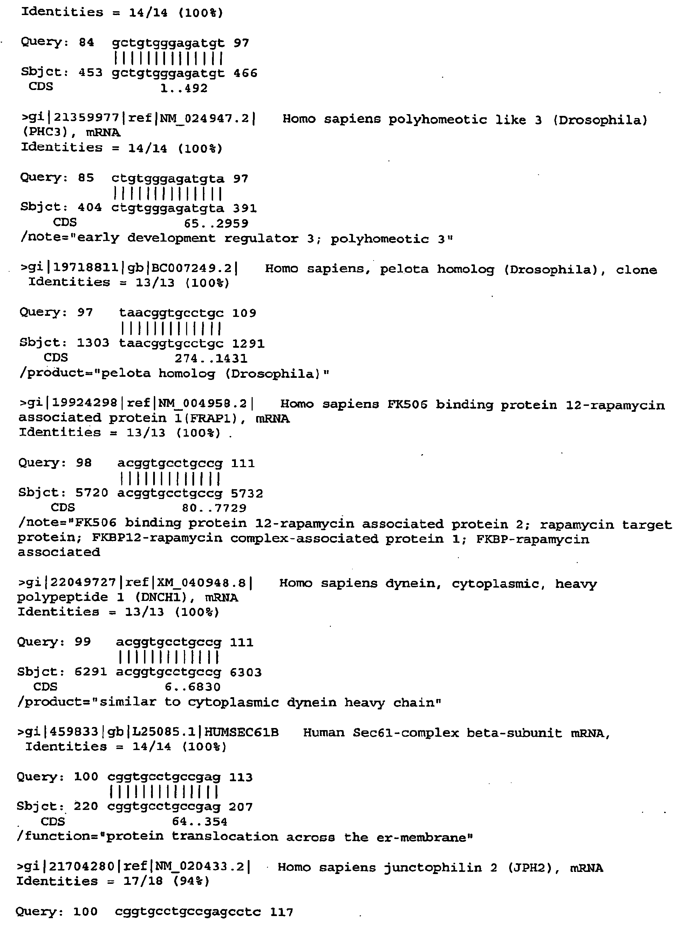

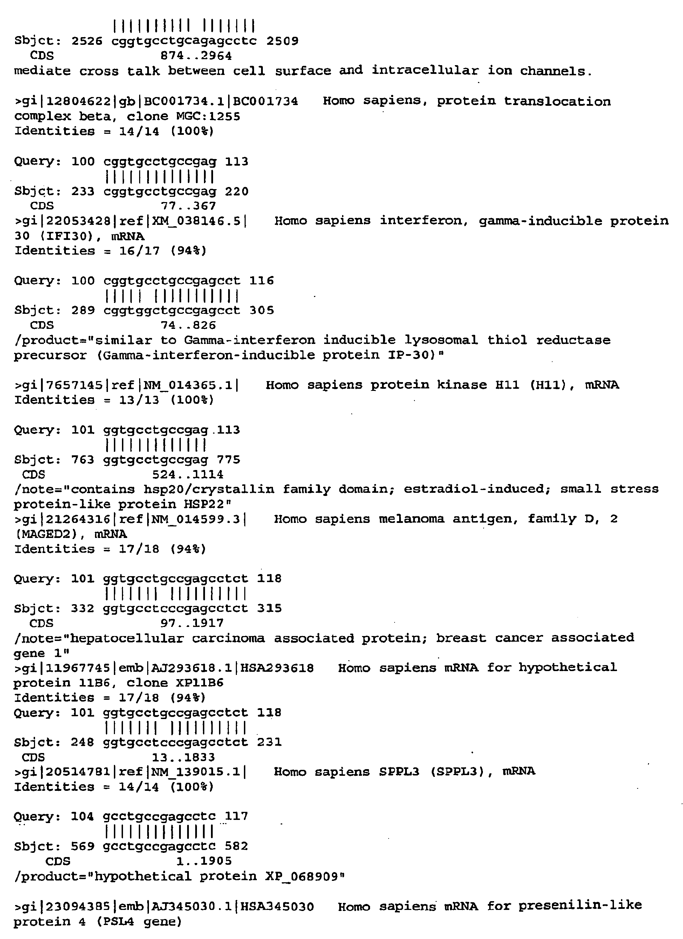

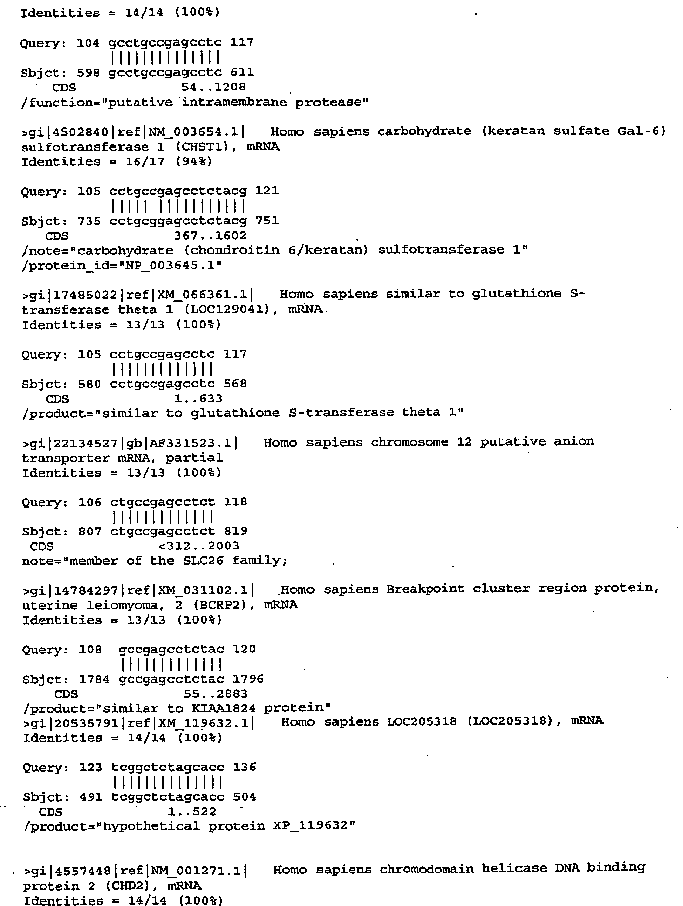

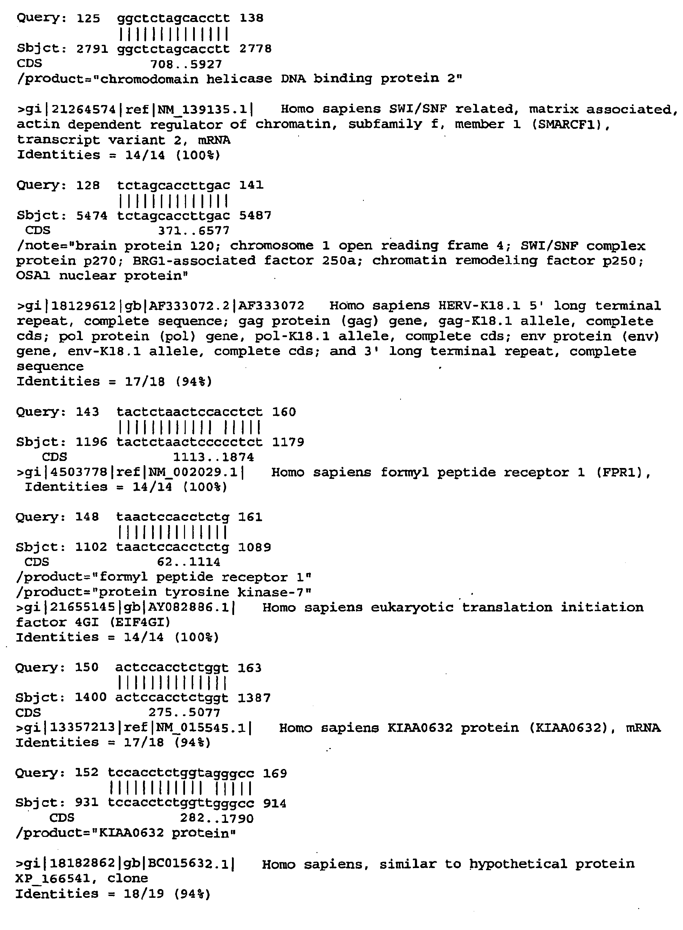

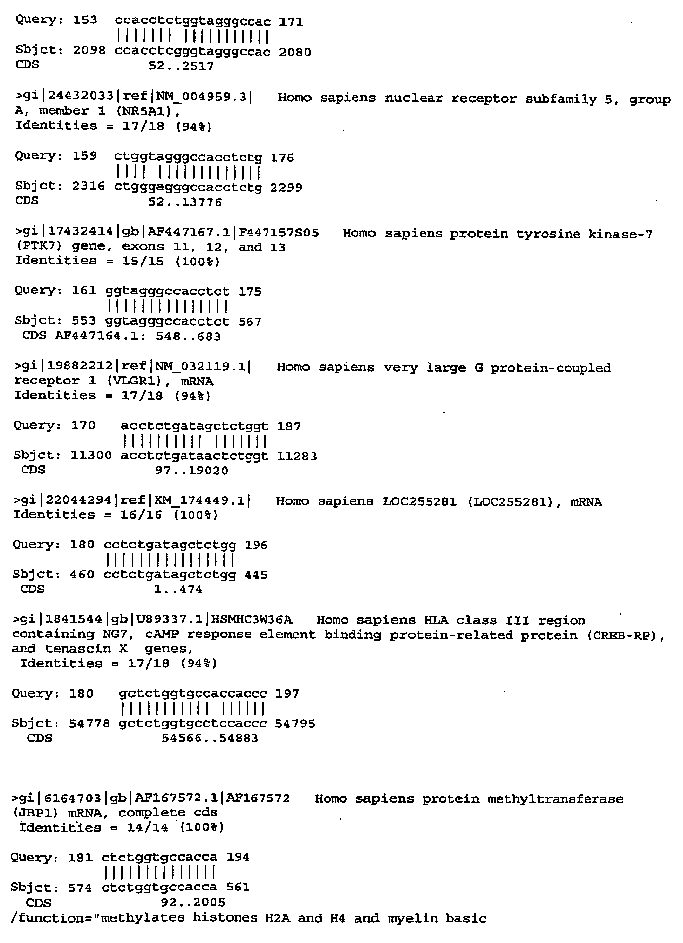

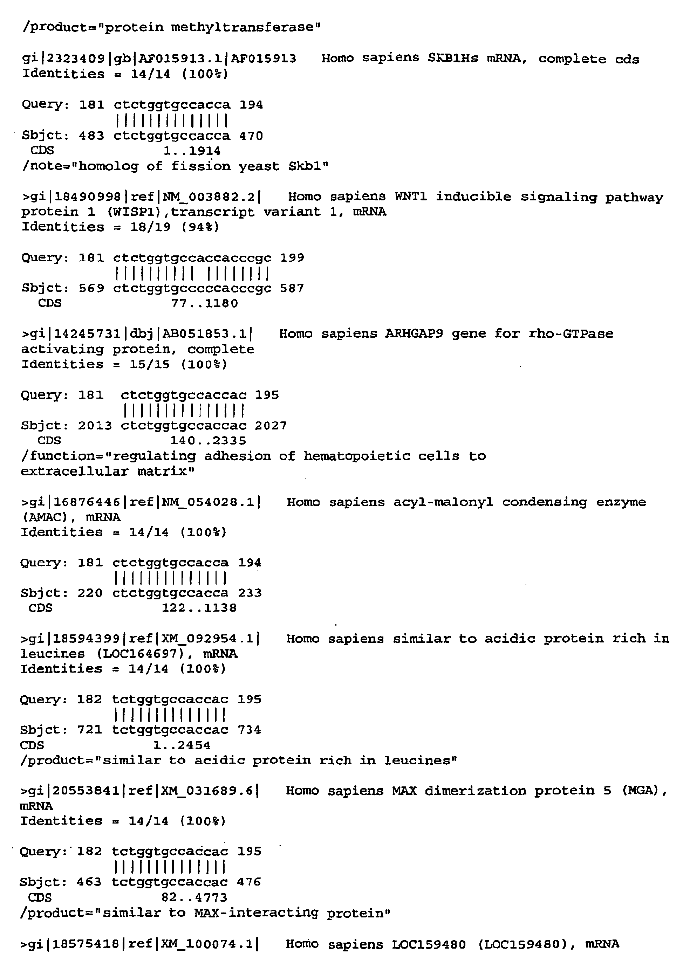

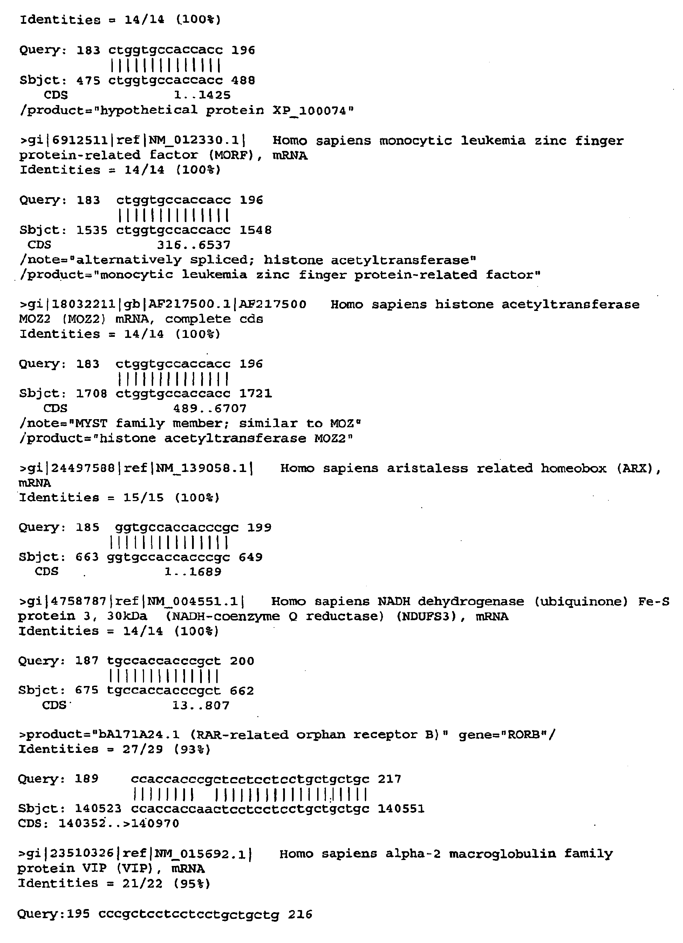

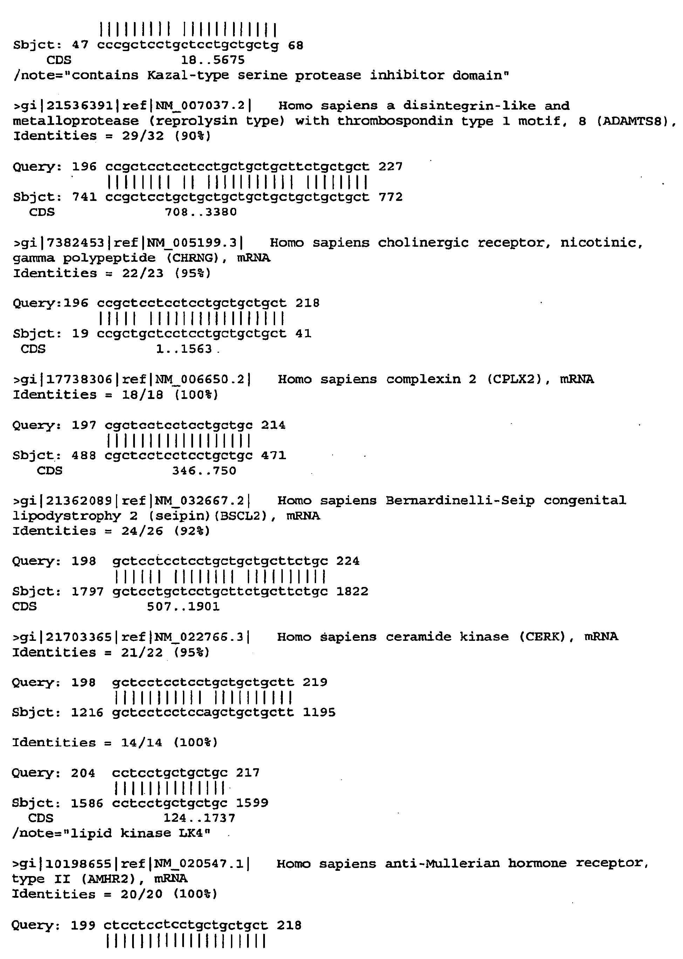

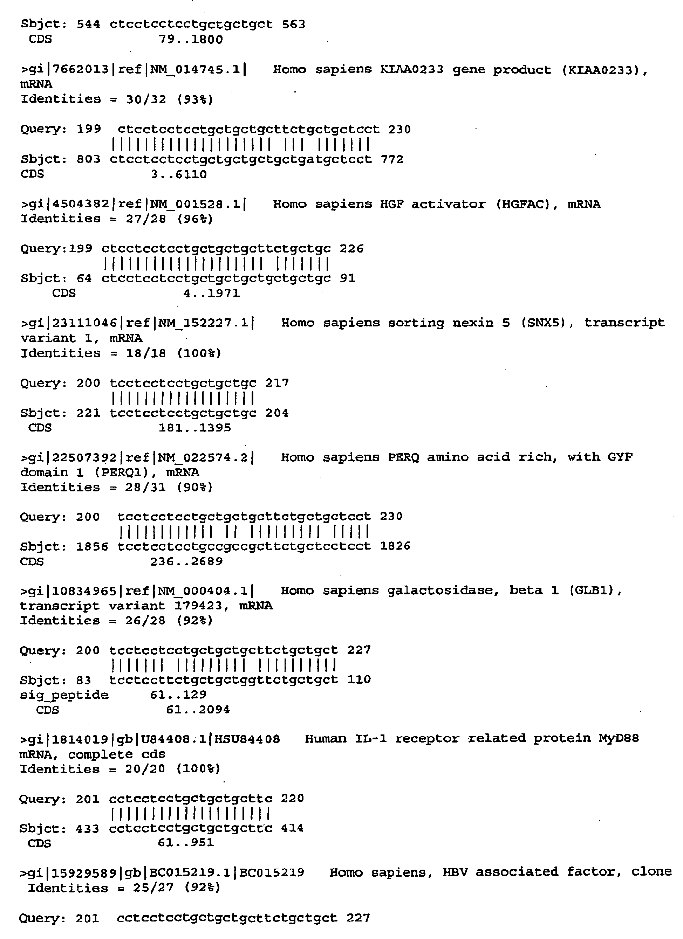

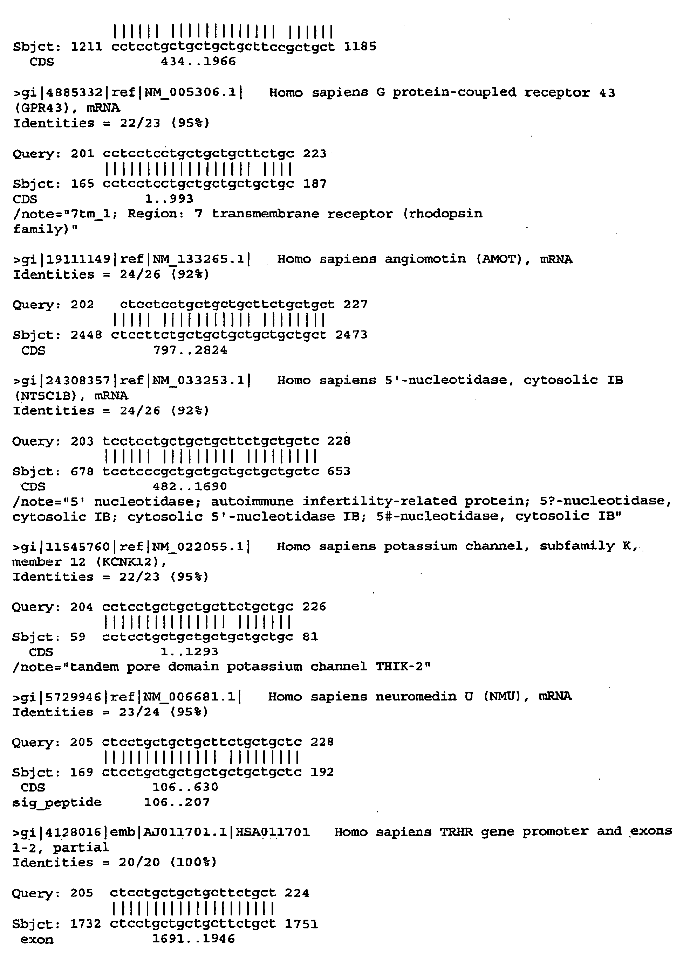

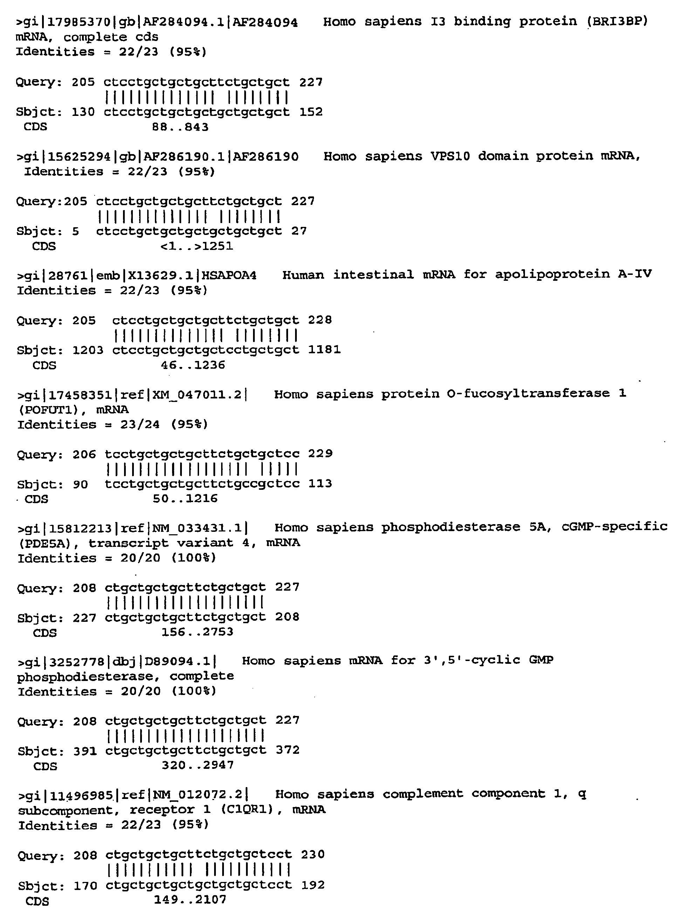

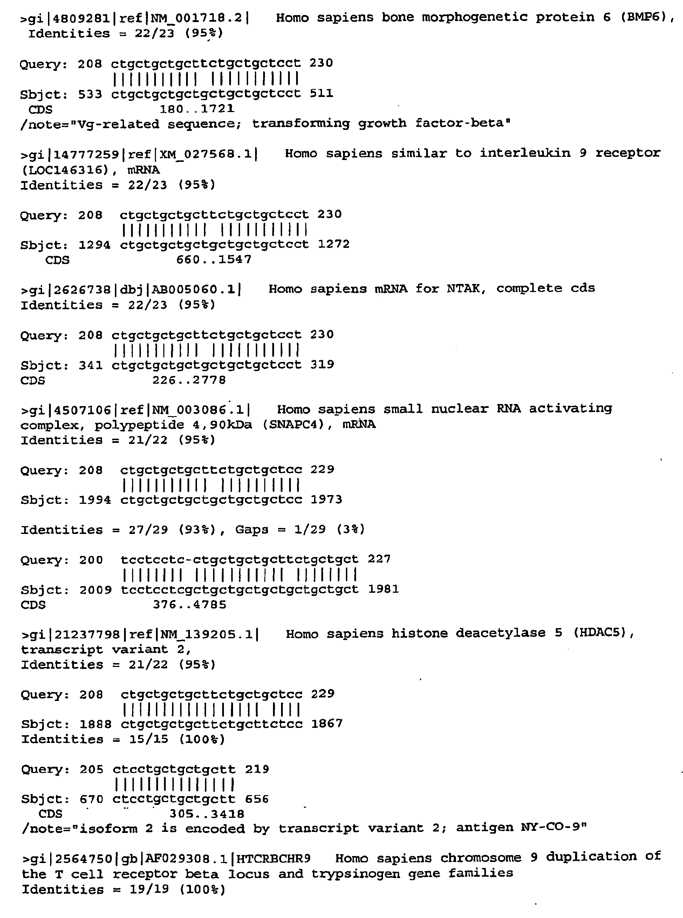

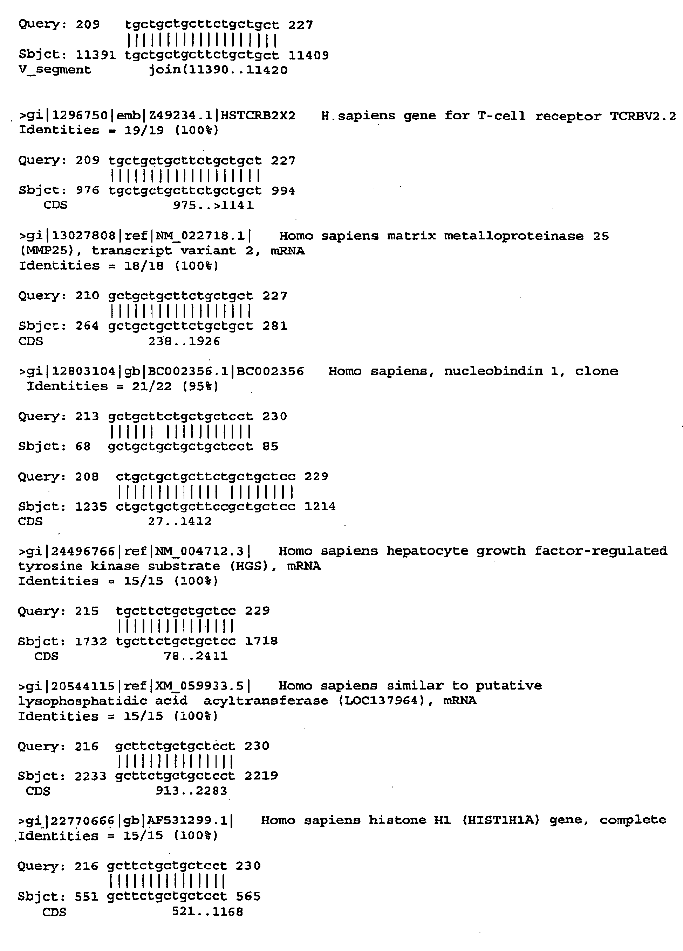

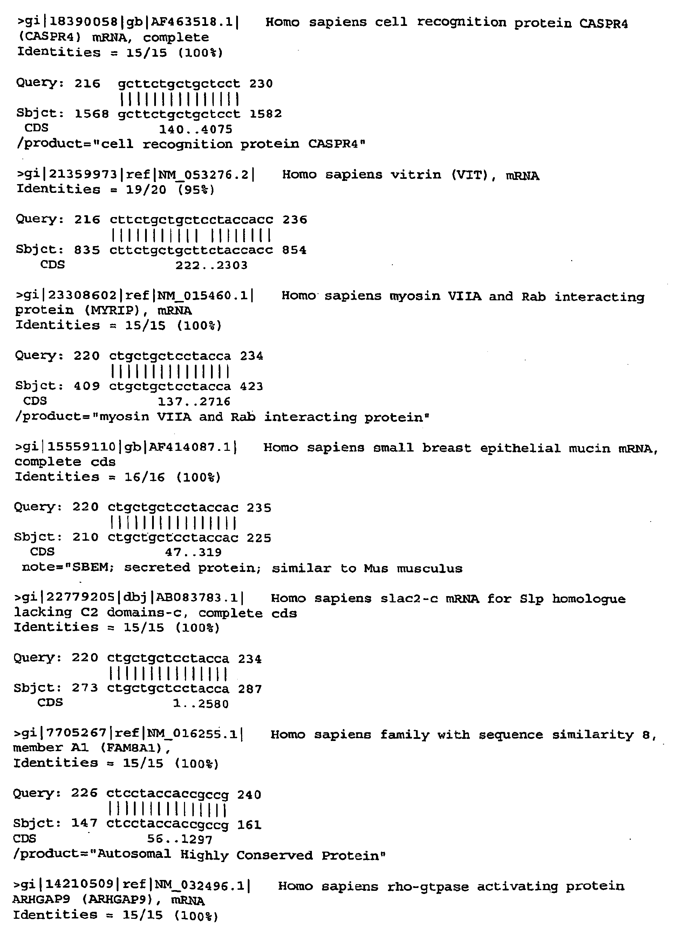

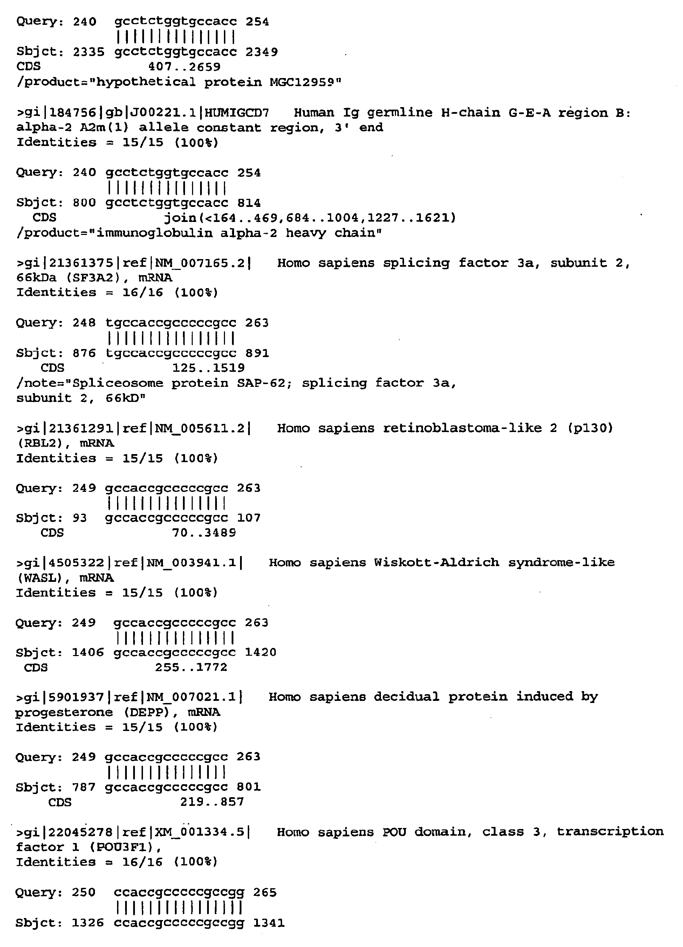

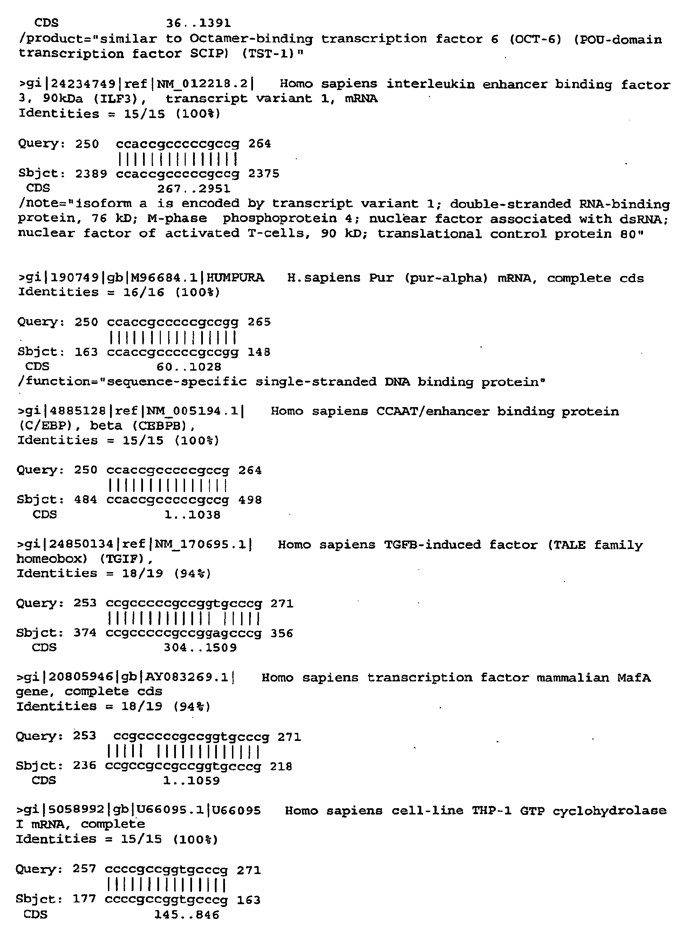

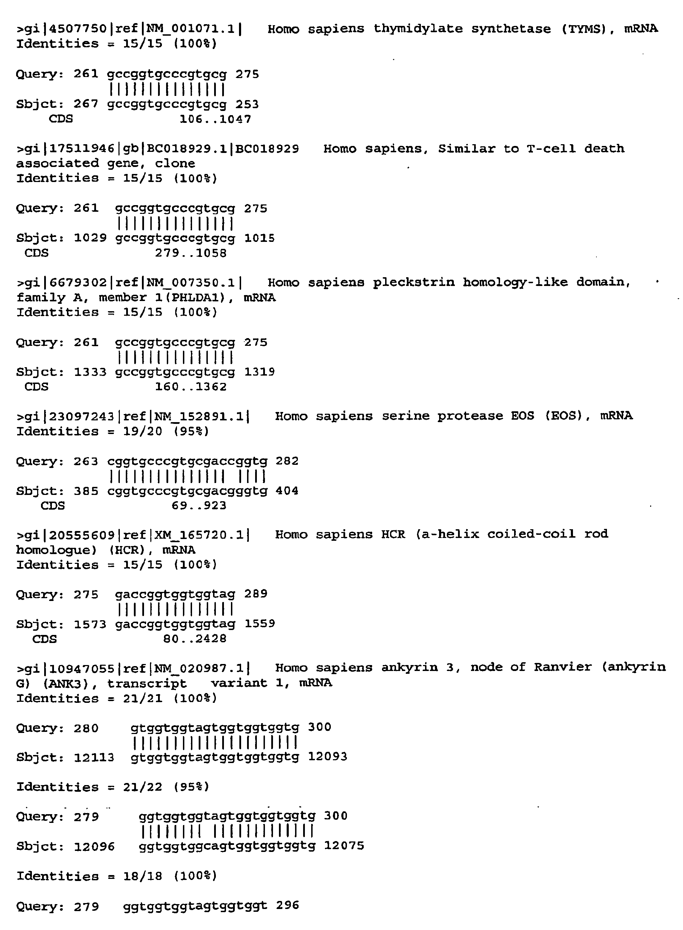

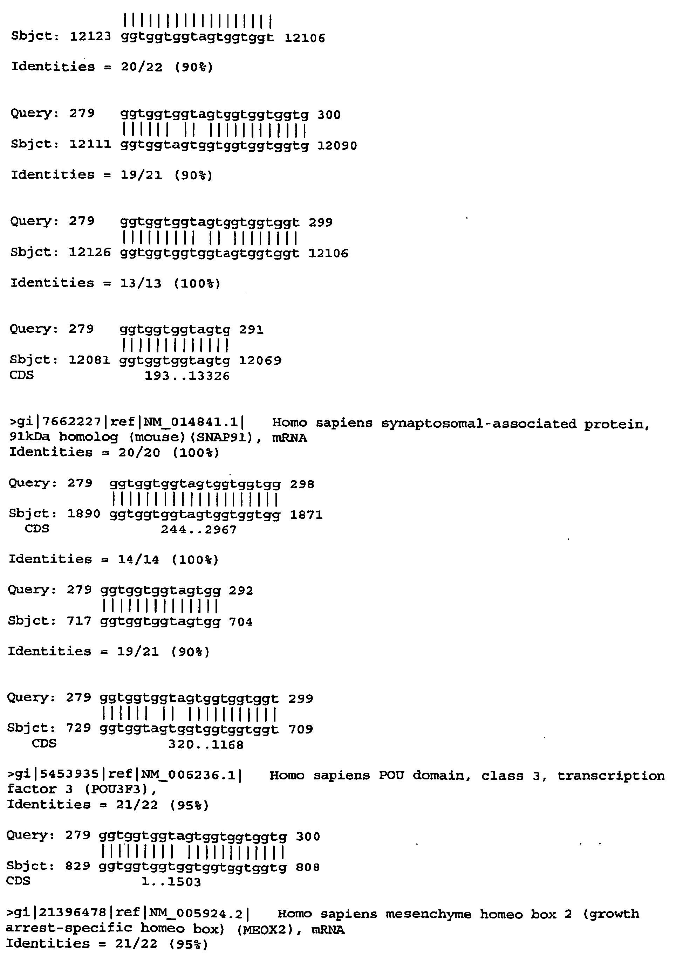

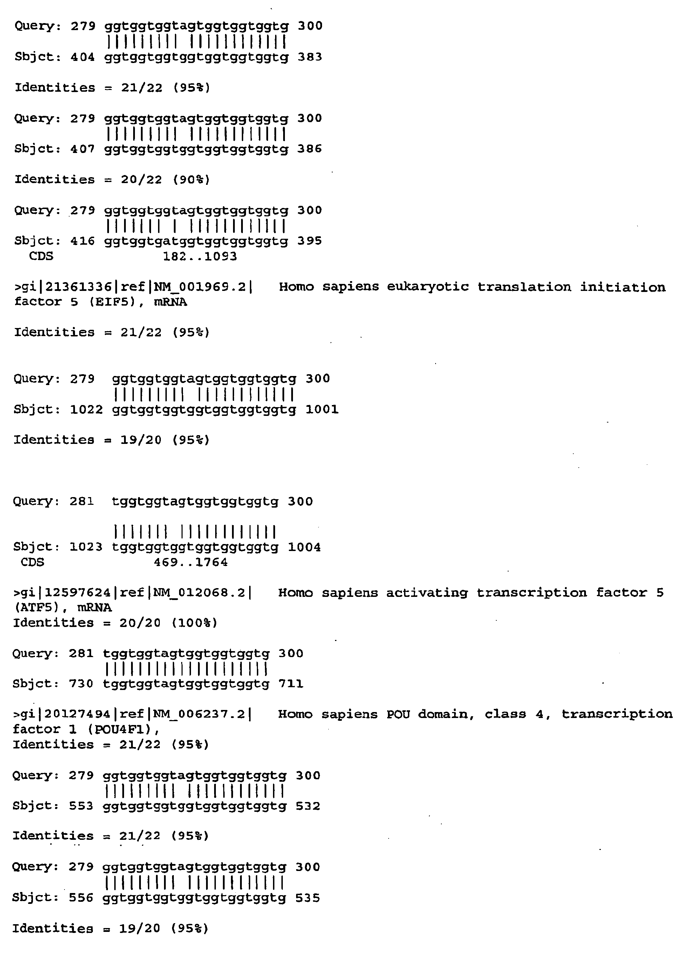

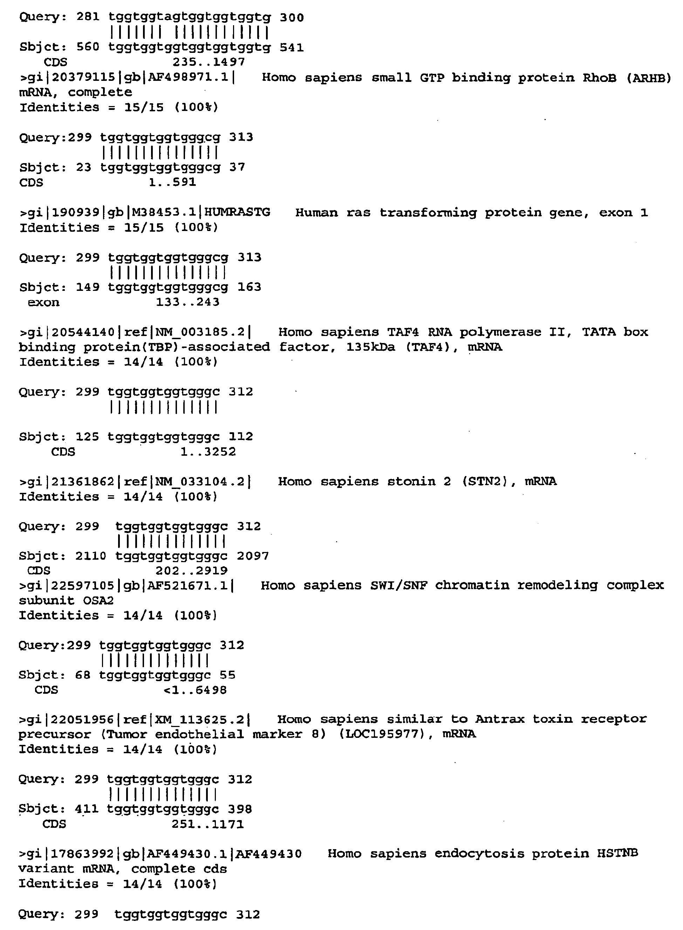

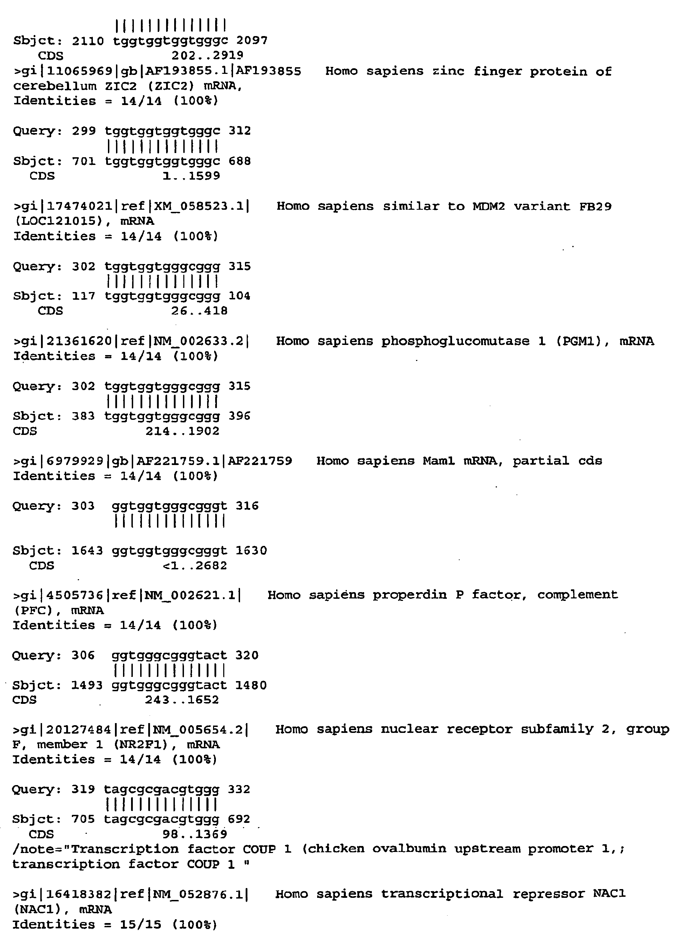

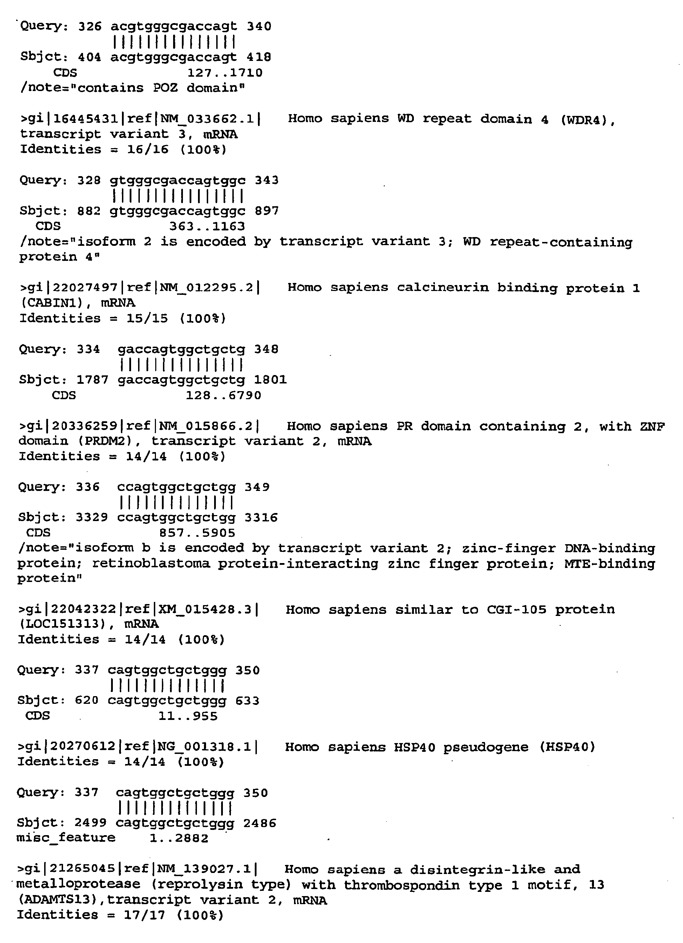

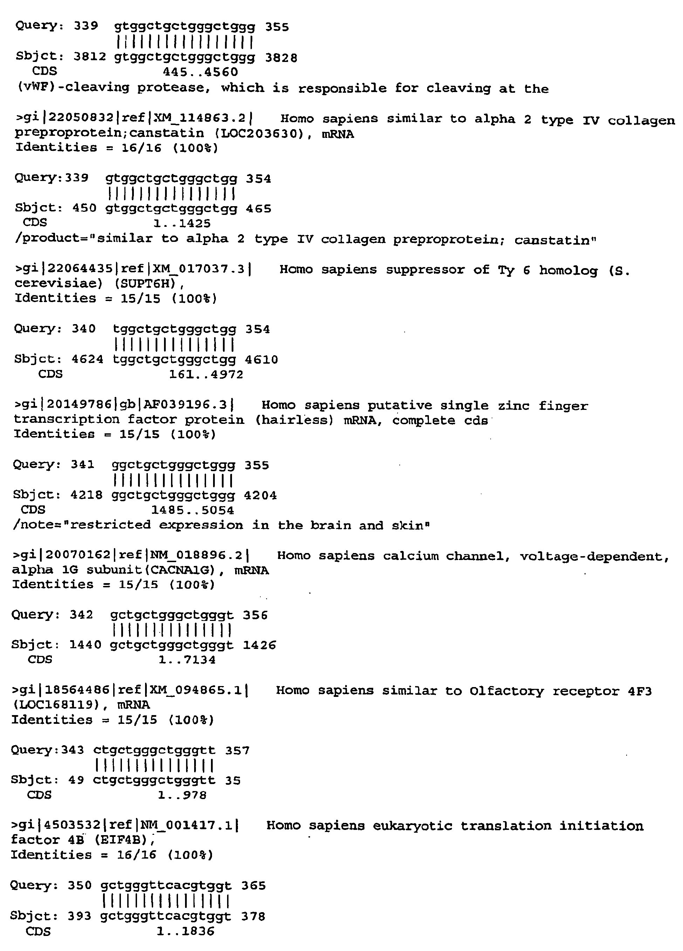

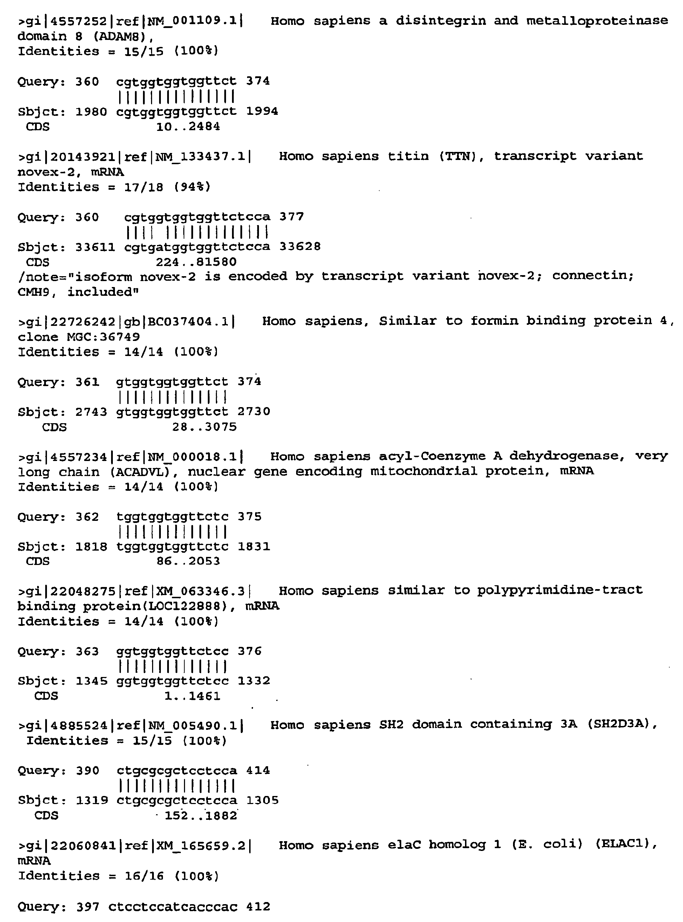

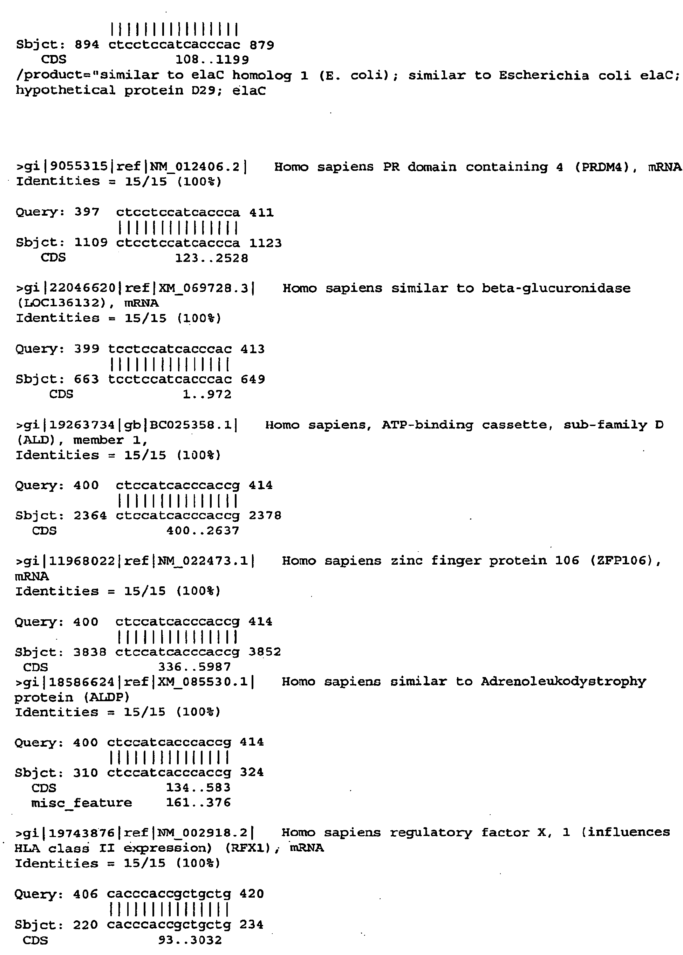

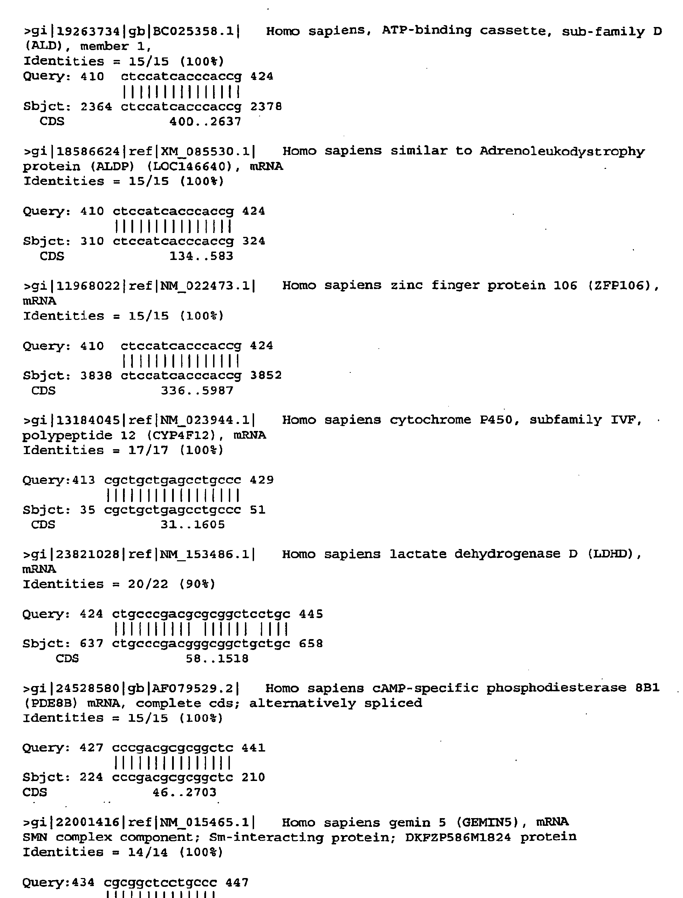

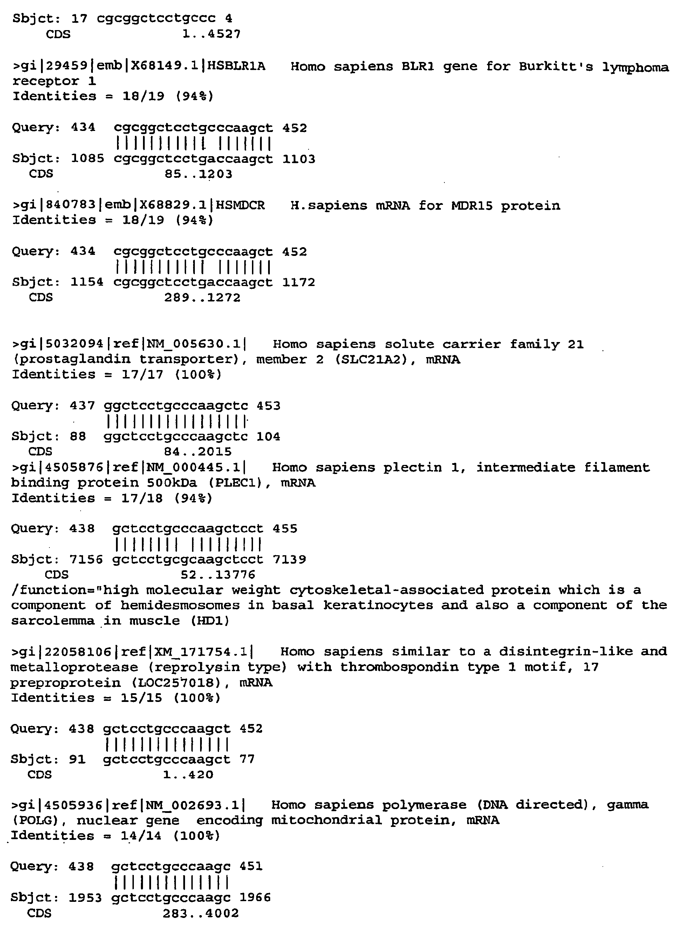

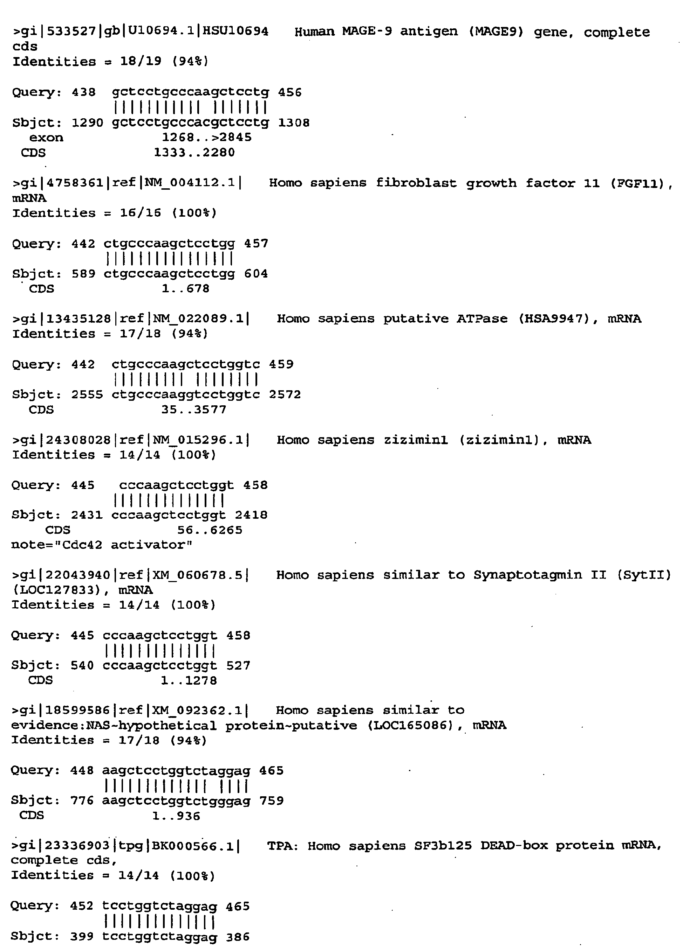

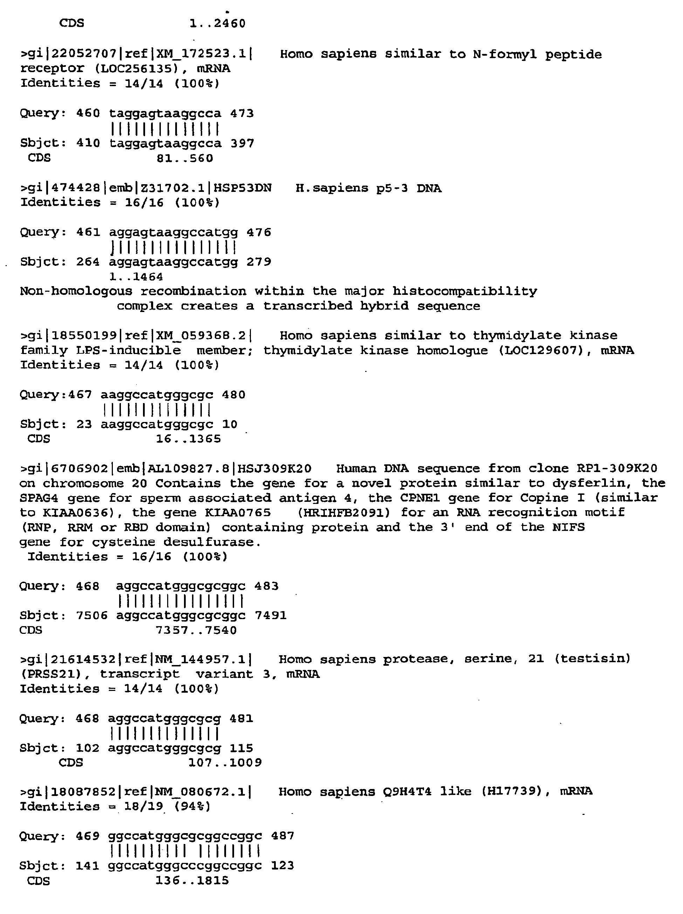

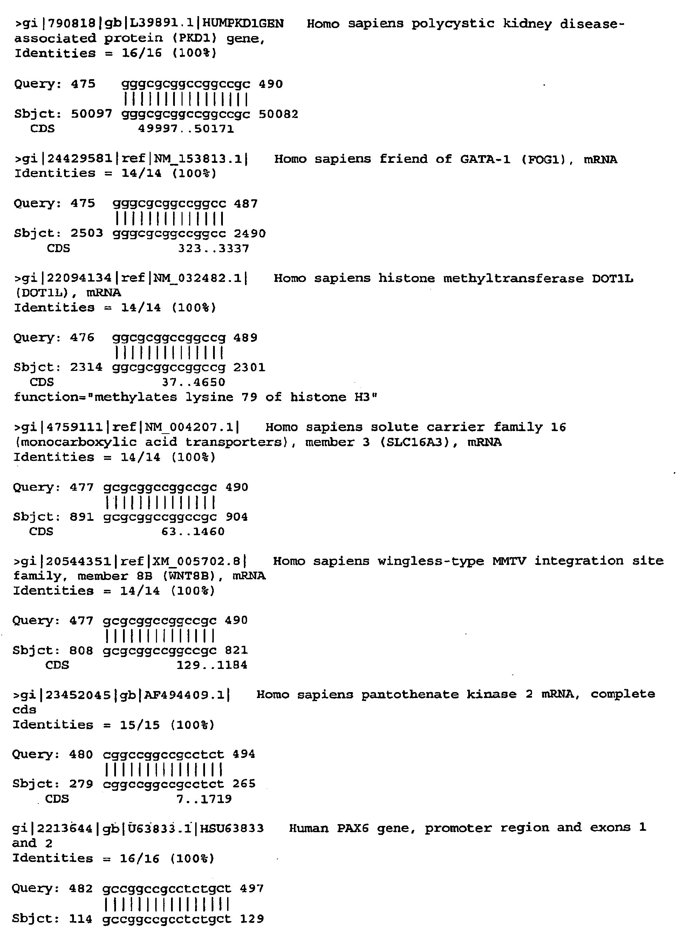

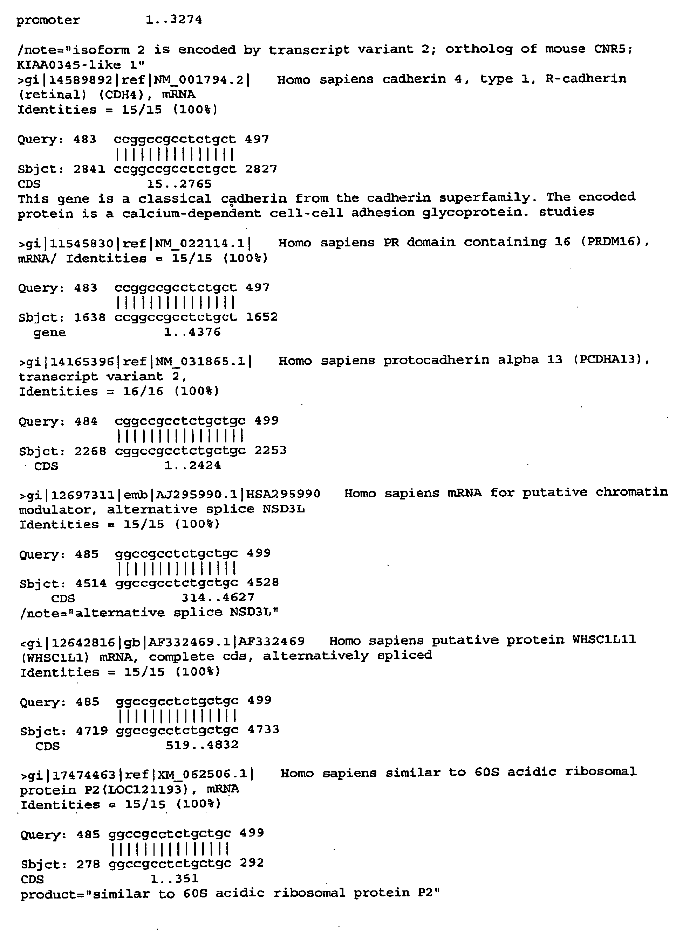

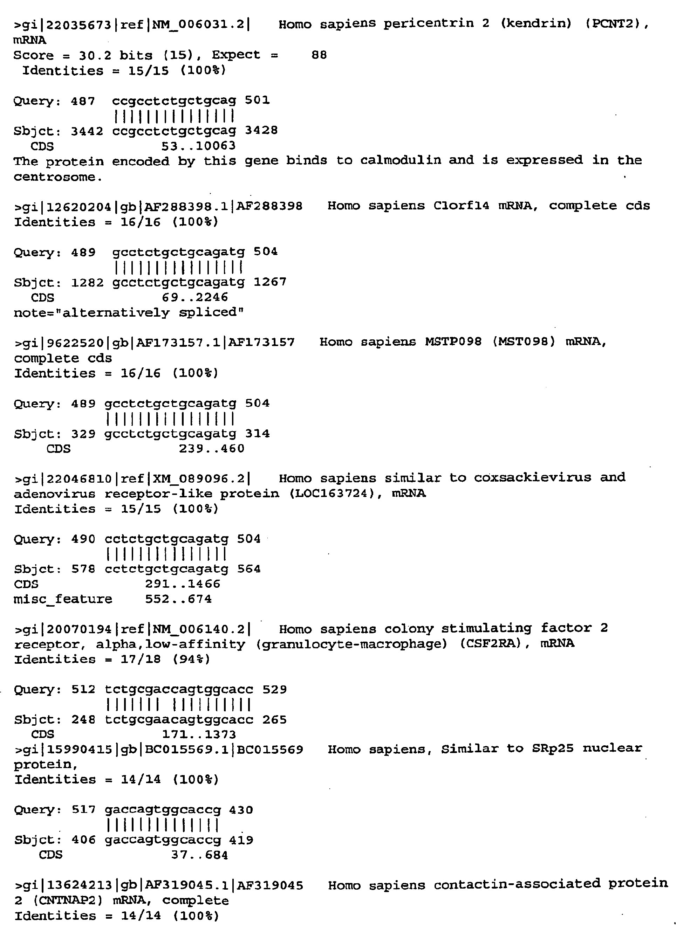

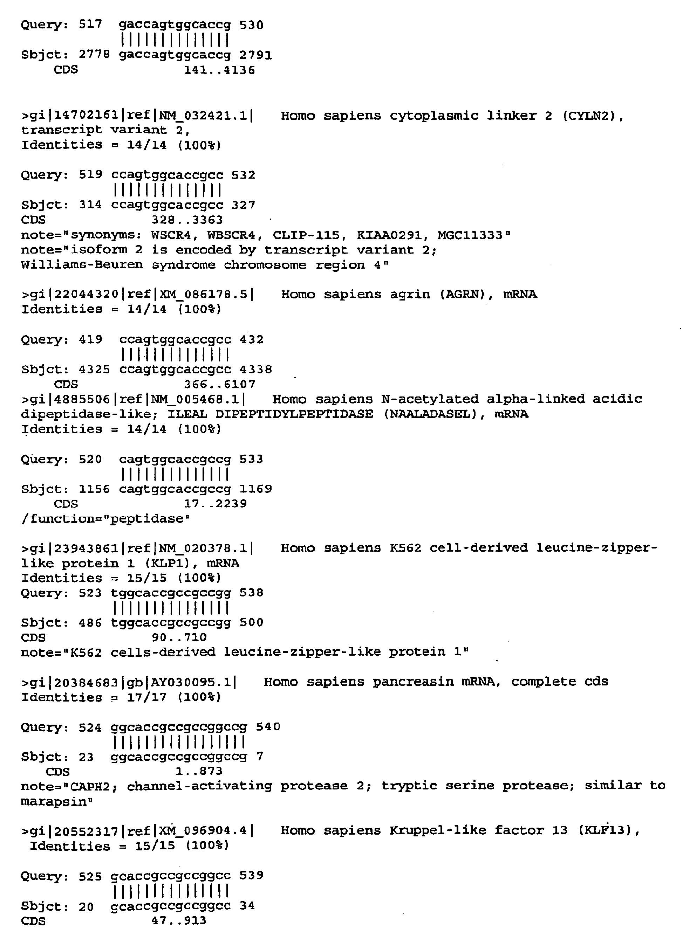

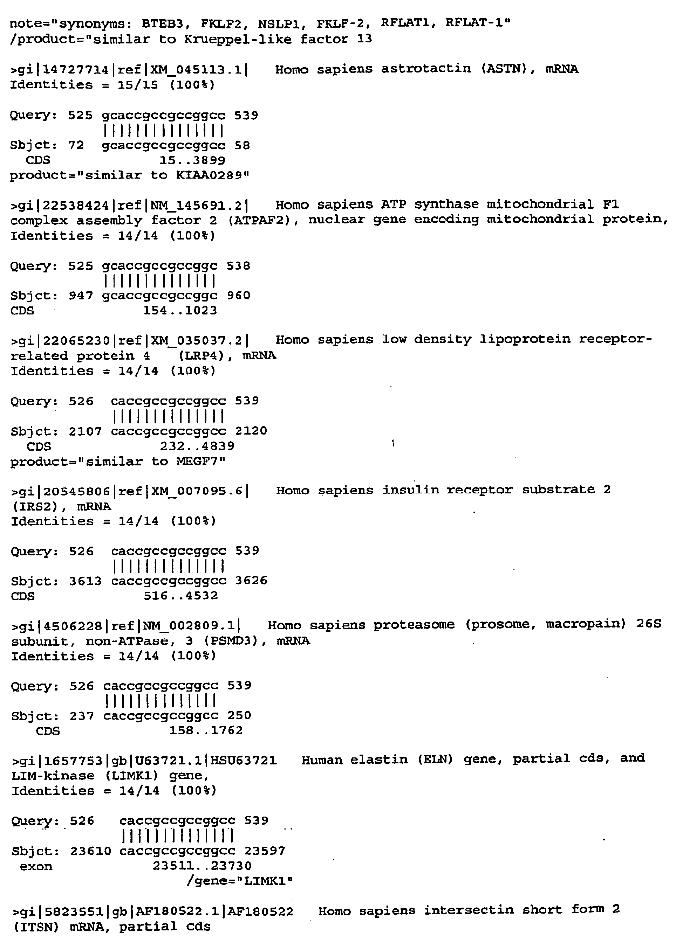

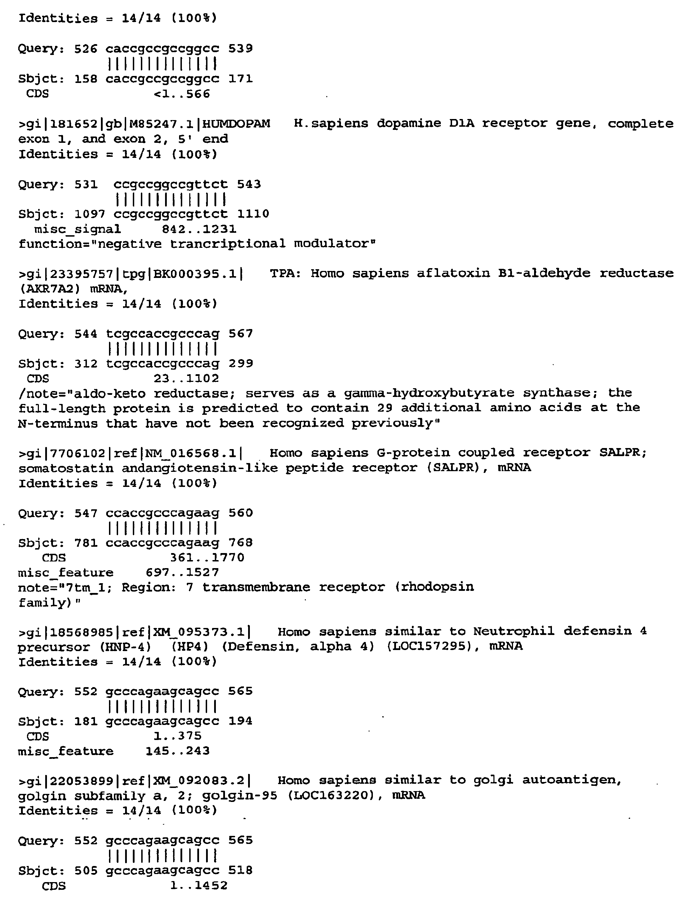

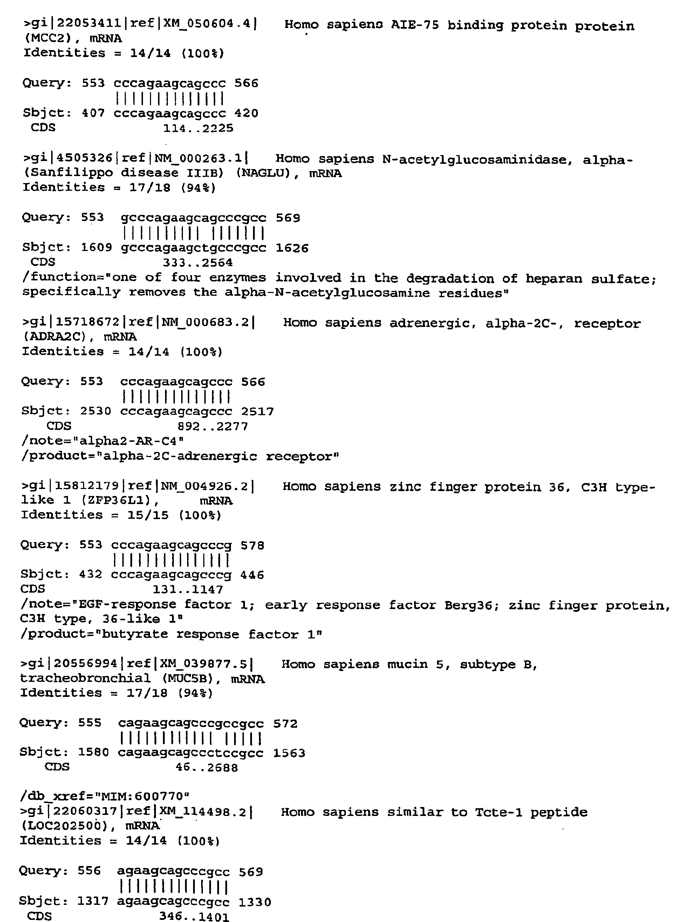

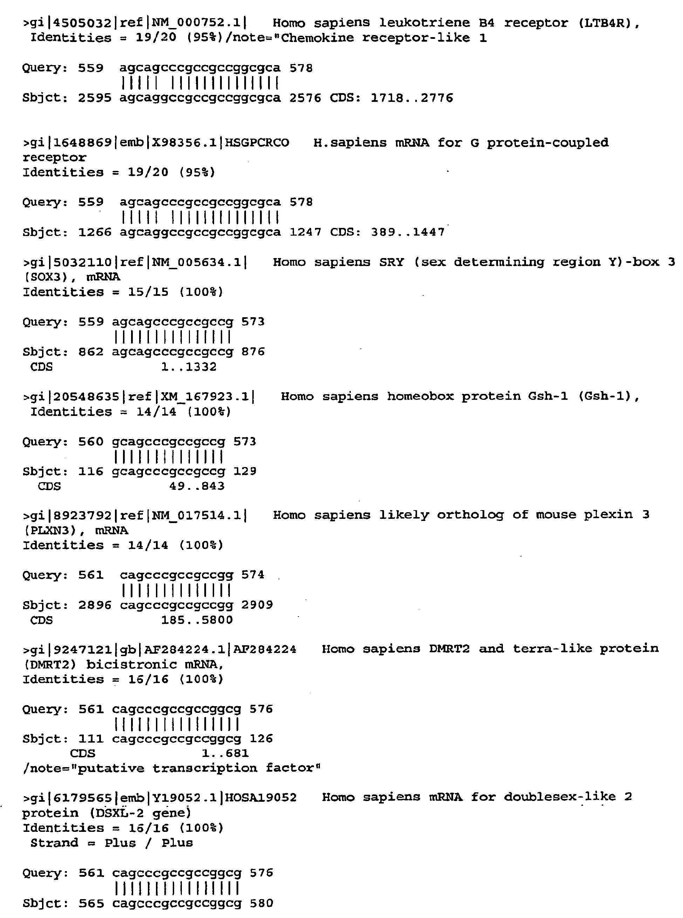

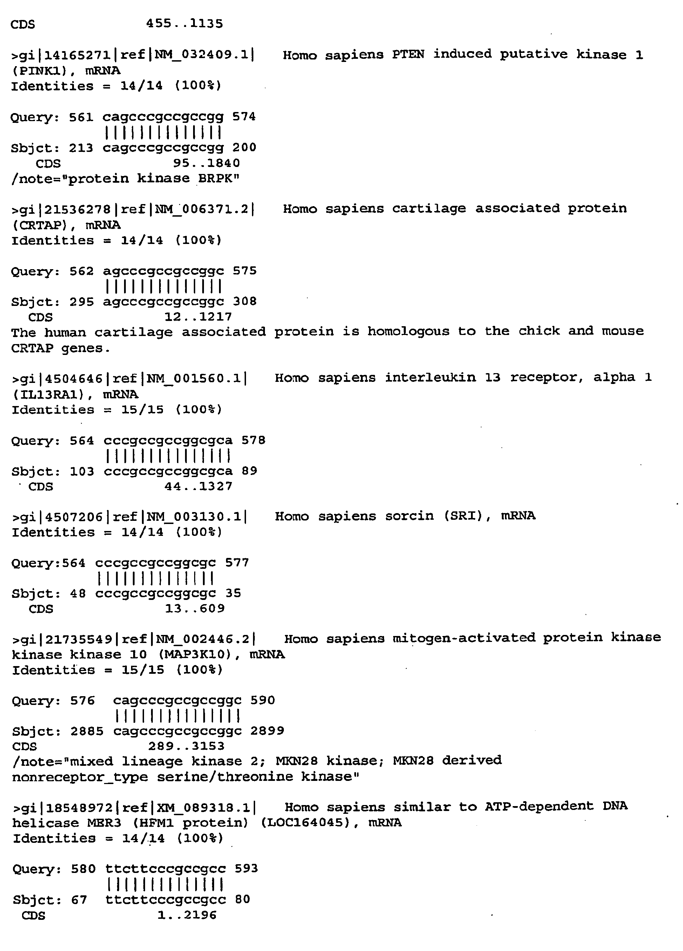

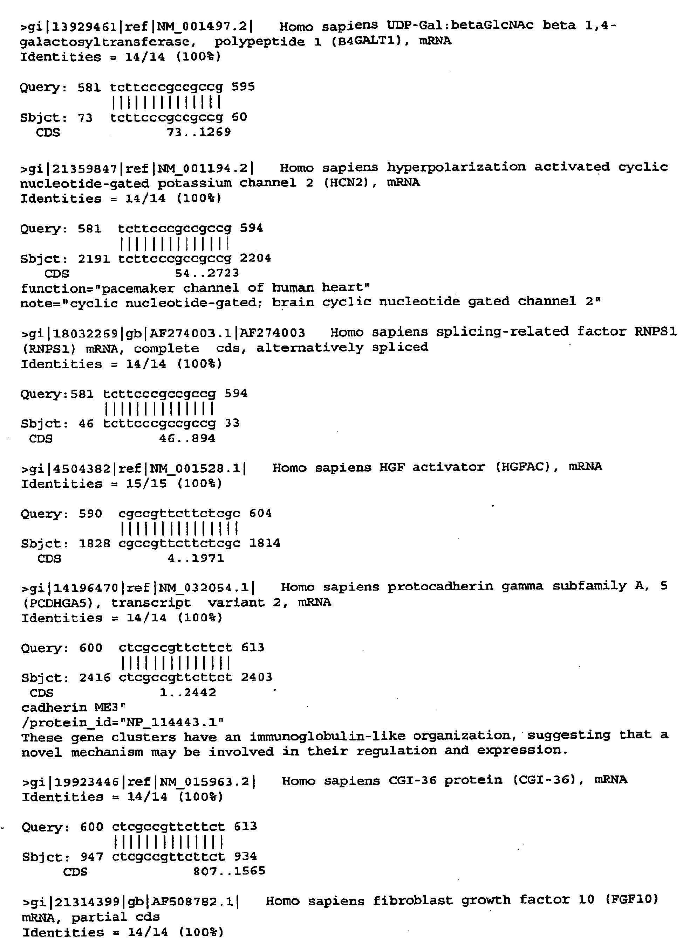

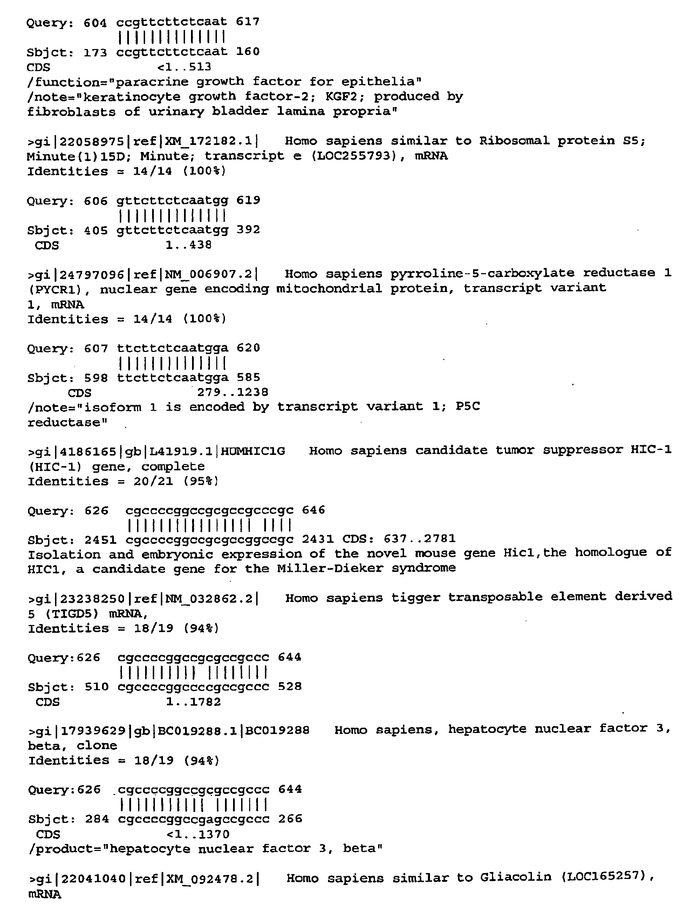

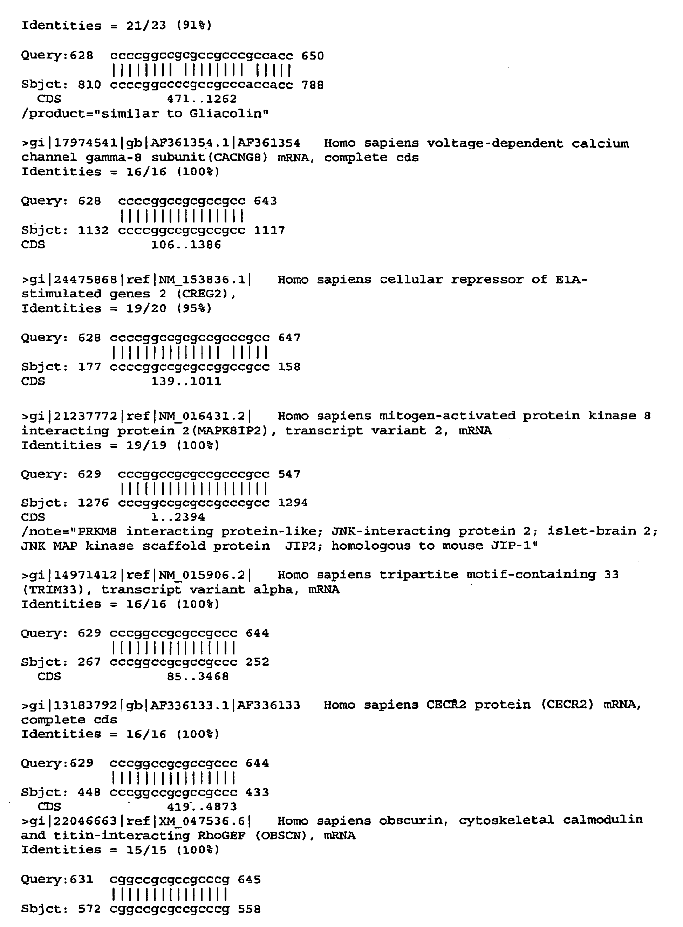

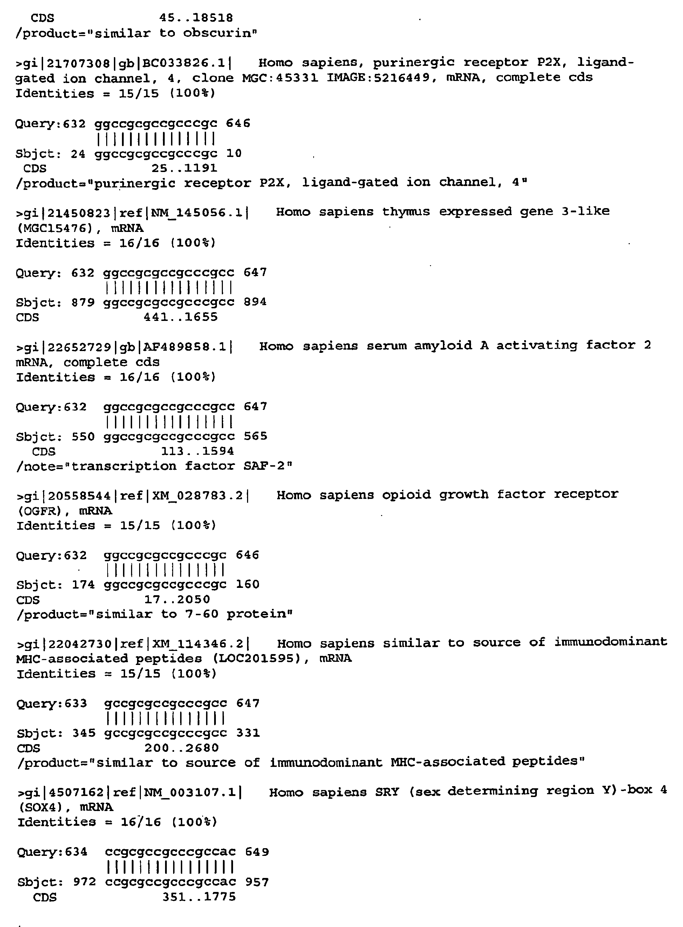

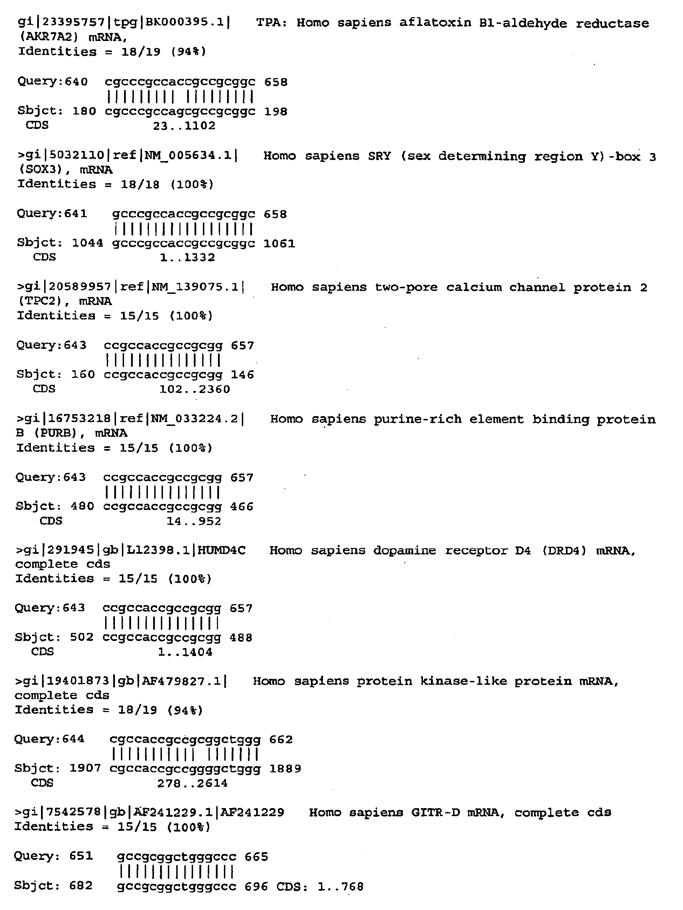

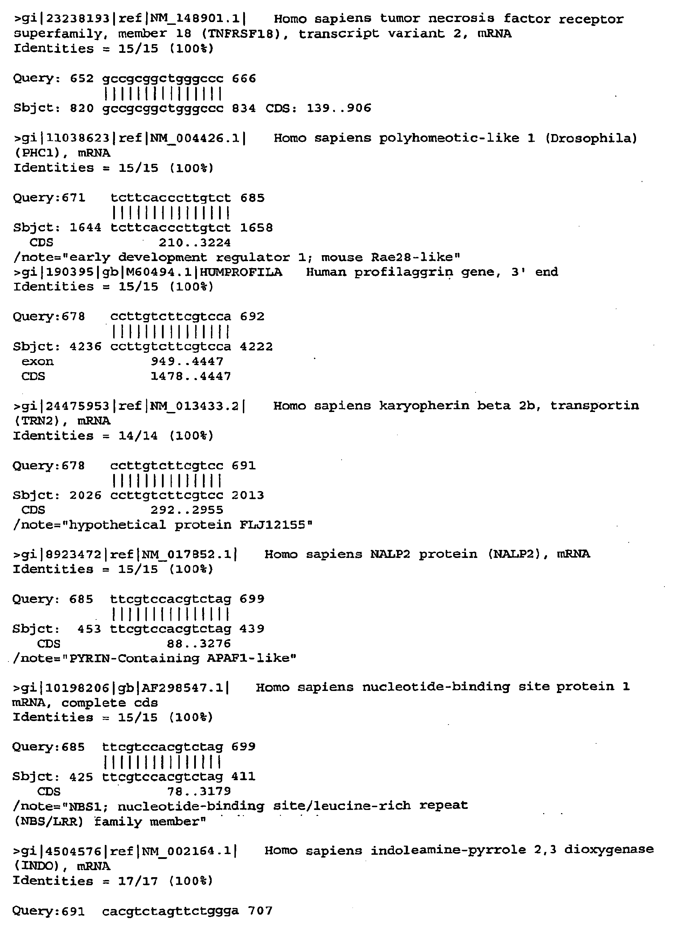

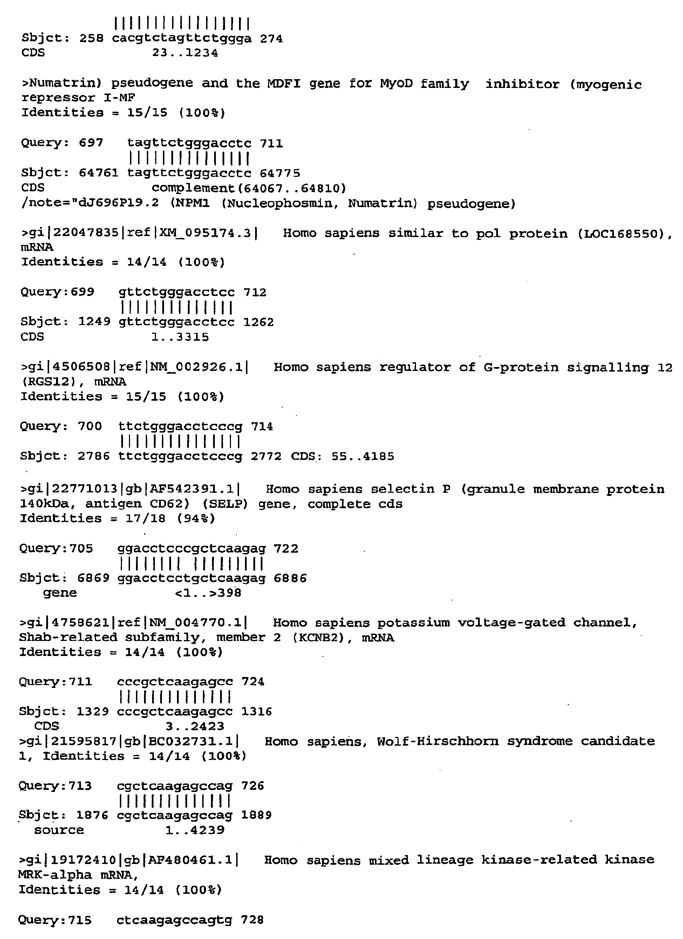

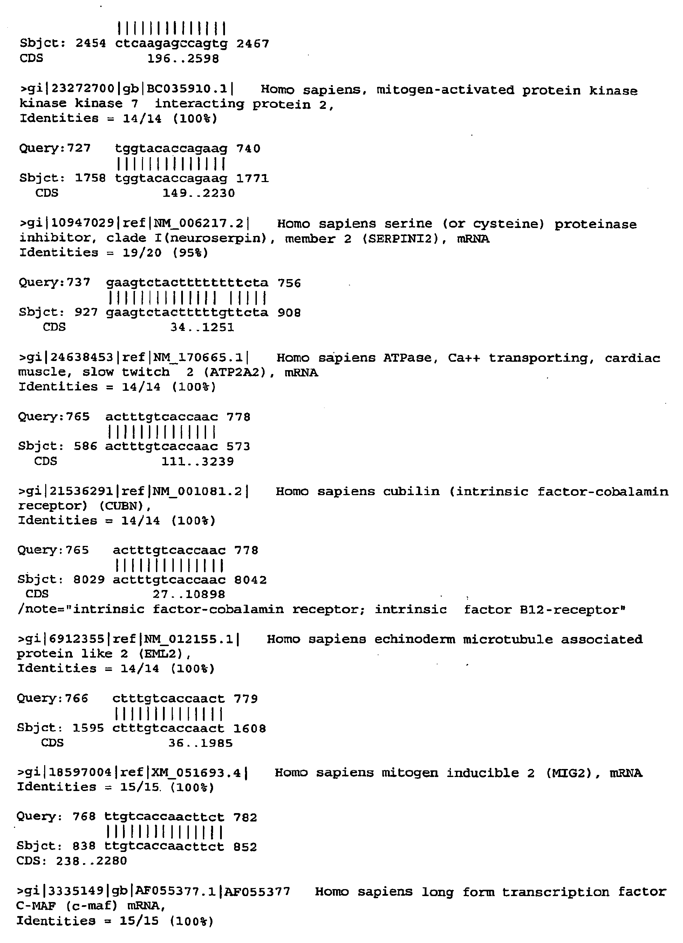

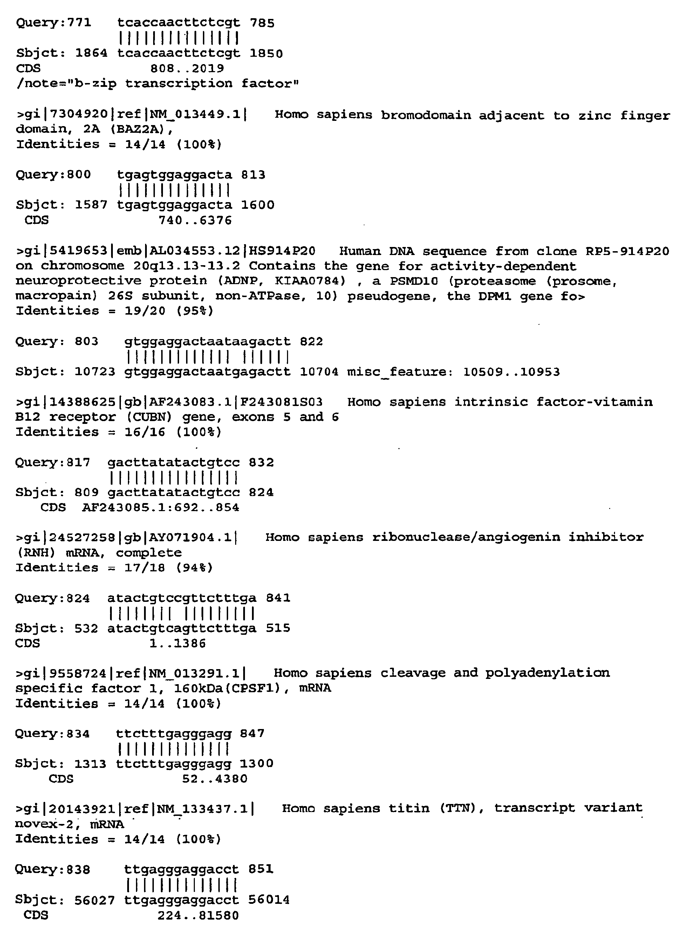

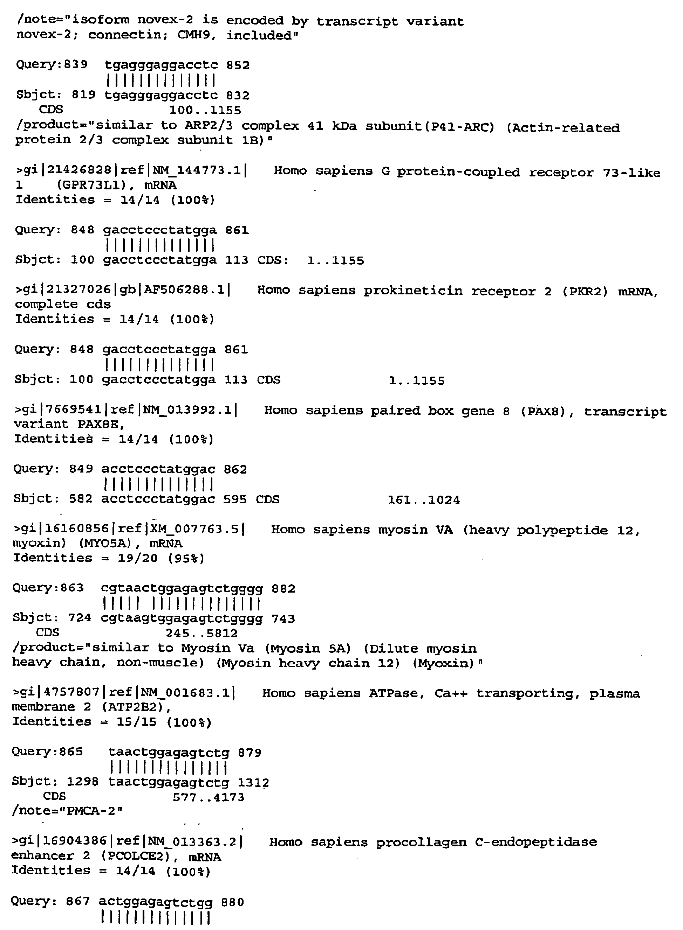

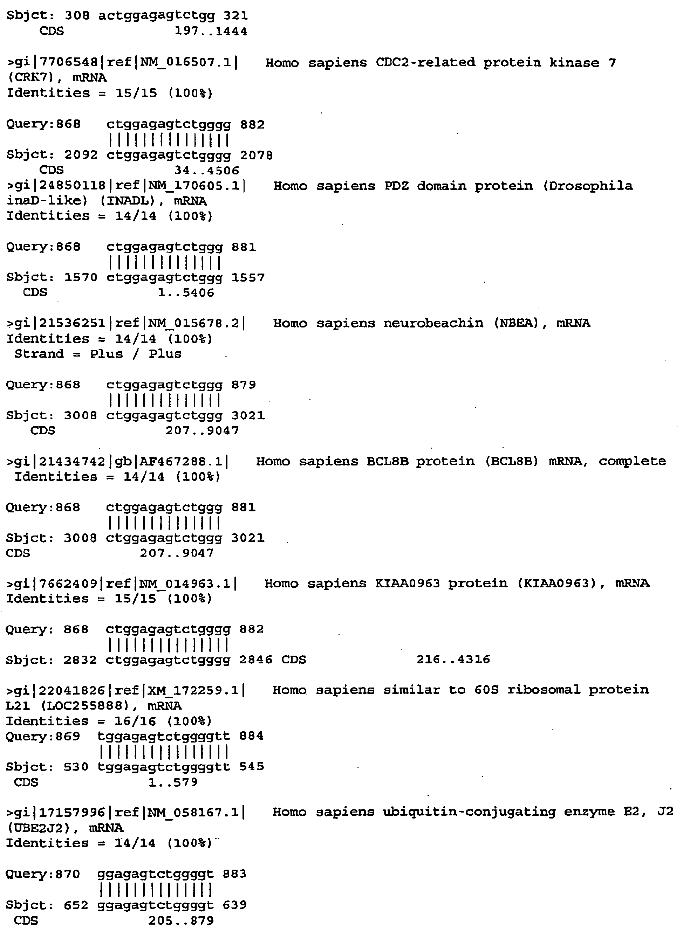

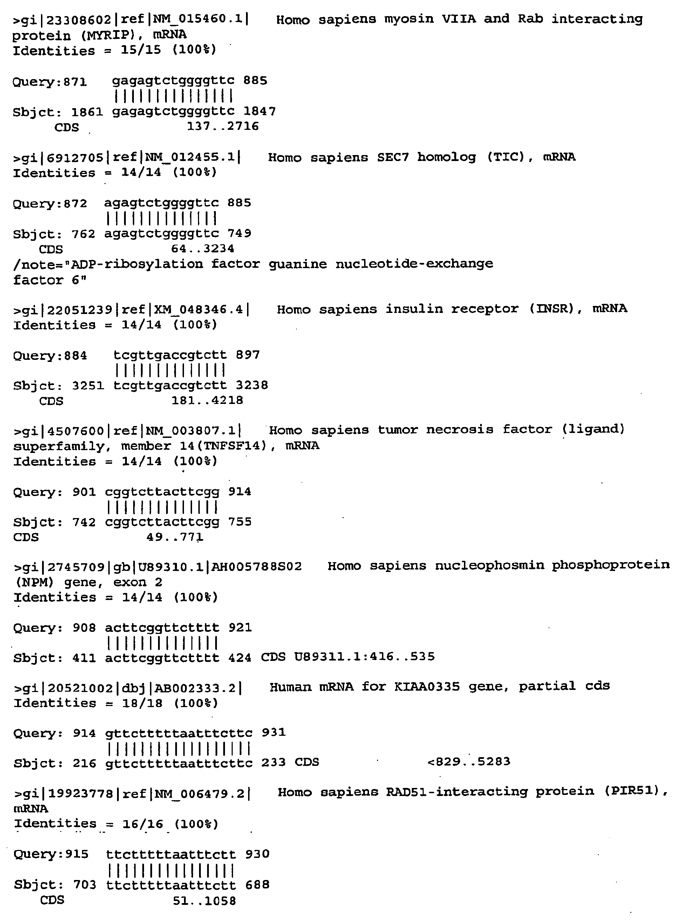

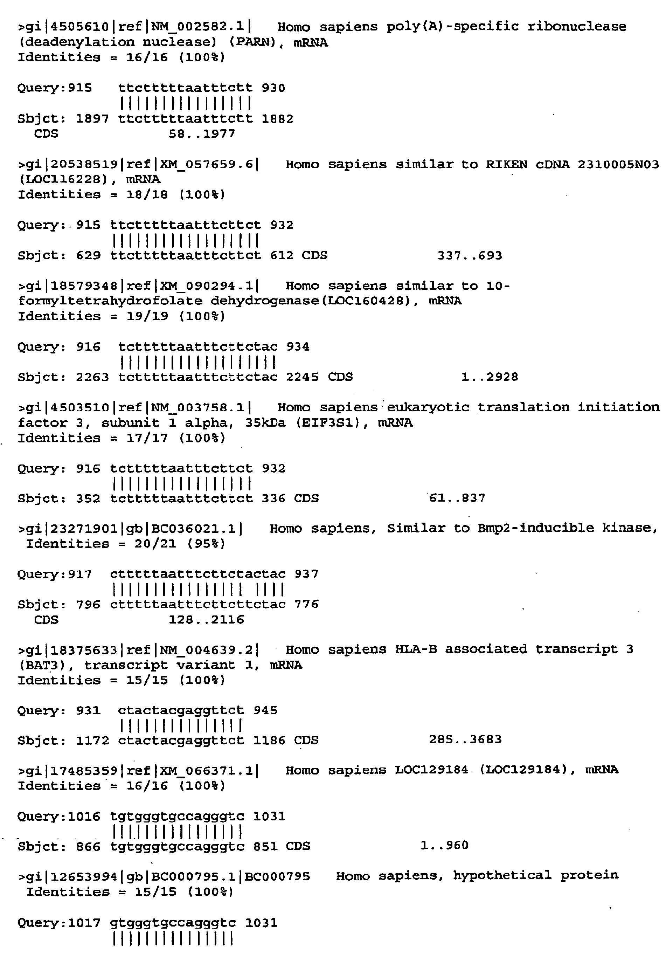

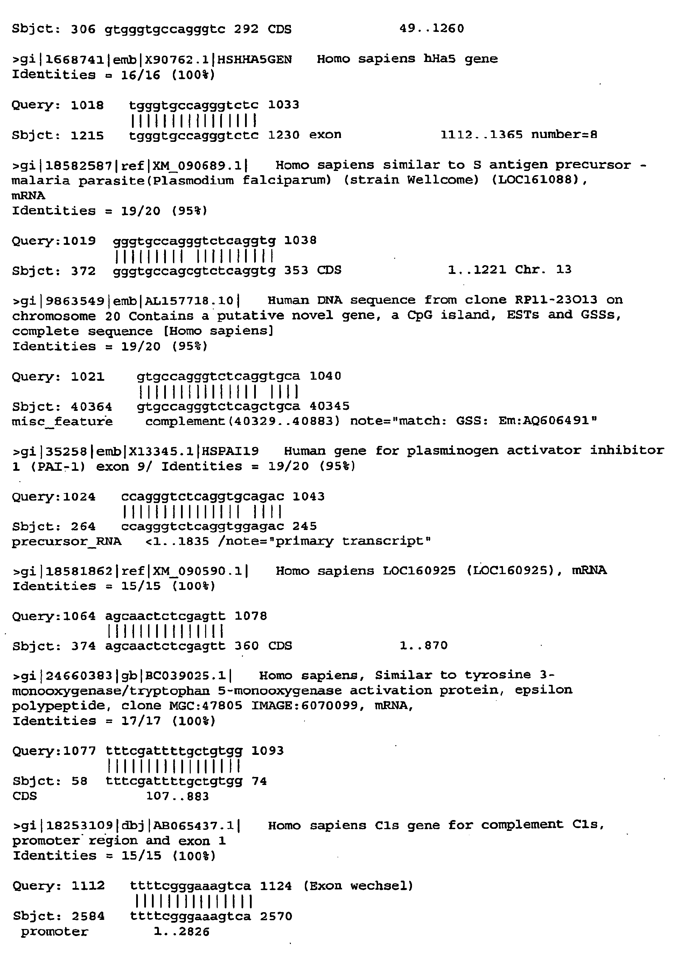

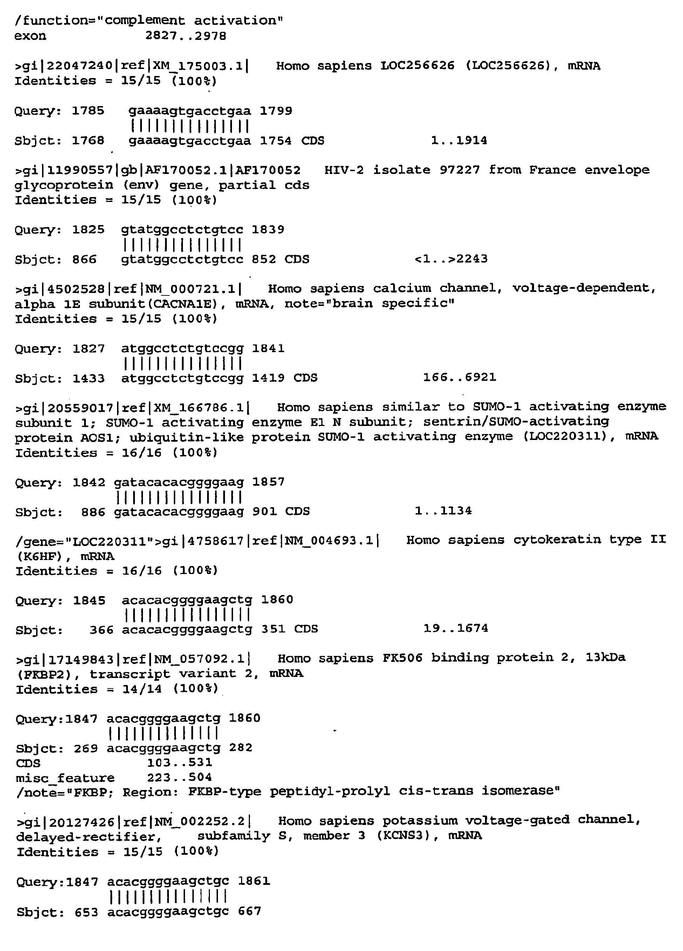

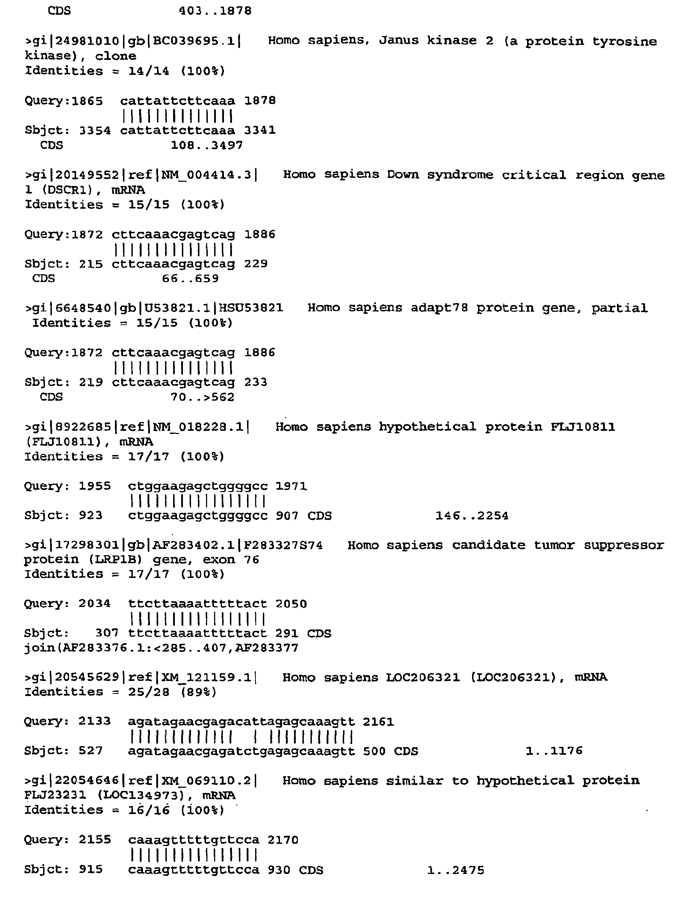

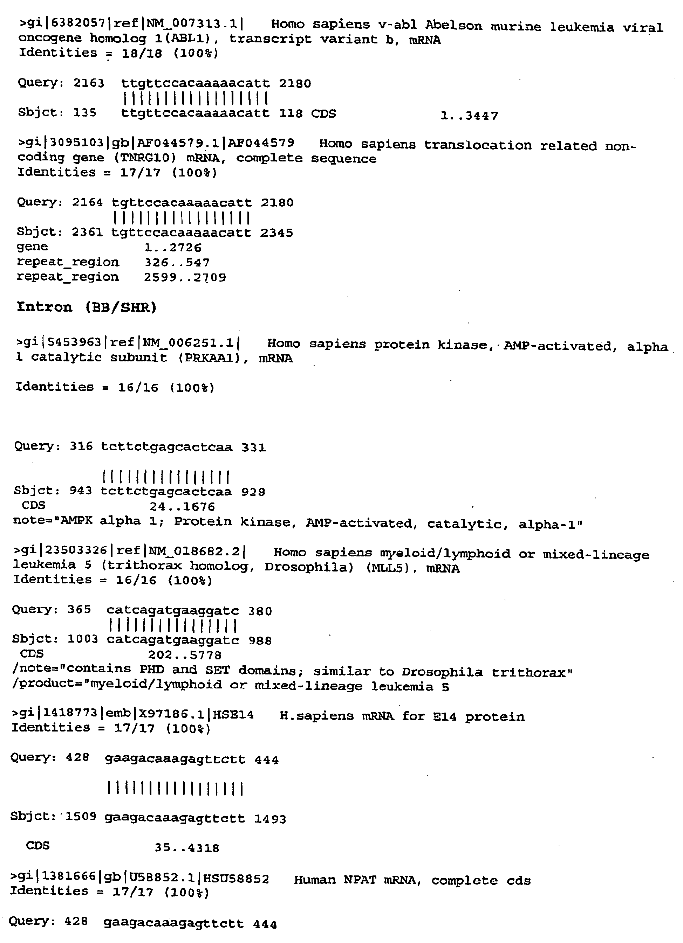

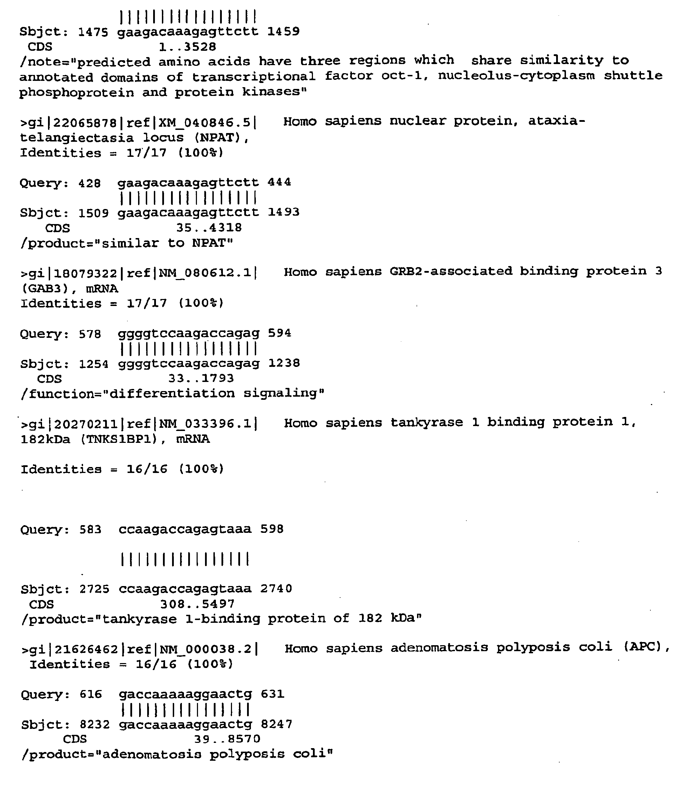

Angesicht der Multifunktionalität von YY1 wurde Antisense-DNA von YY1 mit den in der Genbank vorhandenen Sequenzen auf Überseinstimmung mit anderen Genen geprüft (siehe Figur 13; die angegebenen Positionen (Numerierungen) beziehen sich auf den kodierenden Bereich und nicht auf die Nukleotidnumerierung des Sequenzprotokolls). Diese entsprechenden Sequenzen sollen unter Nutzung von Kern-Lokalisationssignalen (NLS) mit Domänen von YY1 gezielt zur Beeinflussung dieser Gene (funktionelle Domäne) eingesetzt werden (vgl. Fig. 8).

Die Aktivierungsdomänen, Repressionsdomänen und Zinkfinger von YY1 sollen allein und in allen Kombinationsmöglichkeiten miteinander, mit der Antisense-DNA kreiert werden. Die Kombination ist abhängig vom krankheitsbild, den betroffenen Organen und vom Geschlecht.

### Kongene und transgene Tiere

Im Rahmen der vorliegenden Erfindung wurden ferner kongene Ratten, erzeugt, die eine für (das mutierte) YY1 gemäß SEQ ID NO:4 kodierende Nukleinsäuresequenz enthalten. Die Ratten sind durch eine erniedrigte Typ-1-Diabetesinzidenz charakterisiert. In gleicher Weise ist es möglich, transgene Ratten, zu erzeugen, die sich dadurch auszeichnen, dass sie ebenfalls eine für (das mutierte) YY1 kodierende Nukleinsäuresequenz (s.o.) enthalten.

Die Erfindung stellt ferner erstmals ein Verfahren zum Identifieren dianetesprotektiver Wirkstoffe bereit, bei dem den vorgenannten Ratten potentielle Wirksubstanzen verabreicht werden und man überprüft, in wieweit die Neigung, Typ-1-Diabetes zu entwickeln, reduziert wird. Gegenstand der Erfindung ist somit auch die Verwendung einer transgenen oder kongenen nicht-menschlicher Ratte, deren Keim- und somatische Zellen eine Nukleinsäure oder einen Nukleinsäureabschnitt enthalten, die/der für ein Protein mit der in SEQ ID NO:4 (SHR) gezeigten Aminosäuresequenz oder einer dazu homologen Aminosäuresequenz (s.o.) kodiert, wobei die homologe Aminosäuresequenz an Position 303 Methionin und an Position 311 Arginin aufweist, zum Identifizieren Diabetes-protektiver Substanzen.

Die Erfindung betrifft ferner transgene nicht-menschliche Ratten, deren Keim- und somatische Zellen eine Nukleinsäure oder einen Nukleinsäureabschnitt enthalten, die/der für ein Protein mit der in SEQ ID NO:2 gezeigten Aminosäuresequenz oder einer dazu homologen Aminosäuresequenz (s.o.) kodiert, wobei die homologe Aminosäuresequenz an Position 303 Arginin und an Position 311 Lysin aufweist.

### Weitere Erfindungsgegenstände

Die Erfindung betrifft ferner weitere Gegenstände und Ausführungsformen, die sich für den Fachmann vor dem Hintergrund der vorliegenden Offenbarung mühelos erschließen.

In diesem Zusammenhang sind auch Vorrichtungen (Kits) zur Durchführung eines der vorgenannten (Screening-)Verfahren zu nennen.

### Methoden

1. Tierhaltung
2. Erzeugung der kongenen BB.6S
   2.1. Erzeugung weiterer kongener Linien
3. Phänotypische Charakterisierung
   3.1. Diabetesinzidenz und Blutglukosebestimmung
   3.2. Lymphozyten-Phänotypen
   3.3 Blutdruck
4. Statistische Analyse
5. Molekularbiologische Methoden
   5.1. Isolierung Langerhansscher Inseln des Pankreas
   5.2. DNA-Isolierung
   5.3. RNA-Isolierurig aus Geweben
   5.4. RT-PCR
   5.5. Sequenzierung von YY1
   5.6. Eluierung der zweiten Bande bei BB.6S
   5.7. Genexpressionsstudien
   5.8. RNA-Isolierung aus EDTA-Blut
6. Feinkartierung von YY1 (Sicherstellung, dass YY1 in dem diabetesprotektiven Bereich liegt)
7. Prüfung des Polymorphismus im Intron 3 unter Nutzung weiterer Ratten-Stämme
8. Weitere Untersuchungen in vitro
9. Weitere Untersuchungen in vivo
10. Primerbedinguhgen bei der Sequenzierung
11. Mikrosatellitenmarker

### 1. Tierhaltung

Die Tiere wurden unter Semibarrierebedingungen gehalten und hatten freien Zugang zu Pelletfutter (Ssniff R-Zucht, Spezialitäten GmbH, Soest) sowie Wasser (pH 2,5 - 3).

Die Tiere wurden unter einem 12 Stunden Rhythmus gehalten (12h Licht, 12h Dunkelheit). In einem Käfig befanden sich maximal 3 Tiere.

Alle tierexperimentellen Arbeiten erfolgten unter Einhaltung der gültigen Tierschutzbestimmungen.

### 2. Erzeugung der kongenen BB.6S

Käuflich erworbene, männliche SHR/Mol-Ratten (Mollegaard Breeding Ltd, Dänemark) wurden mit diabetischen BB/OK-Weibchen gekreuzt. Die resultierenden F1-Hybriden wurden auf diabetische BB/OK-Ratten insgesamt 7 mal rückgekreuzt. Die Nachkommen jeder Rückkreuzungsgeneration wurden auf Heterozygotie in dem interessierenden chromosomalen mittels PCR-analysierter Mikrosatellitenmarker, die die interessierenden Bereiche flankieren, genetischen analysiert. Die für diesen Bereich heterozygoten Tiere wurden dann für die Erzeugung der nächsten Rückkreuzungsgeneration angepaart. Für den chromosomalen Bereich heterozygoten Tiere der 7 Rückkreuzungsgeneration wurden dann untereinander (intercross) gekreuzt und erneut genetisch analysiert. Zunächst wurden alle Tiere, die für den interessierenden chromosomalen Bereich homozygot für Allele der SHR-Ratten waren, selektiert. Diese Tiere wurden dann erneut mittels 139 PCR-analysierter Mikrosatellitenmarker, die etwa 96% des Genoms der Ratte abdecken, genetisch charakterisiert. Tiere, die dann für diese 139 Marker homozygot für Allele der BB/OK-Ratte waren, wurden für die Etablierung (Founder) der kongenen Linien BB.SHR eingesetzt und phänotypisch charakterisiert.

Das Markerspekrum wurde erweitert, um einerseits den genetischen BB/OK-Background abzusichern (s. Material&Methoden-Teil) und um andererseits möglichst Rekombinationen in kleinen Bereiche erkennen zu können:D6Rat3-D6Rat1-D6wox13-D6Rat101-Ighe/Ckb-D6Mgh2-D6Rat94-D6Rat183-D6Rat10-D6Rat7-D6Rat75-D6Rat9-D6Rat6-D6Rat160-D6Mgh9-D6Rat184.

Diese Linien wurden für die Weiterzucht zwecks Minimierung der Diabetes-protektiven Region eingesetzt. Durch die Etablierung verschiedener subkongener Linien (BB.6Sa-f) mit dem Phänotyp der BB.6S-Ratte, wurde der in Frage kommende chromosomale Bereich auf < 2 cM ( 30-40 Gene) eingegrenzt.

Kartierung des diabetesprotektiven Gens um den Locus D6Mgh2.

**Tabelle 2:**

| cM | | BB.6S | BB.6a | BB.6b | BB.6c | BB.6d | BB.6e | BB.6f |
|---|---|---|---|---|---|---|---|---|
| | D6Mgh4 | BB | BB | BB | BB | BB | BB | BB |
| 2.1 | D6Rat13 | BB | BB | BB | BB | BB | BB | BB |
| 4.4 | D6Wox5 | BB | BB | BB | BB | BB | BB | BB |
| 1.5 | D6Rat66 | BB | BB | BB | BB | BB | BB | BB |
| 2.0 | D6Rat184/ D6Mgh9 | SHR | BB | BB | BB | BB | BB | SHR |
| 0.8 | D6Rat160 | SHR | BB | BB | BB | BB | BB | SHR |
| 1.3 | D6Rat6 | SHR | BB | BB | BB | BB | BB | SHR |
| | D6Rat9 | SHR | BB | BB | BB | BB | BB | SHR |
| 0.4 | D6Rat75 | SHR | BB | BB | BB | BB | BB | |
| 0.4 | D6Rat7 | SHR | BB | BB | BB | BB | BB | SHR |
| 2.1 | D6Rat10 | SHR | BB | BB | BB | BB | BB | SHR |
| 1.6 | D6Rat183 | SHR | BB | SHR | BB | BB | BB | SHR |
| 0.4 | D6Rat94 | SHR | BB | SHR | BB | BB | BB | SHR |
| 1.9 | D6Mgh2 | SHR | SHR | SHR | BB | BB | BB | SHR |
| | Ighe | SHR | SHR | BB | SHR | SHR | SHR | SHR |
| 1.7 | Ckb/D6Rat101 | SHR | SHR | BB | SHR | SHR | BB | SHR |
| 1.0 | D6Rat1 | SHR | SHR | BB | BB | BB | SHR | BB |
| 1.9 | D6Rat3 | SHR | SHR | BB | BB | SHR | SHR | BB |
| | | | | | | | | |
| | Diabetesinzidenz | 15% | 10% | 22% | >50% | >50% | >50% | 14% |

### 2.1. Erzeugung weiterer kongener Linien BB.K(arlsburg)W(ild)R(atte)-Ratten

Neben diabetesresistenten SHR-Ratten wurden auch Wildfangratten zur Erzeugung von kongenen BB.K(arlsburg)W(ild)R(atte)-Ratten verwendet. Analog der BB.6S-Linie wurde der gleiche chromosomale Bereich der BB/OK von 15 cM durch den von Wildfangtieren ausgetauscht (BB.6W). siehe Herstellung kongener Ratten-Linien unter Punkt 2.

### 3. Phänotypische Charakterisierung

Die Körpermasse wurde von nichtdiabetischen Ratten in der zwölften Woche bestimmt.(Waage NAGEMA, VEB Wägetechnik Rapido, Wägebereich 1-1000g, e=0,1g).

Zusätzlich wurden zu diesem Zeitpunkt die Blutglucose, das Serum Insulin, Plasma-Cholesterol und die Triglyzeride bestimmt. Blutproben wurde durch Punktion des Augenplexsuses nach Anästesie mit Isoflurane (Abbott, Wiesbaden, Deutschland) durchgeführt.

Die Blutfette wurden über eine automatische Analyse (Roche Cobas Mira Plus, Roche, Schweiz) durchgeführt.
Das ELISA-Verfahren (Rat Insulin ELISA, Mercodida AB, Uppsala, Schweden) diente zur Bestimmung der Serum-Insulin-Konzentration.

### 3.1. Diabetesinzidenz und Blutglukosebestimmung

Die Diabetesinzidenz der Stämme wurde longitudinal in einer Inzuchtgeneration ermittelt. Dazu wurde vom 50. - 200. Lebenstag zweimal wöchentlich mittels Teststreifen (Diaabur-Test 5000, Boehringer, Mannheim, Deutschland) auf Glukosurie getestet und im positiven Fall der Blutzucker bestimmt (Analyzer ESAT 6660-2, Prüfgerätewerk Medingen GmbH). Wenn innerhalb von 2 Tagen zweimal eine Hyperglykämie (Blutglukose >15 mmol/l entspricht > 300mg/dl) nachgewiesen wurde, galten die Ratten als diabolisch.

### 3.2. Lymphozyten-Phänotyp

Die Lymphozytenisolation von nichtdiabetischen Ratten erfolgte über Dichtgradientenzentrifugation unter Nutzung von Histpaque-1083 (Sigma-Aldrich, Deiseinhofen, Deutschland).

Die Messung erfolgte über eine Zwei-Farben Immunfluoreszenz-Technik. (EPICS Profile II Flow Cytometer (Coulter, Hialeah, USA).

Für die Phänotypisierung wurde eine Mindestanzahl von 0,5 x 106 Zellen festgelegt. Folgende monoklonale Antikörper wurden benutzt:

| Zellen | mAK | Firma |
|---|---|---|
| T-Zellen | R73-FITC | Medizinische Hochschule, Hannover, Deutschland |
| T-Zellen | 3G2-FITC | Medizinische Hochschule, Hannover, Deutschland |
| B-Zellen | OX33-FITC | Pharmigen, Heidelberg, Deutschland |
| NK-Zellen | 10/78-FITC | Pharmigen, Heidelberg, Deutschland |
| TH-Zellen | W3/25-FITC | Serotec, Eching, Deutschland |
| Ts/c-Zellen | 341-FITC | Pharmigen, Heidelberg, Deutschland |
| akt.T-Zellen | OX39-PE | Pharmigen, Heidelberg, Deutschland |
| TH1/TH2-like Zellen | OX-22-PE W3/25-FITC | Pharmigen, Heidelberg, Deutschland Serotec, Eching, Deutschland |

### 3.3. Blutdruck

Der systolische Blutdruck wurde im Alter von 12 bis 14 Wochen zwischen 9.00 bis 11.00 Uhr über die Schwanz-Methode gemessen (Kent Scientific Corporation, Kent, England). Drei separate Punkte wurden benutzt, um einen Endwert zu erhalten.

### 4. Statistische Analyse

Die Berechnung der Mittelwerte und der Standardfehler (SD) erfolgte mit dem Statistikprogramm SPSS. Die Signifikanzen wurden mittels ANOVA Analyse berechnet.

### 5. Molekularbiologische Methoden

### 5.1. Isolierung Langerhansscher Inseln des Pankreas

Die Isolierung der Langerhanschen Inseln erfolgte mittels Kollagenaseverdau (Biochrom) und Dextran-Diclitegradientenzentrifugation (Dextran, Fluka, Schweiz). Es wurden mindestents 3 und maximal 15 Pankreata pro Kollagenaseverdau eingesetzt.

Nach Entnahme der Pankreata wurden diese in die Kollagenaselösung (30 mg Kollagenase (Biochrom) gelöst in 40 ml Hank's Salzlösung (Sigma) bei 37°C für 10 min) überführt und mittels Handschütteln verdaut. Anschließend wurde mit Hanks'Salzlösung das Inselsediment dreimal gewaschen. Nach Abzentrifugation der Inseln (1900 U/min) wurde der Überstand dekantiert und mit Dextranlösung (11 g Dextran/39 ml Hank's Salzlösung) aufgewirbelt. Die Inselemulsion wurde anschließend mit 3 weiteren Dichtegradienten von jeweils 4 ml überschichtet. Nach 20-minütiger Zentrifugation (1900 U/min) erfolgte die Handlesung der Inseln unter dem Mikroskop. Zur Genexpressionsanalyse wurden jeweils 500 Langerhanssche Inseln eingesetzt.

### 5.2. DNA-Isolierung

Die Isolierung der genomische DNA aus Lebergewebe wurde nach Vorschrift des Herstellers (Wizard ^{®}, Genomic DNA Purification Kit, Promega) durchgeführt.

### 5.3. RNA-Isolierung aus Geweben

Die RNA-Isolierung erfolgte nach den Angaben des Herstellers (RnEasy Mini Kit, Qiagen).

### 5.4. RT-PCR

Die Umschreibung der RNA in ssDNA (cDNA) erfolgte mit dem Reversen Transcription System der Firma Promega.

Für die Umschreibung wurde die RNÄ-Konzentration (Photometer, Eppendorf) auf 1,5 µg eingestellt.

Die 1,5µg RNA wurden mit 1µl Random-Hexamerprimer (Promega) versetzt und anschließend auf 13,5µl Endvolumen mit Rnase freiem Wasser (Promega) aufgefüllt. Anschließend wurde dieser RNA-Mix für 5 min bei 65°C erhitzt und danach auf Eis für eine Minute abgekühlt. Nach Zugabe des RT (Reverse Transkriptase)-Mixes (6,5 µl) erfolgte die Umschreibung im Cycler (Thermocycler, Techne, Cambridge) nach folgendem Programm:
50 min 42°C
10 min 70°C

### RT-Mix enthält:

| Volumen | Komponenten | Firma |
|---|---|---|
| + 4µl | 5x MMLV-Puffer | Promega |
| + 1µl | 10mM dNTP's | Promega |
| + 0,5µl | rRNasin | Promega |
| + 1µl | MMLV-Reverse Transkritase | Promega |
| 6,5µl | Endvolumen | |

Nach Beendigung des Cycler-Programmes wurde die ssDNA mit 50µl bidest-Wasser versetzt.

Die ssDNA wurde für die Expressionsstudien sowie für die Sequenzierung genutzt.

### 5.5. Sequenzierung von YY1

Zur Sequenzierung des Gens wurde genomische DNA aus Lebergewebe (Wizard ^{®}, Genomic DNA Purification Kit, Promega) und RNA aus isolierten Langerhansschen Inseln, Pankreas, Leber und Gehirn der BB/OK-, SHR- und BB.6S-Ratte gewonnen (Rneasy Mini Kit, Qiagen). RNA wurde dann in ssDNA bzw. cDNA (siehe Pkt. 5.4) nach Angaben des Herstellers umgeschrieben (Reverse Transcription System, Promega). Sowohl genomische als auch cDNA wurde mittels PCR (Einsatz von GeneAmp^{®} High Fidelity PCR System, Applied BioBiosystems) unter Verwendung verschiedener Primer so amplifiziert, daß überlappende Genprodukte zur Sequenzierung zur Verfügung standen (vgl. Fig. 11; Promotor: K823-F/K825-R*; Promotor und Exon 1: K884-F/K806-R; Exon 1: K801-F/K804-R; K814-F/KK832-R*; Exon 2 und 3: K828-F/K833-R; K831-F/K817-R; Exon 4: K815-F/K870-R; K815-F/K818-R; K816-F/K819-R; K834-F/K836-R). Die PCR wurde wie folgt durchgeführt: 3min 94 °C, gefolgt von 35 Zyklen a 30sec 94 °C, 1 min 55 oder 60 °C (*), 45 sec 72 °C mit anschließender Elongation von 3min bei 72 °C.

Die amplifizierten Genprodukte wurden nach Reinigung mit Amicon^{®}Microcon-PCR-Zentrifalfiltern (Millipore, USA) für die Sequenzier-PCR verwandt. Die gereinigten PCR-Produkte (250 ng) wurden unter Verwendung von ABIPRISM^{®} BigDye Terminators v3.0 Cycle Sequenzing Kit nach Vorschrift des Herstellers (Applied Biosystems) amplifiziert (25 Zyklen: 10 sec. 96 °C, 4 min 60°C). Die amplifizierten Genprodukte wurden anschließend mit Alkohol gefällt, getrocknet, in Formamid (4 µl) und Loading Buffer (1µl) zugenommen, 2 min bei 90 °C denaturiert und danach mit dem ABI PRISM^{®} 377 (Applied Biosystems) sequenziert. Die Sequenzauswertung, einschließlich der Erstellung der Proteinsequenz erfolgte mit der Sequenzanalysesystemsoftware v3.4.1 (Applied Biosystems, USA). Die Genprodukte wurden wiederholt sequenziert und analysiert, um Artefakte ausschließen zu können.

Die Prüfung der Sequenz mit der Genbank erfolgte online (www.ncbi.nlm.nih.gov/BLAST).

### 5.6. Eluierung der zweiten Bande bei BB.6S

Es wurde nach der gelelektrophoretischen Auftrennung die zweite Bande (Pankreas, Leber, Gehirn) herausgeschnitten und mit dem Wizard^{®}PCR Preps DNA Purification System der Firma Promega eluiert. Nach Amplifizierung mit den Primern K831-F/K870-R erfolgte die Sequenzierung am ABI PRISM^{®} 377 (Applied BioBiosystems (vgl. Fig. 12 und SEQ ID NO:7).

### 5.7. Genexpressionsstudien

Aus gewonnener RNA von isolierten Langerhansschen Inseln, Pankreas, Leber, Gehirn, Thymus und Milz von 8 Tage alten Ratten der Inzuchtstämme BB/OK, SHR, BB.6S und LEW ("unbelastete" Kontrolle) erfolgte die Umschreibung in ssDNA (siehe Punkt 5.4). Die optimalen PCR-Bedingungen wurden für jedes Primerpaar unter Verwendung von ssDNA diabetes-resistenter LEW-Ratten erarbeitet. Als Kontrollsituation (housekeeping gene) wurde die Genexpression von GAPDH zugrunde gelegt. Gewebsspezifische cDNA wurde mit den Primern für GAPDH (F: 5'TCCCTCAAGATTGTCAGCAA3'; R: 5'AGATCCACAAACGGATACATT3', Produktgröße = 308bp) und den YY1-Primern K801-F/KK804-R (Exon 1) und K831-F/K8818-R bzw. K831-F/K870-R (Zinkfinger) amplifiziert. Die Genprodukte wurden gelelektrophoretisch aufgetrennt und die Expressionsstärke zwischen den Stämmen unter Berücksichtigung von GAPDH und Nutzung des Typhoon 8600 Variable Mode Imager (Amersham-Pharmacia Biotech, U.K.) quantifiziert.

### 5.8 RNA-Isolierung aus EDTA-Blut

| Produkt | Firma |
|---|---|
| QIAamp^{®} RNA Blood Mini Kit | QIAGEN |

zusätzliche Nutzung von:

| Produkt | Firma |
|---|---|
| QIAshredder | QIAGEN |
| RQ1-RNase free DNase | Promega |

- ausführliche Anleitung im mitgelieferten QIAGEN Mini Handbook

### 6. Feinkartierung von YY1 (Sicherstellung, dass YY1 in dem diabetesprotektiven Bereich liegt)

Die Feinkartierung erfolgte mit Hilfe der Maus-Gensequenz. Anhand dieser Gensequenz konnten Primer hergestellt werden.

### 7. Prüfung des Polymorphismus im Intron 3 unter Nutzung weiterer Ratten-Stämme

Dazu wurden folgende Stämme, die unter gleichen Bedingungen wie BB.6S gehalten werden, benutzt: 2 Wildfangtiere (Wildtyp, M+W), jeweils 1 Tier von diabetes-resistenten, ingezüchteten DA/K, BN/Mol, LEW/K und WOKW-Ratten.

### 8. Weitere Untersuchungen in vitro

Morphologische Untersuchungen des Pankreas von normoglykämischen BB/OK- und BB.6S-Ratten (Lucke, S., Klöting, I., Pusch, A., Heinrich, H.W. and H..J. Hahn. Endocrine pancreas histology of congenic BB-rat strains with reduced diabetes incidence after genetic manipulation on chromosomes 4, 6 and X. Autoimmunity 36, 2003, 143-149).

Untersuchungen an isolierten Langerhansschen Inseln von BB/OK- und BB.6S- Ratten durch Variation der Ca-Konzentration im Kulturmedium.

Calbindin-D28k Proteinexpression mittels Westernblotanalyse unter Basalbedingungen und mit zunehmender Ca-Konzentration im Medium.

### 9. Weitere Untersuchungen in vivo

Untersuchungen zur Frakturheilung bei BB/OK- und diabetes-resistenten LEW.1A-Ratten (38).

BB/OK und BB.6S-Ratten wurden mit Standarddiät (0,8% Ca), mit Ca-reicher (2%) und Ca-armer Diät (0,4%) ernährt (hergestellt von Ssniff R-Zucht, Spezialitäten GmbH, Soest) und bis zur 30. Lebenswoche auf Diabetesentwicklung beobachtet (nicht publiziert).

Verabreichung von Ca-Kanal-Blockern oder verschiedenen Ca-Antagonisten (Quercitin). (nicht publiziert)

### 10. Primerbedingungen bei der Sequenzierung

Für alle Primer gelten folgende Bedingungen:
Denaturierung: 3 min 94 °C
Annealing: 35 Zyklen a 30sec 94°C
   1 min 55 oder 60 °C (*)
   45 sec 72 °C
Elongation: 3 min bei 72 °C.

### 11. Mikrosatellitenmarker

| **Chr.** | **Intern** | **Marker** | **Gesamt** |
|---|---|---|---|
| 1 | R152 | Igf2 | |
| 1 | K6 | D1Mgh10 | |
| 1 | K7 | D1Mgh12 | |
| 1 | K57 | D1Mgh7 | |
| 1 | K58 | D1Mit3 | |
| 1 | K109 | D1Mgh6 | |
| 1 | K136 | D1Mgh4 | |
| 1 | K220 | D1Mgh18 | |
| 1 | K228 | D1Mgh14 | |
| 1 | K524 | D1Rat249 | |
| 1 | K526 | D1Rat167 | |
| 1 | K533 | D1Rat60 | |
| | | | 12 |
| 2 | K110 | D2Mit8 | |
| 2 | K111 | D2Mgh9 | |
| 2 | K138 | D2Mit1 | |
| 2 | K139 | D2Mit4 | |
| 2 | K140 | D2Mit15 | |
| 2 | K230 | D2Mgh14 | |
| 2 | K238 | D2Mit7 | |
| 2 (5) | K280 | D5Mgh7 | |
| 2 | K374 | D2Wox8 | |
| 2 | K376 | D2Wox34 | |
| 2 | K384 | D2Wox1 | |
| | | | 11 |
| 3 | R142 | Svs2p | |
| 3 | K63 | D3Mit1 | |
| 3 | K66 | D3Mgh11 | |
| 3 | K68 | D3Mit10 | |
| 3 | K386 | D3Wox1 | |
| 3 | K393 | D3Wox16 | |
| 3 | K395 | D3Wox25 | |
| | | | 7 |
| 4 | R88 | I1-6 | |
| 4 | K147 | Eno | |
| 4 | K215 | Nos | |
| 4 | K253 | D4Mit9 | |
| 4 | K262 | D4Mit16 | |
| 4 | K264 | D4Mgh9 | |
| 4 | K267 | D4Mgh11 | |
| 4 | K547 | D4Rat9 | |
| | | | 8 |
| 5 | R44 | A2ug | |
| 5 | Cjun | Cjun | |
| 5 | K72 | D5Mgh15 | |
| 5 | K151 | D5Mgh5 | |
| 5 | K270 | D5Mgh1 | |
| 5 | K404 | D5Wox26 | |
| 5 | K412 | D5Wox17 | |
| | | | 7 |
| 6 | R99 | Ckb | |
| 6 | K73 | D6Mgh4 | |
| 6 | K74 | D6Mit5 | |
| 6 | K154 | D6Mit1 | |
| 6 | K155 | D6Mgh5 | |
| 6 | K156 | D6Mit4 | |
| 6 | K158 | D6Mit9 | |
| 6 | K159 | D6Pasl | |
| 6 | K284 | D6Mgh2 | |
| 6 | K288 | D6Mit8 | |
| 6 | K289 | D6Mit2 | |
| 6 | K290 | D6Mit3 | |
| 6 | K416 | D6Wox2 | |
| 6 | K417 | D6Wox17 | |
| 6 | K420 | D6Wox10 | |
| 6 | K506 | D6Kyo4 | |
| 6 | K549 | D6Rat184 | |
| 6 | K554 | D6Rat160 | |
| 6 | K556 | D6Rat101 | |
| 6 | K557 | D6Rat31 | |
| 6 | K625 | D6Rat66 | |
| | | | 21 |
| 7 | K26 | D7Mit9 | |
| 7 | K77 | D7Mit7 | |
| 7 | K162 | D7Mgh7 | |
| 7 | K163 | D7Mgh9 | |
| 7 | K300 | D7Mit16 | |
| 7 | K427 | D7Wox28 | |
| 7 | K428 | D7Wox15 | |
| 7 | K435 | D7Wox2 | |
| | | | 8 |
| 8 | R102 | Apoc3 | |
| 8 | K84 | D8Mit4 | |
| 8 | K116 | D8Mit2 | |
| 8 | K117 | D8Mgh11 | |
| 8 | K166 | D8Mgh4 | |
| 8 | K437 | D8Wox23 | |
| 8 | K444 | D8Wox12 | |
| 8 | K560 | D8Rat51 | |
| | | | 8 |
| 9 | R27 | Cryg | |
| 9 | Inhb | Inhb | |
| 9 | K87 | D9Mit1 | |
| 9 | K88 | D9Mgh2 | |
| 9 | K171 | Aep2 | |
| 9 | K451 | D9Wox20 | |
| 9 | K453 | D9Mgh5 | |
| | | | 7 |
| 10 | R126 | Aep | |
| 10 | Il-4 | I1-4 | |
| 10 | K30 | D10Mgh2 | |
| 10 | K32 | D10Mit10 | |
| 10 | K119 | D10Mgh6 | |
| 10 | K644 | D10Rat4 | |
| 10 | K646 | D10Rat8 | |
| | | | 7 |
| 11 | R22 | Smst | |
| 11 | K39 | D11Mgh5 | |
| 11 | K91 | D11Mit2 | |
| 11 | K120 | D11Mgh3 | |
| 11 | K459 | D11Wox4 | |
| 11 | K516 | D11Rat22 | |
| | | | 6 |
| 12 | K42 | D12Mit5 | |
| 12 | K179 | D12Mit2 | |
| 12 | K182 | D12Mgh4 | |
| 12 | K183 | D12Mit4 | |
| 12 | K468 | D12Wox15 | |
| | | | 5 |
| 13 | Atpa | Atpa | |
| 13 | K11 | D13UW | |
| 13 | K93 | D13Mit1 | |
| 13 | K122 | D13Mgh8 | |
| 13 | K189 | D13Mgh7 | |
| | | | 5 |
| 14 | R40 | Alb | |
| 14 | R130 | Gck | |
| 14 | K99 | D14Mgh2 | |
| 14 | K100 | D14Mgh1 | |
| 14 | K128 | Csna | |
| 14 | K130 | D14Mgh3 | |
| 14 | K337 | D14Mgh4 | |
| 14 | K584 | D14Rat65 | |
| 14 | K588 | D14Rat37 | |
| | | | 9 |
| 15 | K45 | D15Mgh3 | |
| 15 | K102 | D15Mgh6 | |
| 15 | K123 | D15Mgh5 | |
| 15 | K482 | D15Wox5 | |
| 15 | K483 | D15Wox3 | |
| | | | 5 |
| 16 | K48 | D16Mgh4 | |
| 16 | K49 | D16Mit2 | |
| 16 | K170 | D9Mgh1 | |
| 16 | K486 | D16Wox7 | |
| | | | 4 |
| 17 | K35 | D17Mgh3 | |
| 17 | K36 | D17Mgh5 | |
| 17 | K104 | D17Mit7 | |
| 17 | K105 | D17Mit5 | |
| 17 | K491 | D17Wox8 | |
| | | | 5 |
| 18 | R66 | Olf | |
| 18 | R98 | Gjai | |
| 18 | Mit9 | D18Mit9 | |
| 18 | K12 | D18Mgh3 | |
| 18 | K202 | D18Mit4 | |
| 18 | K511 | D18Kyol | |
| 18 | K559 | D18Rat44 | |
| | | | 7 |
| 19 | K206 | D19Mit7 | |
| 19 | K569 | D19Rat12 | |
| 19 | K572 | D19Rat3 | |
| 19 | K575 | D19Rat34 | |
| | | | 4 |
| 20 | R145 | Tnf | |
| 20 | K108 | D20Mgh1 | |
| 20 | K127 | Prkacn | |
| | | | 3 |
| X | Mg3 | DXMgh3 | |
| X | K15 | DXMgh1 | |
| X | R87 | Pfkfb | |
| X | R47 | Ar | |
| X | K657 | DXRat17 | |
| X | K654 | DXRat19 | |
| X | K661 | DXRat103 | |
| X | K661 | DXRat103 | |
| X | K364 | DXWox29 | |
| X | K352 | DXWox17 | |
| X | K359 | DXWox24 | |
| | | | 11 |
| Gesamt | | | 160 |

### Referenzen

1 She, J-X. and M.P. Marrron. Genetic susceptibility factors in typ1 diabetes: linkage, disequilibrium and functional analyses, Cur. Opin. Immunol., 10, 1998, 682-689
2 Morahan, G., Huang, D., Ymer, S.I. et al. Linkage disequilibrium of a type 1 diabetes susceptibility locus with a regulatory IL12B allele. Nature Genet. 27, 2001, 218-221
3 Klöting, I. and L. Vogt. BB/O(ttawa)K (arlsburg) rats: Features of a subline of diabetes-prone BB rats. Diabetes Res., 1991,18,79-87
4 Lühder, F., Woltanski, K.P., Hamann, J. et al. Detection of antibodies against both isoforms of glutamate decarboxylase in BB/OK rats by western blotting and immuno trapping enzyme activity assay. Diabetes Res. 20, 1992, 97-107
5 Ziegler, B., Witt, S., Kohnert, K.D. et al. Characterization of monoclonal islet cell reactive autoantibodies from the diabetic Biobreeding (BB/OK) rat. Acta Diabetol.30, 1993, 201-206
6 Schröder, D., Schmidt, S., Klöting, I. and B. Hehmke. Effect of syngeneic islet antigen administration on complement-dependent antibody-mediated cytotoxicity to islet cells and diabetes onset in diabetes-prone BB/OK rats. Autoimmunity 14,1993, 283-289
7 Vogt, L. and I. Klöting. Genetic analysis of frequencies of phenotypes in the spontaneously diabetic BB rat- a main animal model of autoimmune type-I-diabetes. Diabetes Res., 22, 1993, 105-113
8 Klöting, I., Stark, O. and U. Fischer. Incidence of diabetes in F2 and first backcross hybrids of BB rats of different origin. In: Lessons from animal diabetes II/Eds. Shafrir, Renold, -London, Paris, J.Libbey, 1988, 85-87
9 Klöting, I., and Vogt, L. (1990). Coat colour phenotype, leucopenia and insulin-dependent diabetes mellitus in BB rats. Diabetes Res. 15, 37-39
10 Klöting, I., Stielow, M. and L. Vogt. Development of new animal models in diabetes research: spontaneously hypertensive-diabetic rats. Diabetes Res. 29, 1995, 127-138
11 Klöting, I. and P. Kovacs. Crosses between diabetic BB/OK and wild rats confirm that a 3rd gene is essential for diabetes development. Acta Diabetol. 35, 1998, 109-111
12 Klöting, I., Voigt, B. and P. Kovács. Comparison of genetic variability at microsatellite loci in wild rats and inbred rat strains (Rattus norvegicus). Mamm. Genome. 8, 1997, 589-591
13 Klöting, I., Vogt, L. and T. Serikawa. Locus on chromosome 18 cosegregates with diabetes in the BB/OK rat subline. Diabet. Metab. 21, 1995, 338-344
14 Klöting, I., S. Schmidt and P. Kovacs. Mapping of novel diabetes predisposing and protecting genes in the spontaneously diabetic BB/OK rat. Biochem. Biophys. Res. Commun. 245, 1998, 483-486
15 Klöting, I. and P. Kovacs. Phenotypic differences between diabetes-prone BB rat sublines cosegregate with loci on chromosomes X and 10. Biochem. Mol. Biol. Int. 45, 1998, 865-870
16 Klöting, I. and P. Kovacs. Genes of the immune system cosegregate with the age at onset of diabetes in the BB/OK rat. Biochem. Biophys. Res. Commun. 242, 1998, 461-463
17 Klöting, I. and P. Kovacs. Loci of the immune system are implicated in diabetes frequency and age at onset of diabetes in BB rats. J. Exp. Anim. Sci. 41, 2000, 19-21
18 Klöting, I., Vogt, L., Reetz, I.C. et al. New congenic rat strains for the separate study of MHC and non-MHC genetic effects in the development of diabetes in BB rats. Diabetes Res. 15, 1990, 41-45
19 Voigt, B., Berg, S., Kovács, P. et al. Congenic spontaneously diabetic hypertensive BB.SHR rats. Transplant. Proc. 29, 1997, 1677-1678
20 Klöting, I., Kovacs, P. and B. Kuttler. Phenotypic consequences after restoration of lymphopenia in the diabetes-prone BB/OK rat. Biochem. Biophys. Res. Commun. 239, 1997, 106-110
21 Klöting, I. Voigt, B. and P. Kovacs. Metabolic features of newly established congenic diabetes-prone BB.SHR rat strains. Life Sci. 62, 1998, 973 - 979
22 Kovacs, P. and I. Klöting. Congenic strain confirms putative quantitative trait locus for body weight in the rat. Mamm. Genome 9, 1998, 294-296
23 van den Brandt, J., Kovacs, P. and I. Klöting. Congenic diabetes-prone BB.Sa and BB.Xs rats differ from their progenitor strain BB/OK in frequency and severity of inulindependent diabetes mellitus. Biochem. Biophys. Res. Commun. 263, 1999, 843-847
24 Klöting, I., van den Brandt, J. and B. Kuttler. Genes of SHR rats protect spontaneously diabetic BB/OK rats from diabetes: Lessons from congenic BB.SHR rat strains. Biochem. Biophys. Res. Commun.283, 2001, 399-405
25 Podolin, P.L., Denny, P., Lord, C.J. et al. Congenic mapping of the insulin-dependent diabetes (idd) gene, Idd10, localises two genes mediating the Idd10 Effect and eliminates the candidate Fcgr1. J. Immunology 159, 1997, 1835-1843
26 Podolin, P.L., Denny, P., Armitage, N. et al. Localisation of two insulin-dependent diabetes genes to the Idd10 region on mouse chromosome 3. Mamm. Genome 9, 1998, 283-286
27 Lyons , P.A., Hancock, W.W., Denny, P. et al. The NOD Idd9 genetic interval influences the pathogenicity of insulitis and contains molecular variants of Cd30, Tnfr2, and Cd137. Immunity 13, 2000, 107-115
28 McDuffie, M. Derivation of diabetes-resistant congenic lines from the nonobese diabetic mouse. Clinical Immunology 96, 2000, 119-130
29 Norman, R. A., Dzielak, D. J., Bost, K. L. et al. Galloway. Immune dysfunction contributes to the aetiology of spontaneous hypertension. J. Hypertens. 3, 1985, 261-268
30 Fannon, L.D., Braylan, R. C. and M.I. Phillips. Alterations of lymphocyte populations during development in the spontaneously hypertensive rat. J. Hypertens. 10, 1992, 629-634
31 Ofosu-Appiah, W. and C. Ruggiero. Abnormal activation and loss of suppressor T cells in the spontaneously hypertensive rat. Cell Immunol. 145, 1992, 130-145
32 Klöting, I., van den Brandt, J. and B. Kuttler Different and gradual protection from spontaneous diabetes by alleles of SHR and Wild rats in congenic BB.SHR and BB.KWR rat strains. 10th International Symposium on SHR and Molecular Medicine, 2.-4.5.2001, Berlin-Buch. J. Mol. Med. 79, 2001, B12, Abstr.
33 Watanabe, T.K., Bihoreau, M.T., McCarthy, L.C. et al. A radiation hybrid map of the rat genome containing 5,255 markers. Nat. Genet. 22, 1999, 27-36
34 Tomas, E., Lin, Y.S., Dagher, Z. et al. Hyperglycemia and insulin resistance: possible mechanisms. Ann. N.Y. Acad. Sci. 967, 2002, 43-51
35 Lawlor, M.A. and D.R. Alessi. PKB/Akt: a key mediator of cell proliferation, survival and insulin response. J. Cell Sci. 114, 2001, 2903-2910
36 Summers, S.A., Yin, V.P., Whiteman, E.L. et al. Signaling pathways mediating insulin-stimulated glucose transport. Ann. N.Y. Acad. Sci. 892, 1999, 169-186
37 Lucke, S., Klöting, I., Pusch, A., et.al. Endocrine pancreas histology of congenic BB-rat strains with reduced incidence after genetic manipulation on chromosoms 4,6 and X. Autoimmunity 36, 2003, 143-149)
38 Follak, N., Klöting, I. Ganzer, D. et al. Scanning electron microscopic examinations on retarted bone defect healing in spontaneously diabetic BB/O(ttawa)K(arlsburg) rats. Histol. Histopathol.18, 2003,111-120
39 Pondel, M. Calcitonin and calcitonin receptors: bone and beyond. Int. J. Exp. Pathol. 81, 2001, 405-422
40 Ahren, B. Autonomic regulation of islet hormone secretion-Implications for health and disease. Diabetologia 43, 2000, 393-410
41 Rabinovitch, A., Suarez-Pinzon, W.L., Sooy, K. et al. Expression of calbindin-D(28k) in a pancreatic islet betacell line protects against cytokine-induced apoptosis and necrosis. Endocrinology 142, 2001, 3649-3655
42 Sooy, K., Schermerhorn, T., Noda, M. et al. Calbindin-D(28k) controls (Ca(2+)(i) and insulin release. Evidence obtained from calbindin-d(28k) knockout mice and beta cell lines. J. Biol. Chem. 274, 1999, 34343-34349
43 Lee, J.S., See, R..H., Galvin, K.M. et al. Functional interactions between YY1 and adenovirus E1A. Nucleic Acids Res. 23, 1995, 925-931
44 Bushmeyer, S.M. and M.L. Atchison. Identification of YY1 sequences necessary for association with the nuclear matrix and for transcriptional repression functions. J. Cell Biochem.68, 1998, 484-499
45 Galvin, K.M. and Y. Shi. Multiple mechanisms of transcriptional repression by YY1. Mol. Cell Biol. 17,1997, 3723-3732
46 Houbaviy, H.B., Usheva, A., Shenk, T. et al. Cocrystal structure of YY1 bound to the adeno-associated virus P5 initiator. Proc. Natl. Acad. Sci. 93, 1996, 13577-13582
47 Lewis, B.A., Tullis, G., Seto, E. et al. Adenovirus E1A proteins interact with the cellular YY1 transcription factor. J. Virol.69, 1995,1628-1636
48 Yang, W.M., Inouye, C., Zeng, Y. et al. Transcriptional repression by YY1 is mediated by interaction with a mammalian homolog of the yeast global regulator RPD3. Proc. Natl. Acad. Sci. USA 93,1996,12845-12850
49 Thomas, J.M. and E. Seto. Unlocking the mechanisms of transcription factor YY1: are chromatin modifying enzymes the key. Gene 236, 1999, 197-208
50 Shrivastava, A., Saleque, S., Kalpana, S. et al. Inhibition of transcriptional regulator Yin-Yang-1 by association with c-Myc. Science 262,1993,1889-1892
51 Austen, M., Luscher, B. and J.M. Luscher-Firzlaff. Characterization of the transcriptional regulator YY1. The bipartite transactivation domain is dependent of interaction with the TATA box-binding protein, transcription factor IIB, TAFII55, or c-AMP-responsive element-binding protein (CBP)-binding protein. J. Biol. Chem.272, 1997,1709-1717
52 Seto, E., Lewis, B. and T. Shenk. Interaction between transcription factor SP1 and YY1. Nature 365, 1993, 462-464
53 Lee, J.S., Galvin, K.M. and Y. Shi. Evidence of physical interaction between the zinc-finger transcription factors YY1 and SP1. Proc. Natl. Acad. Sci. USA 90,1993, 6145-6149
54 Lee ,J.S., Zang, X. and Y. Shi. Differential interaction of the CREB/ATF family of transcription factors with p300 and adenovirus E1A. J. Biol. Chem. 271, 1996, 17666-17674
55 Lee, J.S., Galvin, K.M.,See, R.H. et al. Relief of YY1 transcriptional repression by adenovirus E1A is mediated by E1A-associated protein p300.Genes Devel. 9, 1995, 1188-1198
56 Hyde-DeRuyscher, R.P., Jennings, E. and T. Shenk. DNA binding sites for the transcriptional activator/repressor YY1. Nucl. Acid Res. 23, 1995, 4457-4465
57 Tauton, J., Hassig, C.A. and S.L. Schreiber. A mammalian histone deacetylase related to the yeast transcriptional regulator Rpd3p. Science 272,1996, 408-411
58 Yang, W.M., Yao, Y.L., Sun, J.M. et al. Isolation and characterization of c-DNAs corresponding to an additional member of the human histone deacetylase gene family. J. Biol. Chem.272, 1997, 28001-28007
59 Hassig, C.A. and S.L. Schreiber. Nuclear histone acetylases and deacetylases and transcriptional regulation: HATs off to HDACs. Curr. Opin. Chem. Biol.1, 1997, 300-308
60 Hassig, C.A., Tong J.K., Fleischer T.C. et al. A role of histone deacetylase activity in HDAC1-mediated transcriptional repression. Proc. Natl. Acad. Sci. USA 95, 1998, 3519-3524
61 Shi, Y., Seto, E., Chang, L. et al. Transcriptional repression by YY1, a human GLI-Krueppel-related protein and relief of repression by adenovirus E1A protein. Cell 67, 1991, 377-388
62 Usheva, A. and T. Shenk. TATA-binding-protein-independent initiation: YY1, TFIIB and RNA polymerase II direct basal transcription on supercoiled template DNA. Cell 76,1994, 1115-1121
63 Chiang, C.M, and R.G. Roeder. Cloning of an intrinsic human TFIID subunit that interacts with multiple transcriptional activators. Science 267,1995, 531-536
64 Seto, E., Shi, Y. and T. Shenk. YY1 is an initiator sequence-binding protein that directs and activates transcription in vitro. Nature 354,1991, 241-245
65 Kwok, R.P., Lundblad, J.R., Chrivia, J.C. et al. Nuclear protein CBP is a coactivator for the transcription factor CREB. Nature 370,1994,223-226
66 Yao, Y.L., Yang, W.M. and E. Seto. Regulation of the transcription factor YY1 by acetylation and deacetylation. Mol. Cell Biol. 17, 2001, 5979-5991
67 van den Brandt, J., Koväcs, P. and I. Klöting. Features of the metabolic syndrome in the spontaneously hypertriglyceridemic W(istar) O(ttawa)K(arlsburg)W(RT1u) rat. Metabolism 49, 2000, 1140-44
68 van den Brandt, J., Kovacs, P. and I. Klöting. Metabolic features in disease-resistant as well as in hypertensive SHR and newly established obese WOKW inbred rats. Int. J. Obesity 24, 2000, 1618-1622
69 Rosmalen, J.G., Leenen, P.J., Pelegri, C. et al. Islet abnormalities in the pathogenesis of autoimmune diabetes. Trends. Endocrinol. Metab. 13, 2002, 209-214
69b Nishiyama C., Yokota T., Nishiyama M. et al. Molecular clönong of the rat transcription factor YY1. Biosci. Biotechnol. Biochem. 67, 2003, 654-658
70 Kolb, H. Benign versus destructive insulitis. Diabetes Metab. Rev. 13, 1997, 139-146
71 Foulis, A.K., Farquharson, M.A. and A. Meager. Immunoreactive alpha-interferon in insulin-secreting beta cells in type 1 diabetes mellitus. Lancet 2(8573), 1987,1423-1427
72 Huang, X., Yuang, J., Goddard, A. et al. Interferon expression in the pancreas of patients with type 1 diabetes. Diabetes 44, 1995, 658-664
73 Foulis, A.K., McGill, M. and M.A. Farquharson. Insulitis in type 1 (insulin-dependent) diabetes mellitus in man: macrophages, lymphocytes, and interferon-γ containing cells. J. Pathol. 165, 1991, 97-103
74 Yamagata, K., Nakajima, H., Tomita, K. et al. Dominant TCR α chain clontypes and interferon-γ are expressed in the pancreas of patients with recent-onset insulin-dependent diabetes mellitus. Diabetes Res. Clin. Pract. 34, 1996,37-46
75 Huang, X., Hultgren, B., Dybdal, N. et al. Islet expression of interferon-α precedes diabetes in both the BB rat and streptozotocin-treated mice. Immunity 1, 1994,469-478
76 Campbell, I.L., Hobbs, M.V., Dockter, J. et al. Islet inflammation and hyperplasia induced by the pancreatic islet-specific overexpression of interleukin-6 in transgenic mice. Am. J. Pathol. 145, 1994, 157-166
77 DiCosmo, B.F., Picarella, D. and R.A. Flavell. Local production of human IL-6 promotes insulitis but retards the onset of insulin-dependent diabetes mellitus in non-obese diabetic mice. Int. Immunol. 6, 1994,1829-1837
78 Wogensen, L., Lee, M. and N. Sarvetnick. Production of interleukin 10 by islet cells accelerates immune-mediated destruction of β cells in non-obese diabetic mice. J. Exp. Med. 179, 1994,1379-1384
79 Moritani, M., Yoshimoto, K., Tashiro, F. et al. Transgenic expression of IL-10 in pancreatic islet A cells accelerates autoimmune diabetes in non-obese diabetic mice. Int. Immunol. 6, 1994,1927-1936
80 Mueller, R., Krahl, T. and N. Sarvetnick. Pancreatic expression of interleucin-4 abrogates insulitis and autoimmune diabetes in non-obese diabetic (NOD) mice. J. Exp. Med. 184, 1996, 1093-1099
81 Rabinovitch, A. and W.L. Suarez-Pinzon. Cytokines and their roles in pancreatic islet β- cell destruction and insulin-dependent diabetes mellitus. Biochem. Pharmacol. 55, 1998, 1139-1149
82 Klöting, I., van den Brandt, J. and B. Kuttler. Genes of SHR rats protect spontaneously diabetic BB/OK rats from diabetes: Lessons from congenic BB.SHR rat strains. Biochem. Biophys. Res. Commun.283, 2001, 399-405
83 Guo, J., Casolaro, V., Seto, E., et al. Yin-Yang 1 activates interleukin-4 gene expression in T cells. J. Biol. Chem. 276, 2001, 48871-48878
84 Ye ,J., Cippitelli, M., Dorman, L. et al. The nuclear factor YY1 suppresses the human gamma interferon promoter through two mechanisms: inhibition of AP1 binding and activation of a silencer element. Mol. Cell Biol.16, 1996, 4744-4753
85 Hänninen, A., Jalkanen, S., Salmi, M. et al. Macrophages, T-cell receptor usage, and endothelial cell activation in the pancreas at the onset of insulin-dependent diabetes mellitus. J. Clin. Invest. 90, 1992,1901-1910
86 Kay, T.W.H., Campbell, I.L., Oxbrow, L. et al. Overexpression of class I major histocompatibility complex accompanies Insulitis in the non-obese diabetic mouse and is prevented by anti-interferonγ-antibody. Diabetologia,34, 1991,779-785
87 Garban, H.J. and B. Bonavida. Nitric oxide inhibits the transcription repressor Yin-Yang 1 binding activity at the silencer region of the Fas promoter: a pivotal role for nitric oxide in the up-regulation of Fas gene expression in human tumor cells. J. Immunol. 167, 2001, 75-81
88 Like, A.A., Butler, L., Williams, R.M. et al. Spontaneous autoimmune diabetes mellitus in the BB rat. Diabetes 31 (Suppl. 1), 1982, 7-13
89 Janeway, C.A. and P.Travers. Immunologie. Spektrum Akademischer Verlag Heidelberg, Berlin, Oxford.1997; 2 Auflage: 208-236.
90 Reizis, B. and P. Leder. Expression of the mouse Pre-T cell receptor α Gene is controlled by an upstream region containing a transcriptional enhancer. J. Exp. Med. 189,1999,1669-1678
91 Iritani, B.M. and R.N. Eisenman. c-Myc enhances protein synthesis and cell size during B lymphocyte development. Proc. Natl. Acad. Sci. USA, 96, 1999, 13180-13185
102 Dang, C., Resar, L., Emison, E. et al. Function of the c-Myc oncogenic transcription factor. Exp. Cell Res.,253, 1999, 63-77
103 Austen, M., Cerni, C., Luscher-Firzlaff, J.M.and B. Luscher. YY1 can inhibit c-Myc function through a mechanism requiring DNA binding of YY1 but neither its transactivation domain nor direct interaction with c-Myc. Oncogene 30, 1998, 511-520
104 Menssen, A. and H. Hermeking. Characterization of the c-Myc-regulated transcriptome by SAGE: Identification and analysis of c-Myc target genes. Proc. Natl. Acad. Sci. U S A 99, 2002, 6274-6279
105 Schwab, M., Varmus, H.E., Bishop, J.M. et al. Chromosome localisation in normal human cells and neuroplastomas of a gene related to c-Myc. Nature 308,1984, 288-291
106 Näu, M.M., Brooks, B.J., Battey, J. et al. L-myc, a new myc-related gene amplified and expressed in human small cell lung cancer. Nature 318, 1985, 69-73
107 Sugiyama, A., Kume, A., Nemoto, K. et al. Isolation and characterization of s-myc, a member of the rat myc gene family. Proc. Natl. Acad. Sci. U S A 86, 1989, 9144-9148
108 Asker, C., Steinitz, M., Andersson, K. et al. Nucleotide sequence of the rat B-myc gene. Oncogene 4, 1989,1523-1527
109 Blackwell, T.K., Huang, J., Ma, A. et al. Binding of myc proteins to canonical and noncanonical DNA sequences. Mol. Cell Biol. 13,1993, 5216-5224
110 Blackwood, E.M. and R.N. Eisenmann. Max: a helix-loop-helix zipper protein that forms a sequence-specific DNAbinding complex with Myc. Science 251,1991, 1211-1217
111 Grandori, C., Cowley, S.M., James, L.P. et al. The Myc/Max/Mad network and the transcriptional control of cell behaviour. Annu. Rev. Cell Dev. Biol. 16, 2000, 653-699
112 Klöting, I. and L. Vogt. Influence of age and metabolic state of mothers on the life expectancy of non-diabetic offspring in diabetes-prone BB/OK rats. Proceedings of the Fifth FELASA Symposium, Ed. Bunyan, J., 1994, 265-269
131 Stocco, D.M. Tracking the role of a StAR in the sky of the new milennium. Mol. Endocrinol. 15, 2001, 1245-1254
132 Nackley, A.C., Shea-Eaton, W., Lopez, D. et.al. Repression of the steroidogenic acute regulatory gene by the multifunctional transcription factor Yin Yang 1. Endocrinology 143, 2002,1085-1096
133 Christenson, L.K., Osborne, T.F., McAllister, J.M. et al. Conditional response of the human steroidogenic acute regulatory protein gene promoter to sterol regulatory element binding protein-1a. Endocrinology 142, 2001, 28-36
134 Ericsson, J., Usheva, A. and P.A. Edwards.. YY1 is a negative regulator of transcription of three sterol regulatory element-binding protein-responsive genes. J. Biol. Chem.274, 1999, 14508-14513
135 Amemiya-Kudo, M., Shimano, H., Yoshikawa ,T. et al. Promoter analysis of the mouse sterol regulatory element-binding protein-1c gene. J. Biol. Chem.275, 2000, 31078-31085
136 Amemiya-Kudo, M., Shimano, H., Hasty, A.H. et al. Transcriptional activities of nuclear SREBP-1a, -1c. and -2 to different target promoters of lipogenic and cholesterogenic genes. J. Lipid Res. 43, 2002, 1220-1235
137 Shea-Eaton, W., Lopez, D. and M.P. McLean. Yin Yang 1 protein negatively regulates high-density lipoprotein receptor gene transcription by disrupting binding of sterol regulatory element binding protein to the sterol regulatory element. Endocrinology 142, 2001, 49-58
138 Bennett, M.K. and T.F. Osborne . Nutrient regulation of gene expression by the sterol regulatory element binding proteins: increased recruitment of gene-specific coregulatory factors and selective hyperacetylation of histone H 3 in vivo. Proc. Natl. Acad. Sci. USA 97, 2000, 6340-6344
139 Hasty, A.H., Shimano, H., Yahagi, N. et al. Sterol regulatory element-binding protein-1 is regulated by glucose at the transcriptional level. J. Biol. Chem. 275, 2000, 31069-31077
140 Sun, L., Halaihel, N.et al. Role of sterol regulatory element-binding protein 1 in regulation lipid metabolism and glomerulosclerosis in diabetes mellitus. J. Biol. Chem. 277, 2002, 18019-18927
141 Sewter, C., Berger, D., et al. Human obesity and type 2 diabetes are associated with alterations in SREBP 1 isoform expression that are reproduced ex vivo by tumor necrosis factor. Diabetes 51, 2002,1035-1041
142 Hua, X., Yokoyama, C., Wu, J. et al. SREBP-2, a second basic-helix-loop-helix-leucine zipper protein that stimulates transcription by binding to a sterol regulatory element. Proc. Natl. Acad. Sci. USA 90, 1993,11603-11607
143 Lund, J. and H.F. DeLuca. Biologically active metabolite of vitamin D3 from bone, liver, and blood serum. J Lipid Res 7, 1966, 739-744
144 Löffler, G. and P.E. Petrides. Biochemie und Pathobiochemie. Springer Verlag Berlin, Heidelberg, New York 1998; 6. Auflage: 657-659
145 Barker, A.R., McDonnell, D.P., Huges, M. et al. Cloning and expression of full length cDNA and coding human vitamin D receptor. Proc. Nat. Acad. Sci. 85, 1988, 3294-3298
146 Brumbaugh, P.F. and Haussler M.R. Nuclear and cytoplasmatic binding components for vitamin D metabolites. Life Sci 1975; 16: 353-362.
147 DeLuca H.F. and m.T. Cantorna. Vitamin D: its role and uses in immunology. FASEB J 2001; 15: 2579-2585.
148 Guo B., Aslam F., van Wijnen A.J. et al. YY1 regulates vitamin D receptor/retinoid X receptor mediated transactivation of the vitamin D responsive osteocalcin gene. Proc Natl Acad Sci USA 1997; 94: 121-126.
149 Raval-Pandya M., Dhawan P., Barletta F. et al. YY1 represses Vitamin D receptor mediated 25-Hydroxyvitamin D3 24-Hydroxylase transcription: relief of repression by CREB-binding protein. Mol Endocrinology 2001; 15: 1035-1046.
150 Kasuga H., Hosogane N., Matsuoka K. et al. Characterization of transgenic rats constitutively expressing vitamin D-24-hydroxylase gene. Biochem Biophys Res Commun 2002; 297(5): 1332-1338.
151 Chang T.J., Lei H.H., Yeh J.I. et al. Vitamin D receptor gene polymorphisms influence susceptibility to type 1 diabetes mellitus in the Taiwanese population. Clin Endocrinology 2000; 52: 575-580.
152 Li Y.C., Kong J., Wei M. et al. 1,25 Dihydroxyvitamin D3 is a negative endocrine regulator of the renin-angiotensin-system. J Clin Invest 2002; 110: 229-238.
153 Bhalla S.S., Robitaille L. and M. Nemer. Cooperative action by GATA-4 and YY1 of the cardiac b-type natriuretic peptide promotor. 2001 J Biol Chem 276: 11439-11445.
154 Charron F. and M. Nemer. GATA transcription factors and cardiac development. Semin Cell Dev Biol 1999; 10(1): 85-91.
155 Vincent C.K., Gualberto A., Patel C.V. et al. Different regulatory sequences control creatine kinase-M gene expression in directly injected skeletal and cardiac muscle. Mol Biol Cell 1993; 13(2): 1264-1272.
156 Lee T.C., Zhang Y. and R.J. Schwartz. Bifunctional transcriptional properties of YY1 in regulating muscle actin and c-myc gene expression during myogenesis. Oncogene 1994; 9(4): 1047-1052.
157 Chen C.Y. and R.J. Schwartz. Competition between negative acting YY1 versus positive acting serum response factor and tinman homologue Nkx-2.5 regulates cardiac alpha-actin promotor activity. Mol Endocrinol 1997; 11(6): 812-822.
158 MacLellan W.R., Lee T.C., Schwartz R.J. et al. Transforming growth factor-beta response elements of the alpha-actin gene. Combinatorial action of serum response factor, YY1, and SV40 enhancer-binding protein, TEF-1. J Biol Chem 1994; 269(24): 16754-16760.
159 Patten M., Hartogenis W.F. and C.S. Long. Interleukin 1β Is a negative transcriptional regulator of alphal-adrenic induces gene expression in cultured cardiac myotytes. J Biol Chem 1996; 271(35): 21134-21141.
160 Tan, T.P., Nonaka, K., Nuckolls, G.H., Liu, Y.H. , Maxson, R.E. , Slavkin, H.C. and L. Shum. YY1 activates Msx2 gene independent of bone morphogenetic protein signalling. Nucl. Acids Res. 30, 2002, 1213-1223
161 Lumsden, A. and R. Krumlauf. Patterning the vertebrate neuraxis. Science 274, 1996, 1109-1115
162 Chariot, A., Gielen, J., Merville, M.P. and V. Bours. The homeodomain-containing proteins: an update on their interacting partners. Biochem. Pharmacol. 58, 1999, 1851-1857
163 Bendall, A.J. and C. Abate-Shen. Roles for Msx and Dlx homeoproteins in vertebrate development. Gene 247, 2000,17-31.
164 Liu,Y.H., Ma, L., Kundu,R., Ignelzi,M., Sangiorgi,F., Wu,L., Luo,W., Snead, M.L. and R. Maxson . Function of the Msx2 gene in the morphogenesis of the skull. Ann. N.Y. Acad. Sci.785, 1996, 48-58
165 Cohen, M. M., Jr. Craniofacial disorders caused by mutations in homeobox genes MSX1 and MSX2. J. Craniofac. Genet Dev. Biol.20, 2000, 19-25
166 Pan, Z., Lichtler, A.C. and W.B. Upholt. DNase I hypersensitive sites in the chromatin of the chicken Msx2 gene differ in anterior and posterior limb mesenchyme, calvarial osteoblasts and embryonic fibroblasts. Biochem. Mol. Biol. Int. 46, 1998, 549-557
167 Donohoe, M.E., Zhang, X., McGinnis, L., Biggers, J., Li,E. and Y. Shi. Targeted disruption of mouse Yin Yang 1 transcription factor resülts in peri-implantation lethality. Mol. Cell. Biol. 19, 1999, 7237-7244
168 Jansen, B. and U.Zangemeister-Wittke.Antisense therapy for cancer-the time of truth. Lancet Oncol. 3, 2002, 672-683
169 Chen, Y. A novel single-stranded DNA expression vector. Expert Opin. Biol. Ther. 2, 2002, 735-740

## Patentansprüche

1. Protein, **dadurch gekennzeichnet, daß** es die in SEQ ID NO:4 dargestellte Aminosäuresequenz aufweist.

2. Protein, das ein Homologes des Proteins nach Anspruch 1 ist und eine zu der in SEQ ID NO:4 dargestellten Sequenz homologe Aminosäuresequenz mit einem Homologiegrad von mindestens 95% aufweist, **dadurch gekennzeichnet, dass** das Protein an Position 303 Arginin und an Position 311 Lysin aufweist, wobei das Protein eine diabetesprotektive Wirkung aufweist.

3. Protein nach Anspruch 2, das einen Homologiegrad von mindestens 97% aufweist.

4. Protein nach Anspruch 2 oder 3, das einen Homologiegrad von mindestens 99% aufweist.

5. Peptid, **dadurch gekennzeichnet, dass** es ein Fragment des Proteins nach den Ansprüchen 1 bis 4 ist und eine Aminosäuresequenz aufweist, die den die Aminosäurepositionen 303 und 311 in SEQ ID NO:4 umfassenden Sequenzbereich enthält.

6. Peptid nach Anspruch 5, **dadurch gekennzeichnet, dass** es eine Länge von 53 bis 315 Aminosäuren aufweist.

7. Nukleinsäure, **dadurch gekennzeichnet, dass** sie ein für ein Protein oder ein Peptid nach den Ansprüchen 1 bis 6 kodiert.

8. Nukleinsäure nach Anspruch 7, **dadurch gekennzeichnet, dass** sie die in SEQ ID NO:3 dargestellte Nukleinsäuresequenz aufweist.

9. Nukleinsäure nach Anspruch 7, **dadurch gekennzeichnet, dass** sie eine Nukleinsäuresequenz aufweist, die den die Nukleinsäurepositionen 979-981 und 1003-1005 in SEQ ID NO:3 umfassenden Sequenzbereich enthält.

10. Verfahren zur Bestimmung der Neigung, an Typ-1-Diabetes zu erkranken, **dadurch gekennzeichnet, dass** man genomische DNA aus isolierten mononukleäre Blutzellen amplifiziert, sequenziert und man Änderungen oder Abweichungen der Nukleinsäuresequenz, die für ein Protein mit der in SEQ ID NO:6 dargestellten Aminosäuresequenz kodiert, bestimmt, wobei Abweichungen von Codons (nicht-stille Mutationen) eine erhöhte Neigung anzeigen, an einer Autoimmunerkrankung, Typ-2-Diabetes, Krebs oder Mineral- und Lipidstoffwechselstörungen zu erkranken.

11. Verfahren nach Anspruch 10, **dadurch gekennzeichnet, dass**, man zur Amplifikation die Primer K815-F/K817-R; K815-F/K875-R; K821-F/K817-R; K821-F/K870-R; K874-F/K870-R; K823-F/K825-R; K884-F/K806-R; K801-F/K804-R; K814-F/K832-R; K828-F/K833-R; K831-F/K817-R; K815-F/K870-R; K815-F/K818-R; K816-F/K819-R; K834-F/K836-R, F15/R12; F15/R14; F15/R13; F57/R16; F57/R20; F57/R21; F59/R25; F9/R30; F59/R33; F95/R34; F96/R39; F95/R48; F95/R50; F60/R7; F60/R8; F60/R66; F60/R67; F96/R76; F96/R77; F96/R81; F96/R83; F33/R1; F33/R4; F33/R15; F39/R28; F40/R3; oder F41/R5, wie in Figur 11 dargestellt, verwendet.

12. Verfahren zur Bestimmung der Neigung, an Typ-1-Diabetes zu erkranken, **dadurch gekennzeichnet, dass** man RNA aus isolierten mononukleäre Blutzellen oder Gewebsbiopsien isoliert, amplifiziert und quantifiziert, wobei eine erhöhte/verminderte Expression des Transkriptionsfaktors Yin-Yang-1 eine erhöhte/verminderte Neigung, an Typ-1-Diabetes, Autoimmunerkrankungen im allgemeinen, Typ-2-Diabetes; Krebs oder Mineral- und Lipidstoffwechselstörungen zu erkranken, anzeigt.

13. Verwendung eines Proteins oder Peptids nach den Ansprüchen 1 bis 6 zur Herstellung einer pharmazeutischen Zusammensetzung.

14. Verwendung einer Nukleinsäure nach den Ansprüchen 7 bis 9 oder eines Antisense-Oligonukleotids zu derselben zur Herstellung einer pharmazeutischen Zusammensetzung.

15. Pharmazeutische Zusammensetzung, die eine Nukleinsäure nach den Ansprüchen 7 bis 9 oder ein Antisense-Oligonukleotid zu derselben enthält.

16. Pharmazeutische Zusammensetzung, die ein Protein und/oder ein Peptid nach den Ansprüchen 1 bis 6 enthält.

17. Zusammensetzung nach Anspruch 15 oder 16, **dadurch gekennzeichnet, dass** sie ferner pharmazeutisch verträgliche Hilfs- und/oder Trägerstoffe enthält.

18. Zusammensetzung nach den Ansprüchen 15 bis 17, **dadurch gekennzeichnet, dass** die Zusammensetzung zur intravenösen Applikation formuliert ist.

19. Transgene Ratte, **dadurch gekennzeichnet, daß** deren Keim- und somatische Zellen eine Nukleinsäure oder einen Nukleinsäureabschnitt enthalten, die/der für ein Protein mit der in SEQ ID NO:2 gezeigten Aminosäuresequenz oder einer dazu homologen Aminosäuresequenz mit einem Homologiegrad von mindestens 95% kodiert, wobei die homologe Aminosäure-sequenz an Position 303 Methionin und an Position 311 Arginin aufweist.

20. Transgene Ratte nach Anspruch 19, **dadurch gekennzeichnet, dass** sie das Protein im Pankreas exprimiert.

21. Verwendung einer transgenen oder kongenen Ratte, dessen Keim- und somatische Zellen eine Nukleinsäure oder einen Nukleinsäureabschnitt enthalten, die/der für ein Protein mit der in SEQ ID NO:2 gezeigten Aminosäuresequenz oder einer dazu homologen Aminosäuresequenz mit einem Homologiegrad von mindestens 95% kodiert, wobei die homologe Aminosäure-sequenz an Position 303 Methionin und an Position 311 Arginin aufweist, zum Identifizieren Diabetes-protektiver Substanzen.

22. Verwendung nach Anspruch 21, **dadurch gekennzeichnet, dass** der Homologiegrad mindestens 97% beträgt.

23. Verwendung nach Anspruch 21 oder 22, **dadurch gekennzeichnet, dass** der Homologiegrad mindestens 99% beträgt.

## Claims

1. Protein comprising the amino acid sequence shown in SEQ ID NO: 4.

2. Protein which is a homologue of the protein according to claim 1, and which comprises an amino acid sequence homologous to the sequence shown in SEQ ID No: 4 with a homology of at least 95%, wherein the protein comprises arginine in position 303 and lysine in position 311, wherein the protein has a protective effect in diabetes.

3. Protein according to claim 2 having a homology of at least 97%.

4. Protein according to claim 2 or 3 having a homology of at least 99%.

5. Peptide which is a fragment of the protein according to claims 1 to 4, and which has an amino acid sequence comprising the sequence region comprising amino acid position 303 and 311 in SEQ ID NO: 4.

6. Peptide according to claim 5 having a length of 53 to 315 amino acids.

7. Nucleic acid encoding a protein or a peptide according to claims 1 to 6.

8. Nucleic acid according to claim 7 comprising the nucleic acid sequence shown in SEQ ID NO: 3.

9. Nucleic acid according to claim 7 having a nucleic acid sequence comprising the sequence region comprising the nucleic acid positions 979-981 and 1003-1005 in SEQ ID NO: 3.

10. Method for determining the disposition of getting diabetes type-1, **characterised in that** genomic DNA from isolated mononuclear blood cells is amplified, sequenced, and changes or deviations from the nucleic acid sequence encoding a protein with the amino acid sequence shown in SEQ ID No: 6 are determined, wherein deviations of codons (non-silent mutations) indicate an increased disposition of getting an autoimmune disease, type-2 diabetes, cancer or disorders of mineral or lipid metabolism.

11. Method according to claim 10, **characterized in that**, for amplification, the primers K815-F/K817-R; K815-F/K875-R; K821-F/K817-R; K821-F/K870-R; K874-F/K870-R; K823-F/K825-R; K884-F/K806-R; K801-F/K804-R; K814-F/K832-R; K828-F/K833-R; K831-F/K817-R; K815-F/K870-R; K815-F/K818-R; K816-F/K819-R; K834-F/K836-R; F15/R12; F15/R14; F15/R13; F57/R16; F57/R20; F57/R21; F59/R25; F9/R30; F59/R33; F95/R34; F96/R39; F95/R48; F95/R50; F60/R7; F60/R8; F60/R66; F60/R67; F96/R76, F96/R77, F96/R81; F96/R83; F33/R1; F33/R4; F33/R15; F39/R28; F40/R3; or F41/R5, as shown in Figure 11, are used.

12. Method for determining the disposition of getting diabetes type-1, **characterized in that** RNA from isolated mononucleic blood cells or tissue biopsies is isolated, amplified and quantified, wherein an increased/decreased expression of the transcription factor Yin-Yang-1 indicates an increased/decreased disposition of getting type-1 diabetes, autoimmune diseases in general, type-2 diabetes, cancer or disorders of mineral or lipid metabolism.

13. Use of a protein or peptide according to claims 1 to 6 for the preparation of a pharmaceutical composition.

14. Use of a nucleic acid according to claims 7 to 9 or an antisense oligonucleotide thereof for preparing a pharmaceutical composition.

15. Pharmaceutical composition comprising a nucleic acid according to claims 7 to 9 or an antisense oligonucleotide thereof.

16. Pharmaceutical composition comprising a protein and/or a peptide according to claims 1 to 6.

17. Composition according to claims 15 or 16, further comprising pharmaceutically acceptable auxiliary and/or carrier substances.

18. Composition according to claims 15 to 17, **characterized in that** the composition is formulated for intravenous application.

19. Transgenic rat **characterized in that** its germ cells and somatic cells comprise a nucleic acid or nucleic acid region encoding a protein having the amino acid sequence shown in SEQ ID NO: 2 or an amino acid sequence homologous thereto with a homology of at least 95%, wherein the homologous amino acid sequence has methionine in position 303 and arginine in position 311.

20. Transgenic rat according to claim 19, **characterized in that** it expresses the protein in the pancreas.

21. Use of a transgenic or congenic rat, whose germ cells and somatic cells comprise a nucleic acid or a nucleic acid region encoding a protein having the amino acid sequence shown in SEQ ID No: 2 or an amino acid sequence homologous thereto having a homology of at least 95%, wherein the homologous amino acid sequence has methionine in position 303 and arginine in position 311, for identifying substances protective against diabetes.

22. Use according to claim 21, wherein the homology is at least 97%.

23. Use according to claim 21 or 22, wherein the homology is at least 99%.

## Revendications

1. Protéine comprenant la séquence d'acides aminés représentée en tant que Séquence N° 4.

2. Protéine homologue d'une protéine conforme à la revendication 1, comprenant une séquence d'acides aminés homologue de la séquence représentée en tant que Séquence N° 4, à un degré d'homologie d'au moins 95 %, laquelle protéine comporte un résidu d'arginine en position 303 et un résidu de lysine en position 311, et laquelle protéine possède un effet protecteur contre le diabète.

3. Protéine conforme à la revendication 2, dont le degré d'homologie est d'au moins 97 %.

4. Protéine conforme à la revendication 2 ou 3, dont le degré d'homologie est d'au moins 99 %.

5. Peptide qui est un fragment d'une protéine conforme à l'une des revendications 1 à 4, et qui présente une séquence d'acides aminés comportant la région qui entoure les résidus d'acides aminés placés en positions 303 et 311 dans la Séquence N° 4.

6. Peptide conforme à la revendication 5, qui est long de 53 à 315 résidus d'acides aminés.

7. Acide nucléique codant une protéine ou un peptide conforme à l'une des revendications 1 à 6.

8. Acide nucléique conforme à la revendication 7, comprenant la séquence d'acide nucléique représentée en tant que Séquence N° 3.

9. Acide nucléique conforme à la revendication 7, qui présente une séquence d'acide nucléique comportant la région qui entoure les nucléotides placés en positions 979-981 et 1003-1005 dans la Séquence N° 3.

10. Procédé de détermination du risque d'être atteint d'un diabète de type I, **caractérisé en ce que** l'on amplifie et séquence de l'ADN génomique provenant de globules sanguins mononucléaires isolés et l'on en détermine les écarts et variations par rapport à la séquence d'acide nucléique qui code une protéine présentant la séquence d'acides aminés représentée en tant que Séquence N° 6, étant entendu que des écarts de codons (mutations non-silencieuses) indiquent un risque accru d'être atteint d'une maladie auto-immune, d'un diabète de type 2, d'un cancer ou de troubles du métabolisme des minéraux ou des lipides.

11. Procédé conforme à la revendication 10, **caractérisé en ce que** l'on utilise, pour opérer l'amplification, les amorces K815-F/K817-R, K815-F/K875-R, K821-F/K817-R, K821-F/K870-R, K874-F/K870-R, K823-F/K825-R, K884-F/K806-R, K801-F/K804-R, K814-F/K832-R, K828-F/K833-R, K831-F/K817-R, K815-F/K870-R, K815-F/K818-R, K816-F/K819-R, K834-F/K836-R, F15/R12, F15/R14, F15/R13, F15/R16, F57/R20, F57/R21, F59/R25, F9/R30, F59/R33, F95/R34, F96/R39, F95/R48, F95/R50, F60/R7, F60/R8, F60/R66, F60/R67, F96/R76, F96/R77, F96/R81, F96/R83, F33/R1, F33/R4, F33/R15, F39/R28, F40/R3, ou F41/R5, comme on l'a représenté sur la figure 11.

12. Procédé de détermination du risque d'être atteint d'un diabète de type I, **caractérisé en ce que** l'on isole de l'ARN à partir de globules sanguins mononucléaires isolés ou de biopsies tissulaires, et l'on amplifie et quantifie cet ARN, étant entendu qu'une expression augmentée/diminuée du facteur de transcription Yin-Yang-1 indique un risque accru/réduit d'être atteint d'un diabète de type I, de maladies auto-immunes en général, d'un diabète de type 2, d'un cancer ou de troubles du métabolisme des minéraux ou des lipides.

13. Utilisation d'une protéine ou d'un peptide, conforme à l'une des revendications 1 à 6, en vue de la préparation d'une composition pharmaceutique.

14. Utilisation d'un acide nucléique, conforme à l'une des revendications 7 à 9, ou d'un oligonucléotide anti-sens d'un tel acide nucléique, en vue de la préparation d'une composition pharmaceutique.

15. Composition pharmaceutique qui contient un acide nucléique, conforme à l'une des revendications 7 à 9, ou un oligonucléotide anti-sens d'un tel acide nucléique.

16. Composition pharmaceutique qui contient une protéine et/ou un peptide, conforme(s) à l'une des revendications 1 à 6.

17. Composition conforme à la revendication 15 ou 16, qui comprend en outre des adjuvants et/ou véhicules pharmacologiquement admissibles.

18. Composition conforme à l'une des revendications 15 à 17, **caractérisée en ce que** cette composition est formulée en vue d'une administration par voie intraveineuse.

19. Rat transgénique, **caractérisé en ce que** ses cellules germinales et ses cellules somatiques contiennent un acide nucléique ou une région d'acide nucléique qui code une protéine présentant la séquence d'acides aminés représentée en tant que Séquence N° 2 ou une séquence d'acides aminés homologue de cette séquence, à un degré d'homologie d'au moins 95 %, laquelle séquence d'acides aminés homologue comporte un résidu de méthionine en position 303 et un résidu d'arginine en position 311.

20. Rat transgénique conforme à la revendication 19, **caractérisé en que** la protéine est exprimée dans son pancréas.

21. Utilisation d'un rat transgénique ou congénique, dont les cellules germinales et les cellules somatiques contiennent un acide nucléique ou une région d'acide nucléique codant une protéine qui présente la séquence d'acides aminés représentée en tant que Séquence N° 2 ou une séquence d'acides aminés homologue de cette séquence, à un degré d'homologie d'au moins 95 %, laquelle séquence d'acides aminés homologue comporte un résidu de méthionine en position 303 et un résidu d'arginine en position 311, en vue de l'identification de substances protectrices contre un diabète.

22. Utilisation conforme à la revendication 21, pour laquelle ledit degré d'homologie est d'au moins 97 %.

23. Utilisation conforme à la revendication 21 ou 22, pour laquelle ledit degré d'homologie est d'au moins 99 %.
